(19) Europäisches Patentamt / European Patent Office / Office européen des brevets

(11) EP 2 888 233 B1

(12) **EUROPEAN PATENT SPECIFICATION**

(45) Date of publication and mention of the grant of the patent:
**28.09.2016 Bulletin 2016/39**

(21) Application number: **13719386.8**

(22) Date of filing: **17.04.2013**

(51) Int Cl.:
***C07D 213/82*** *(2006.01)* ***A61K 31/444*** *(2006.01)*

(86) International application number:
**PCT/EP2013/001134**

(87) International publication number:
**WO 2013/156154 (24.10.2013 Gazette 2013/43)**

(54) **SUBSTITUTED 6-AMINO-NICOTINAMIDES BEARING AN OH-CONTAINING GROUP AS KCNQ2/3 MODULATORS**

SUBSTITUIERTE 6-AMINONIKOTINAMIDE, DIE EINE OH-HALTIGE GRUPPE ALS KCNQ-2/3-MODULATOREN TRAGEN

6-AMINO-NICOTINAMIDES SUBSTITUÉS COMPORTANT UN GROUPE CONTENANT DE L'OH EN TANT QUE MODULATEURS KCNQ2/3

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.04.2012 EP 12002709**

(43) Date of publication of application:
**01.07.2015 Bulletin 2015/27**

(73) Proprietor: **Grünenthal GmbH**
**52078 Aachen (DE)**

(72) Inventors:
- **LUCAS, Simon**
  **A-3012 Wolfsgraben (AT)**
- **KÜHNERT, Sven**
  **52355 Düren (DE)**
- **BAHRENBERG, Gregor**
  **52156 Monschau-Konzen (DE)**
- **SCHRÖDER, Wolfgang**
  **52074 Aachen (DE)**
- **KLESS, Achim**
  **52072 Aachen (DE)**

(56) References cited:
**WO-A1-2010/102809** **WO-A1-2012/052167**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to substituted 6-amino-nicotinamides bearing an OH-containing group, to pharmaceutical compositions containing these compounds and also to these compounds for use in the treatment and/or prophylaxis of pain and further diseases and/or disorders.

The treatment of pain, in particular of neuropathic pain, is of great importance in medicine. There is a worldwide need for effective pain therapies. The urgent need for action for a target-orientated treatment of chronic and non-chronic states of pain appropriate for the patient, by which is to be understood the successful and satisfactory treatment of pain for the patient, is also documented in the large number of scientific works which have recently been published in the field of applied analgesics and of fundamental research into nociception.

A pathophysiological feature of chronic pain is the overexcitability of neurons. Neuronal excitability is influenced decisively by the activity of $K^+$ channels, since these determine decisively the resting membrane potential of the cell and therefore the excitability threshold. Heteromeric $K^+$ channels of the molecular subtype KCNQ2/3 (Kv7.2/7.3) are expressed in neurons of various regions of the central (hippocampus, amygdala) and peripheral (dorsal root ganglia) nervous system and regulate the excitability thereof. Activation of KCNQ2/3 $K^+$ channels leads to a hyperpolarization of the cell membrane and, accompanying this, to a decrease in the electrical excitability of these neurons. KCNQ2/3-expressing neurons of the dorsal root ganglia are involved in the transmission of nociceptive stimuli from the periphery into the spinal marrow (Passmore et al., J. Neurosci. 2003; 23(18): 7227-36).

It has accordingly been possible to detect an analgesic activity in preclinical neuropathy and inflammatory pain models for the KCNQ2/3 agonist retigabine (Blackburn-Munro and Jensen, Eur J Pharmacol. 2003; 460(2-3); 109-16; Dost et al., Naunyn Schmiedebergs Arch Pharmacol 2004; 369(4): 382-390).

The KCNQ2/3 $K^+$ channel thus represents a suitable starting point for the treatment of pain; in particular of pain selected from the group consisting of chronic pain, acute pain, neuropathic pain, inflammatory pain, visceral pain and muscular pain (Nielsen et al., Eur J Pharmacol. 2004; 487(1-3): 93-103), in particular of neuropathic and inflammatory pain.

Moreover, the KCNQ2/3 $K^+$ channel is a suitable target for therapy of a large number of further diseases, such as, for example, migraine (US2002/0128277), cognitive diseases (Gribkoff, Expert Opin Ther Targets 2003; 7(6): 737-748), anxiety (Korsgaard et al., J Pharmacol Exp Ther. 2005, 14(1): 282-92), epilepsy (Wickenden et al., Expert Opin Ther Pat 2004; 14(4): 457-469; Gribkoff, Expert Opin Ther Targets 2008, 12(5): 565-81; Miceli et al., Curr Opin Pharmacol 2008, 8(1): 65-74), urinary incontinence (Streng et al., J Urol 2004; 172: 2054-2058), dependency (Hansen et al., Eur J Pharmacol 2007, 570(1-3): 77-88), mania/bipolar disorders (Dencker et al., Epilepsy Behav 2008, 12(1): 49-53) and dystonia-associated dyskinesias (Richter et al., Br J Pharmacol 2006, 149(6): 747-53).

Substituted compounds that have an affinity for the KCNQ2/3 $K^+$ channel are e.g. known from the prior art (WO 2008/046582, WO 2010/046108, WO 2010/102809 and WO 2002/066036 and WO2012/052167).

DE 25 13 949 and GB 1 420 987 disclose substituted nicotinamides and derivatives thereof as coupling components for the preparation of azo dyes.

There is a demand for further compounds having comparable or better properties, not only with regard to affinity to KCNQ2/3 $K^+$ channels per se *(potency, efficacy)*.

Thus, it may be advantageous to improve the metabolic stability, the solubility in aqueous media or the permeability of the compounds. These factors can have a beneficial effect on oral bioavailability or can alter the PK/PD (pharmacokinetic/pharmacodynamic) profile; this can lead to a more beneficial period of effectiveness, for example. A weak or non-existent interaction with transporter molecules, which are involved in the ingestion and the excretion of pharmaceutical compositions, is also to be regarded as an indication of improved bioavailability and at most low interactions of pharmaceutical compositions. Furthermore, the interactions with the enzymes involved in the decomposition and the excretion of pharmaceutical compositions should also be as low as possible, as such test results also suggest that at most low interactions, or no interactions at all, of pharmaceutical compositions are to be expected.

In addition, it may be advantageous if the compounds show a high selectivity towards other receptors of the KCNQ family (*specificity*), e.g. towards KCNQ1, KCNQ3/5 or KCNQ4. A high selectivity may have a positive effect on the side effects profile: for example it is known that compounds which (also) have an affinity to KCNQ1 are likely to have a potential for cardial side effects. Therefore, a high selectivity towards KCNQ1 may be desirable. However, it may also be advantageous for the compounds to show a high selectivity towards other receptors. For instance, it may be advantageous for the compounds to show a low affinity for the hERG ion channel or the L-type calcium ion channel (phenylalkylamine-, benzothiazepin-, dihydropyridine-binding site) since these receptors are known to possibly have a potential for cardial side effects. Further, an improved selectivity towards binding to other endogenic proteins (i.e. receptors or enzymes) may result in a better side effects profile and, consequently to an improved tolerance.

It was therefore an object of the invention to provide new compounds having advantages over the compounds of the prior art. These compounds should be suitable in particular as pharmacological active ingredients in pharmaceutical compositions, preferably in pharmaceutical compositions for the treatment and/or prophylaxis of disorders and/or diseases which are mediated, at least in part, by KCNQ2/3 $K^+$ channels.

That object is achieved by the subject-matter of the patent claims and by the subject-matter described herein.

It has been found, surprisingly, that substituted compounds of the general formula (I) given below are suitable for the treatment of pain. It has also been found, surprisingly, that substituted compounds of the general formula (I) given below also have an excellent affinity for the KCNQ2/3 $K^+$ channel and are therefore suitable for the prophylaxis and/or treatment of disorders and/or diseases that are mediated at least in part by KCNQ2/3 $K^+$ channels. The substituted compounds thereby act as modulators, i.e. agonists or antagonists, of the KCNQ2/3 $K^+$ channel.

The present invention therefore relates to a substituted compound of general formula (I),

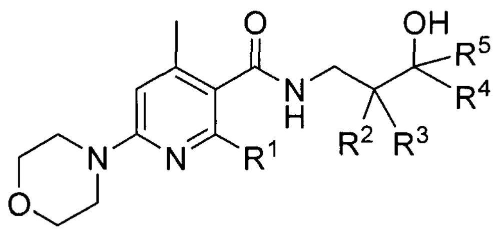

**(I)**,

wherein

$R^1$ represents a $C_{2-6}$-aliphatic residue, unsubstituted or mono- or polysubstituted; or a $C_{3-6}$-cycloaliphatic residue or a 3 to 6 membered heterocycloaliphatic residue, in each case unsubstituted or mono- or polysubstituted, and in each case optionally bridged via a $C_{1-4}$ aliphatic group, which in turn may be unsubstituted or mono- or polysubstituted;

or represents $OR^6$, wherein
$R^6$ represents a $C_{1-6}$-aliphatic residue, unsubstituted or mono- or polysubstituted; or a $C_{3-6}$-cycloaliphatic residue, unsubstituted or mono- or polysubstituted;
$R^2$ and $R^3$ independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted;
with the proviso that at least one of $R^2$ and $R^3$ denotes H or is linked via a carbon atom (to the remaining part of the superordinate structure, i.e. of general formula (I)),
$R^4$ and $R^5$ independently of one another represent H; $CH_2F$; $CHF_2$; $CF_3$; a $C_{1-4}$-aliphatic residue, wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;
on the condition that if $R^4$ and/or $R^5$ denote(s) a 3 to 6 membered heterocycloaliphatic residue, the 3 to 6 membered heterocycloaliphatic residue is linked via a carbon atom (to the remaining part of the superordinate structure, i.e. of general formula (I)),
or
$R^2$ and $R^3$
and/or $R^4$ and $R^5$
in pairs, in each case independently of one another, together with the carbon atom connecting them, form a $C_{3-10}$-cycloaliphatic residue or a 3 to 10 membered heterocycloaliphatic residue, in each case unsubstituted or mono- or polysubstituted,
and the respective remaining substituents of $R^2$ and $R^3$ each independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted,
with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue, wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;

on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted,
and $R^3$ represents H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted;
and $R^5$ represents H; $CH_2F$; $CHF_2$; $CF_3$; a $C_{1-4}$-aliphatic residue, wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;
on the condition that if $R^5$ denotes a 3 to 6 membered heterocycloaliphatic residue, the 3 to 6 membered heterocycloaliphatic residue is linked via a carbon atom,
in which an "aliphatic group" and an "aliphatic residue" can in each case be branched or unbranched, saturated or unsaturated,
in which a "cycloaliphatic residue" and a "heterocycloaliphatic residue" can in each case be saturated or unsaturated,
in which "mono- or polysubstituted" with respect to an "aliphatic group" and an "aliphatic residue" relates, with respect to the corresponding residues or groups, to the substitution of one or more hydrogen atoms each independently of one another by at least one substituent selected from the group consisting of F, Cl, Br, I, $NO_2$, $NH_2$, an NH($C_{1-4}$ aliphatic residue), an N($C_{1-4}$ aliphatic residue)$_2$, a NH-C(=O)-$C_{1-4}$ aliphatic residue, a N($C_{1-4}$-aliphatic residue)-C(=O)-$C_{1-4}$ aliphatic residue, a NH-S(=O)$_2$-$C_{1-4}$ aliphatic residue, a N($C_{1-4}$-aliphatic residue)-S(=O)$_2$-$C_{1-4}$ aliphatic residue, =O, OH, $OCH_2F$, $OCHF_2$, $OCF_3$, a O-$C_{1-4}$-aliphatic residue, a O-C(=O)-$C_{1-4}$-aliphatic residue, SH, $SCF_3$, a S-$C_{1-4}$-aliphatic residue, S(=O)$_2$OH, a S(=O)-$C_{1-4}$-aliphatic residue, a S(=O)$_2$-$C_{1-4}$-aliphatic residue, a S(=O)$_2$-O-$C_{1-4}$-aliphatic residue, a S(=O)$_2$-NH-$C_{1-4}$-aliphatic residue, a S(=O)$_2$-N($C_{1-4}$-aliphatic residue)$_2$, CN, $CH_2F$, $CHF_2$, $CF_3$, CHO, COOH, a $C_{1-4}$-aliphatic residue, $CH_2OH$, $CH_2$-$OCH_3$, $C_2H_4$-OH, $C_2H_4$-$OCH_3$ $CH_2$-$CF_3$, a C(=O)-$C_{1-4}$-aliphatic residue, a C(=O)-O-$C_{1-4}$-aliphatic residue, a $C_{3-6}$-cycloaliphatic residue, a 3 to 6 membered heterocycloaliphatic residue, C(=O)-$NH_2$, a C(=O)-NH($C_{1-4}$ aliphatic residue), and a C(=O)-N($C_{1-4}$ aliphatic residue)$_2$;
in which "mono- or polysubstituted" with respect to a "cycloaliphatic residue" and a "heterocycloaliphatic residue" relates, with respect to the corresponding residues, to the substitution of one or more hydrogen atoms each independently of one another by at least one substituent selected from the group consisting of F, Cl, Br, I, $NO_2$, $NH_2$, an NH($C_{1-4}$ aliphatic residue), an N($C_{1-4}$ aliphatic residue)$_2$, a NH-C(=O)-$C_{1-4}$ aliphatic residue, a N($C_{1-4}$-aliphatic residue)-C(=O)-$C_{1-4}$ aliphatic residue, a NH-S(=O)$_2$-$C_{1-4}$ aliphatic residue, a N($C_{1-4}$-aliphatic residue)-S(=O)$_2$-$C_{1-4}$ aliphatic residue, =O, OH, $OCH_2F$, $OCHF_2$, $OCF_3$, a O-$C_{1-4}$-aliphatic residue, a O-C(=O)-$C_{1-4}$-aliphatic residue, SH, $SCF_3$, a S-$C_{1-4}$-aliphatic residue, S(=O)$_2$OH, a S(=O)-$C_{1-4}$-aliphatic residue, a S(=O)$_2$-$C_{1-4}$-aliphatic residue, a S(=O)$_2$-O-$C_{1-4}$-aliphatic residue, a S(=O)$_2$-NH-$C_{1-4}$-aliphatic residue, a S(=O)$_2$-N($C_{1-4}$-aliphatic residue)$_2$, CN, $CH_2F$, $CHF_2$, $CF_3$, CHO, COOH, a $C_{1-4}$-aliphatic residue, $CH_2OH$, $CH_2$-$OCH_3$, $C_2H_4$-OH, $C_2H_4$-$OCH_3$ $CH_2$-$CF_3$, a C(=O)-$C_{1-4}$-aliphatic residue, a C(=O)-O-$C_{1-4}$-aliphatic residue, a $C_{3-6}$-cycloaliphatic residue, a 3 to 6 membered heterocycloaliphatic residue, C(=O)-$NH_2$, a C(=O)-NH($C_{1-4}$ aliphatic residue), and a C(=O)-N($C_{1-4}$ aliphatic residue)$_2$;
optionally in the form of a single stereoisomer or a mixture of stereoisomers, in the form of the free compound and/or a physiologically acceptable salt thereof and/or a physiologically acceptable solvate, in particular hydrate, thereof.

The term "single stereoisomer" preferably means in the sense of the present invention an individual enantiomer or diastereomer. The term "mixture of stereoisomers" preferably means in the sense of this invention the racemate and mixtures of enantiomers and/or diastereomers in any mixing ratio.

The term "physiologically acceptable salt" preferably comprises in the sense of this invention a salt of at least one compound according to the present invention and at least one physiologically acceptable acid or base.

A physiologically acceptable salt of at least one compound according to the present invention and at least one physiologically acceptable acid preferably refers in the sense of this invention to a salt of at least one compound according to the present invention with at least one inorganic or organic acid which is physiologically acceptable - in particular when used in human beings and/or other mammals. Examples of physiologically acceptable acids are: hydrochloric acid, hydrobromic acid, sulphuric acid, methanesulphonic acid, p-toluenesulphonic acid, carbonic acid, formic acid, acetic acid, oxalic acid, succinic acid, tartaric acid, mandelic acid, fumaric acid, maleic acid, lactic acid, citric acid, glutamic acid, saccharic acid, monomethylsebacic acid, 5-oxoproline, hexane-1-sulphonic acid, nicotinic acid, 2, 3 or 4-aminobenzoic acid, 2,4,6-trimethylbenzoic acid, $\alpha$-lipoic acid, acetyl glycine, hippuric acid, phosphoric acid, aspartic acid. Citric acid and hydrochloric acid are particularly preferred. Hydrochloride salts and citrate salts are therefore particularly preferred salts.

A physiologically acceptable salt of at least one compound according to the present invention and at least one physiologically acceptable base preferably refers in the sense of this invention to a salt of at least one compound

according to the present invention as an anion with at least one preferably inorganic cation, which is physiologically acceptable - in particular when used in human beings and/or other mammals. Particularly preferred are the salts of the alkali and alkaline earth metals but also ammonium salts $[NH_xR_{4-x}]^+$, in which x = 0, 1, 2, 3 or 4 and R represents a branched or unbranched $C_{1-4}$ aliphatic residue residue, in particular (mono-) or (di)sodium, (mono-) or (di)potassium, magnesium or calcium salts.

The terms "$C_{1-6}$-aliphatic residue", "$C_{2-6}$-aliphatic residue", and "$C_{1-4}$-aliphatic residue", preferably comprise in the sense of this invention acyclic saturated or unsaturated aliphatic hydrocarbon residues, which can be branched or unbranched and also unsubstituted or mono- or polysubstituted, containing 1 to 6, or 2 to 6, or 1 to 4 carbon atoms, respectively, i.e. $C_{1-6}$ alkanyls, $C_{2-6}$ alkanyls, $C_{2-6}$ alkenyls and $C_{2-6}$ alkynyls as well as $C_{1-4}$ alkanyls, $C_{2-4}$ alkenyls and $C_{2-4}$ alkynyls, respectively. In this case, alkenyls comprise at least one C-C double bond (a C=C-bond) and alkynyls comprise at least one C-C triple bond (a C≡C-bond). Preferably, aliphatic residues are selected from the group consisting of alkanyl (alkyl) and alkenyl residues, more preferably are alkanyl residues. Preferred $C_{1-6}$ alkanyl residues are selected from the group consisting of methyl, ethyl, n-propyl, 2-propyl, n-butyl, isobutyl, sec.- butyl, tert.-butyl, n-pentyl, isopentyl, neopentyl and n-hexyl. Preferred $C_{1-4}$ alkanyl residues are selected from the group consisting of methyl, ethyl, n-propyl, 2-propyl, n-butyl, isobutyl, sec.-butyl and tert.-butyl. Preferred $C_{2-6}$ alkenyl residues are selected from the group consisting of ethenyl (vinyl), propenyl ($-CH_2CH=CH_2$, $-CH=CH-CH_3$, $-C(=CH_2)-CH_3$), butenyl, pentenyl and hexenyl. Preferred $C_{2-4}$ alkenyl residues are selected from the group consisting of ethenyl (vinyl), propenyl ($-CH_2CH=CH_2$, $-CH=CH-CH_3$, $-C(=CH_2)-CH_3$) and butenyl. Preferred $C_{2-6}$ alkynyl residues are selected from the group consisting of ethynyl, propynyl ($-CH_2-C\equiv CH$, $-C\equiv C-CH_3$), butynyl, pentynyl and hexynyl Preferred $C_{2-4}$ alkynyl residues are selected from the group consisting of ethynyl, propynyl ($-CH_2-C\equiv CH$, $-C\equiv C-CH_3$) and butynyl.

The terms "$C_{3-6}$-cycloaliphatic residue" and "$C_{3-10}$-cycloaliphatic residue" preferably mean for the purposes of this invention cyclic aliphatic hydrocarbons containing 3, 4, 5 or 6 carbon atoms and 3, 4, 5, 6, 7, 8, 9 or 10 carbon atoms, respectively, wherein the hydrocarbons in each case can be saturated or unsaturated (but not aromatic), unsubstituted or mono- or polysubstituted. The cycloaliphatic residues can be bound to the respective superordinate general structure via any desired and possible ring member of the cycloaliphatic residue. The cycloaliphatic residues can also be condensed with further saturated, (partially) unsaturated, (hetero)cyclic, aromatic or heteroaromatic ring systems, i.e. with cycloaliphatic, heterocycloaliphatic, aryl or heteroaryl residues which can in turn be unsubstituted or mono- or polysubstituted. $C_{3-10}$ cycloaliphatic residue can furthermore be singly or multiply bridged such as, for example, in the case of adamantyl, bicyclo[1.1.1]pentyl, bicyclo[2.2.1]heptyl or bicyclo[2.2.2]octyl. Preferred $C_{3-10}$ cycloaliphatic residues are selected from the group consisting of cyclopropyl, cyclobutyl, cyclopentyl, cyclohexyl, cycloheptyl, cyclooctyl, cyclononyl, cyclodecyl, adamantyl,

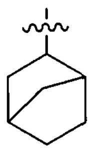 , 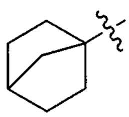 , 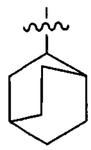 , 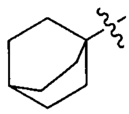 ,

cyclopentenyl, cyclohexenyl, cycloheptenyl and cyclooctenyl. Preferred $C_{3-6}$ cycloaliphatic residues are selected from the group consisting of cyclopropyl, cyclobutyl, cyclopentyl, cyclohexyl, cyclopentenyl and cyclohexenyl.

The terms "3-6-membered heterocycloaliphatic residue", "4-7-membered heterocycloaliphatic residue" and "3-10-membered heterocycloaliphatic residue" preferably mean for the purposes of this invention heterocycloaliphatic saturated or unsaturated (but not aromatic) residues having 3-6, i.e. 3, 4, 5 or 6 ring members, and 4-7, i.e. 4, 5, 6 or 7 ring members, and 3-10, i.e. 3, 4, 5, 6, 7, 8, 9 or 10 ring members, respectively, in which in each case at least one, if appropriate also two or three carbon atoms are replaced by a heteroatom or a heteroatom group each selected independently of one another from the group consisting of O, S, $S(=O)_2$, N, NH and $N(C_{1-8}$ alkyl), preferably $N(CH_3)$, wherein the ring members can be unsubstituted or mono- or polysubstituted. The heterocycloaliphatic residue can be bound to the superordinate general structure via any desired and possible ring member of the heterocycloaliphatic residue if not indicated otherwise. The heterocycloaliphatic residues can also be condensed with further saturated, (partially) unsaturated (hetero)cycloaliphatic or aromatic or heteroaromatic ring systems, i.e. with cycloaliphatic, heterocycloaliphatic, aryl or heteroaryl residues, which can in turn be unsubstituted or mono- or polysubstituted. The term "condensed" also optionally includes spirocycles, i.e. an at least bicyclic ring system, wherein the heterocycloaliphatic residue is connected through just one (spiro)atom with a further saturated, (partially) unsaturated (hetero)cycloaliphatic or aromatic or heteroaromatic ring system. Example of such spirocycles are e.g.

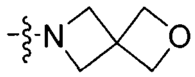

and

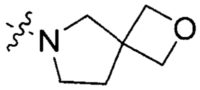

The heterocycloaliphatic residues can furthermore optionally be singly or multiply bridged with a $C_1$- or $C_2$-aliphatic group such as, for example, in the case of

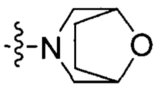, 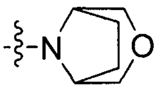,

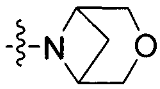, 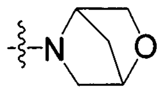, 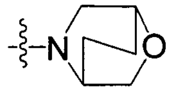 and 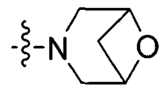

Preferred heterocycloaliphatic residues are selected from the group consisting of azetidinyl, aziridinyl, azepanyl, azocanyl, diazepanyl, dithiolanyl, dihydroquinolinyl, dihydropyrrolyl, dioxanyl, dioxolanyl, dioxepanyl, dihydroindenyl, dihydropyridinyl, dihydrofuranyl, dihydroisoquinolinyl, dihydroindolinyl, dihydroisoindolyl, imidazolidinyl, isoxazolidinyl, morpholinyl, oxiranyl, oxetanyl, oxazepanyl, pyrrolidinyl, piperazinyl, 4-methylpiperazinyl, piperidinyl, pyrazolidinyl, pyranyl, tetrahydropyrrolyl, tetrahydropyranyl, tetrahydroquinolinyl, tetrahydroisoquinolinyl, tetrahydroindolinyl, tetrahydrofuranyl, tetrahydropyridinyl, tetrahydrothiophenyl, tetrahydropyridoindolyl, tetrahydronaphthyl, tetrahydrocarbolinyl, tetrahydroisoxazolopyridinyl, thiazolidinyl, tetrahydroimidazo[1,2-a]pyrazinyl, octahydropyrrolo[1,2-a]pyrazinyl and thiomorpholinyl. More preferred heterocycloaliphatic residues are pyrrolidinyl, piperidinyl, oxazepanyl, azetidinyl, morpholinyl, piperazinyl, tetrahydroquinolinyl, tetrahydroisoquinolinyl, dihydroindolinyl, and dihydroisoindolyl. Most preferred heterocycloaliphatic residues are pyrrolidinyl, piperidinyl, oxazepanyl, azetidinyl, tetrahydroquinolinyl, tetrahydroisoquinolinyl, dihydroindolinyl, and dihydroisoindolyl.

The term "aryl" preferably means for the purpose of this invention aromatic hydrocarbons having 6 to 14 ring members, i.e. 6, 7, 8, 9, 10, 11, 12, 13 or 14 ring members, including phenyls and naphthyls. Each aryl residue can be unsubstituted or mono- or polysubstituted, wherein the aryl substituents can be the same or different and in any desired and possible position of the aryl. The aryl can be bound to the superordinate general structure via any desired and possible ring member of the aryl residue. The aryl residues can also be condensed with further saturated, (partially) unsaturated, (hetero)cycloaliphatic, aromatic or heteroaromatic ring systems, i.e. with a cycloaliphatic, heterocycloaliphatic, aryl or heteroaryl residue, which can in turn be unsubstituted or mono- or polysubstituted. Examples of condensed aryl residues are benzodioxolanyl and benzodioxanyl. Preferably, aryl is selected from the group consisting of phenyl, 1-naphthyl, 2-naphthyl, fluorenyl and anthracenyl, each of which can be respectively unsubstituted or mono- or polysubstituted. A particularly preferred aryl is phenyl, unsubstituted or mono- or polysubstituted.

The term "heteroaryl" for the purpose of this invention preferably represents a 5 or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted; in the case of substitution on the heteroaryl, the substituents can be the same or different and be in any desired and possible position of the heteroaryl. The binding to the superordinate general structure can be carried out via any desired and possible ring member of the heteroaryl residue. The heteroaryl can also be part of a bi- or polycyclic system having up to 14 ring members, wherein the ring system can be formed with further saturated, (partially) unsaturated, (hetero)cycloaliphatic or aromatic or heteroaromatic rings, i.e. with a cycloaliphatic, heterocycloaliphatic, aryl or heteroaryl residue, which can in turn be unsubstituted or mono- or polysubstituted. It is preferable for the heteroaryl residue to be selected from the group consisting of benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, furyl (furanyl), imidazolyl, imidazothiazolyl, indazolyl, indolizinyl, indolyl, isoquinolinyl, isoxazoyl, isothiazolyl, indolyl, naphthyridinyl, oxazolyl, oxadiazolyl, phenazinyl, phenothiazinyl, phthalazinyl, pyrazolyl, pyridyl

(2-pyridyl, 3-pyridyl, 4-pyridyl), pyrrolyl, pyridazinyl, pyrimidinyl, pyrazinyl, purinyl, phenazinyl, thienyl (thiophenyl), triazolyl, tetrazolyl, thiazolyl, thiadiazolyl and triazinyl. Furyl, pyridyl, oxazolyl, thiazolyl and thienyl are particularly preferred.

The term "cycloaliphatic residue bridged via a $C_{1-4}$-aliphatic group" preferably means for the purpose of the invention that the expression "cycloaliphatic residue" has the above-defined meaning and that said residue is bound to the respective superordinate general structure via a $C_{1-4}$-aliphatic group. The $C_{1-4}$ aliphatic group can in all cases be branched or unbranched, unsubstituted or mono- or polysubstituted. The $C_{1-4}$ aliphatic group can in all cases be furthermore saturated or unsaturated, i.e. can be a $C_{1-4}$ alkylene group, a $C_{2-4}$ alkenylene group or a $C_{2-4}$ alkynylene group. Preferably, the $C_{1-4}$-aliphatic group is a $C_{1-4}$ alkylene group or a $C_{2-4}$ alkenylene group, more preferably a $C_{1-4}$ alkylene group. Preferred $C_{1-4}$ alkylene groups are selected from the group consisting of $-CH_2-$, $-CH_2-CH_2-$, $-CH(CH_3)-$, $-CH_2-CH_2-CH_2-$, $-CH(CH_3)-CH_2-$, $-CH(CH_2CH_3)-$, $-CH_2-(CH_2)_2-CH_2-$, $-CH(CH_3)-CH_2-CH_2-$, $-CH_2-CH(CH_3)-CH_2-$, $-CH(CH_3)-CH(CH_3)-$, $-CH(CH_2CH_3)-CH_2-$, $-C(CH_3)_2-CH_2-$, $-CH(CH_2CH_2CH_3)-$ and $-C(CH_3)(CH_2CH_3)-$. Preferred $C_{2-4}$ alkenylene groups are selected from the group consisting of $-CH=CH-$, $-CH=CH-CH_2-$, $-C(CH_3)=CH_2-$, $-CH=CH-CH_2-CH_2-$, $-CH_2-CH=CH-CH_2-$, $-CH=CH-CH=CH-$, $-C(CH_3)=CH-CH_2-$, $-CH=C(CH_3)-CH_2-$, $-C(CH_3)=C(CH_3)-$ and $-C(CH_2CH_3)=CH-$. Preferred $C_{2-4}$ alkynylene groups are selected from the group consisting of $-C\equiv C-$, $-C\equiv C-CH_2-$, $-C\equiv C-CH_2-CH_2-$, $-C\equiv C-CH(CH_3)-$, $-CH_2-C\equiv C-CH_2-$ and $-C=C-C=C-$.

In relation to "aliphatic residue" and "aliphatic group" the term "mono- or polysubstituted" preferably refers in the sense of this invention, with respect to the corresponding residues or groups, to the single substitution or multiple substitution, e.g. disubstitution, trisubstitution and tetrasubstitution, of one or more hydrogen atoms each independently of one another by at least one substituent selected from the group consisting of F, Cl, Br, I, $NO_2$, $NH_2$, an $NH(C_{1-4}$ aliphatic residue), an $N(C_{1-4}$ aliphatic residue$)_2$, a $NH-C(=O)-C_{1-4}$ aliphatic residue, a $N(C_{1-4}$ aliphatic residue$)-C(=O)-C_{1-4}$ aliphatic residue, a $NH-S(=O)_2-C_{1-4}$ aliphatic residue, $N(C_{1-4}$ aliphatic residue$)-S(=O)_2-C_{1-4}$ aliphatic residue, =O, OH, $OCH_2F$, $OCHF_2$, $OCF_3$, a $O-C_{1-4}$-aliphatic residue, a $O-C(=O)-C_{1-4}$-aliphatic residue, SH, $SCF_3$, a $S-C_{1-4}$-aliphatic residue, $S(=O)_2OH$, a $S(=O)-C_{1-4}$-aliphatic residue, a $S(=O)_2-C_{1-4}$-aliphatic residue, a $S(=O)_2-O-C_{1-4}$-aliphatic residue, a $S(=O)_2-NH-C_{1-4}$-aliphatic residue, a $S(=O)_2-N(C_{1-4}$-aliphatic residue$)_2$, CN, $CH_2F$, $CHF_2$, $CF_3$, CHO, COOH, a $C_{1-4}$-aliphatic residue, $CH_2OH$, $CH_2-OCH_3$, $C_2H_4-OH$, $C_2H_4-OCH_3$ $CH_2-CF_3$, a $C(=O)-C_{1-4}$-aliphatic residue, a $C(=O)-O-C_{1-4}$-aliphatic residue, a $C_{3-6}$-cycloaliphatic residue, a 3 to 6 membered heterocycloaliphatic residue, $C(=O)-NH_2$, a $C(=O)-NH(C_{1-4}$ aliphatic residue), and a $C(=O)-N(C_{1-4}$ aliphatic residue$)_2$. The term "polysubstituted" with respect to polysubstituted residues and groups includes the polysubstitution of these residues and groups either on different or on the same atoms, for example trisubstituted on the same carbon atom, as in the case of $CF_3$ or $CH_2CF_3$, or at various points, as in the case of $CH(OH)-CH=CH-CHCl_2$. A substituent can if appropriate for its part in turn be mono- or polysubstituted. The multiple substitution can be carried out using the same or using different substituents.

In relation to "cycloaliphatic residue" and "heterocycloaliphatic residue" the term "mono- or polysubstituted" preferably refers in the sense of this invention, with respect to the corresponding residues, to the single substitution or multiple substitution, e.g. disubstitution, trisubstitution and tetrasubstitution, of one or more hydrogen atoms each independently of one another by at least one substituent selected from the group consisting of F, Cl, Br, I, $NO_2$, $NH_2$, an $NH(C_{1-4}$ aliphatic residue), an $N(C_{1-4}$ aliphatic residue$)_2$, a $NH-C(=O)-C_{1-4}$ aliphatic residue, a $N(_{1-4}$ aliphatic residue$)-C(=O)-C_{1-4}$ aliphatic residue, a $NH-S(=O)_2-C_{1-4}$ aliphatic residue, a $N(C_{1-4}$ aliphatic residue$)-S(=O)_2-C_{1-4}$ aliphatic residue, =O, OH, $OCH_2F$, $OCHF_2$, $OCF_3$, a $O-C_{1-4}$-aliphatic residue, a $O-C(=O)-C_{1-4}$-aliphatic residue, SH, $SCF_3$, a $S-C_{1-4}$-aliphatic residue, $S(=O)_2OH$, a $S(=O)-C_{1-4}$-aliphatic residue, a $S(=O)_2-C_{1-4}$-aliphatic residue, a $S(=O)_2-O-C_{1-4}$-aliphatic residue, a $S(=O)_2-NH-C_{1-4}$-aliphatic residue, a $S(=O)_2-N(C_{1-4}$-aliphatic residue$)_2$, CN, $CH_2F$, $CHF_2$, $CF_3$, CHO, COOH, a $C_{1-4}$-aliphatic residue, $CH_2OH$, $CH_2-OCH_3$, $C_2H_4-OH$, $C_2H_4-OCH_3$ $CH_2-CF_3$, a $C(=O)-C_{1-4}$-aliphatic residue, a $C(=O)-O-C_{1-4}$-aliphatic residue, a $C_{3-6}$-cycloaliphatic residue, a 3 to 6 membered heterocycloaliphatic residue, $C(=O)-NH_2$, a $C(=O)-NH(C_{1-4}$ aliphatic residue), and a $C(=O)-N(C_{1-4}$ aliphatic residue$)_2$. The term "polysubstituted" with respect to polysubstituted residues and groups includes the polysubstitution of these residues and groups either on different or on the same atoms, for example disubstituted on the same carbon atom, as in the case of 1,1-difluorocyclohexyl, or at various points, as in the case of 1-chloro-3-fluorocyclohexyl. A substituent can if appropriate for its part in turn be mono- or polysubstituted. The multiple substitution can be carried out using the same or using different substituents.

Preferred substituents of "aliphatic residue" and "aliphatic group" are selected from the group consisting of F, Cl, Br, I, $NO_2$, $NH_2$, an $NH(C_{1-4}$ aliphatic residue), an $N(C_{1-4}$ aliphatic residue$)_2$, =O, OH, $OCH_2F$, $OCHF_2$, $OCF_3$, a $O-C_{1-4}$-aliphatic residue, SH, $SCF_3$, a $S-C_{1-4}$-aliphatic residue, a $S(=O)-C_{1-4}$-aliphatic residue, a $S(=O)_2-C_{1-4}$-aliphatic residue, a $S(=O)_2-NH-C_{1-4}$-aliphatic residue, a $S(=O)_2-N(C_{1-4}$-aliphatic residue$)_2$, CN, $CH_2F$, $CHF_2$, $CF_3$, a $C_{1-4}$-aliphatic residue, $CH_2OH$, $CH_2-OCH_3$, $C_2H_4-OH$, $C_2H_4-OCH_3$ $CH_2-CF_3$, a $C(=O)-C_{1-4}$-aliphatic residue, a $C(=O)-O-C_{1-4}$-aliphatic residue, $CONH_2$, a $C(=O)-NH(C_{1-4}$ aliphatic residue), and a $C(=O)-N(C_{1-4}$ aliphatic residue$)_2$.

Preferred substituents of "cycloaliphatic residue" and "heterocycloaliphatic residue" are selected from the group consisting of F, Cl, Br, I, $NO_2$, $NH_2$, an $NH(C_{1-4}$ aliphatic residue), an $N(C_{1-4}$ aliphatic residue$)_2$, =O, OH, $OCH_2F$, $OCHF_2$, $OCF_3$, a $O-C_{1-4}$-aliphatic residue, SH, $SCF_3$, a $S-C_{1-4}$-aliphatic residue, a $S(=O)-C_{1-4}$-aliphatic residue, a $S(=O)_2-C_{1-4}$-aliphatic residue, a $S(=O)_2-NH-C_{1-4}$-aliphatic residue, a $S(=O)_2-N(C_{1-4}$-aliphatic residue$)_2$, CN, $CH_2F$, $CHF_2$,

$CF_3$, a $C_{1-4}$-aliphatic residue, $CH_2OH$, $CH_2$-$OCH_3$, $C_2H_4$-OH, $C_2H_4$-$OCH_3$ $CH_2$-$CF_3$, a C(=O)-$C_{1-4}$-aliphatic residue, a C(=O)-O-$C_{1-4}$-aliphatic residue, $CONH_2$, a C(=O)-NH($C_{1-4}$ aliphatic residue), and a C(=O)-N($C_{1-4}$ aliphatic residue)$_2$.

The compounds according to the invention are defined by substituents, for example by $R^1$, $R^2$ and $R^3$ (1$^{st}$ generation substituents) which are for their part if appropriate substituted (2$^{nd}$ generation substituents). Depending on the definition, these substituents of the substituents can for their part be re-substituted (3$^{rd}$ generation substituents). If, for example, $R^1$ is = a $C_{2-6}$ aliphatic residue (1$^{st}$ generation substituent), then the $C_{2-6}$ aliphatic residue can for its part be substituted, for example with a NH-$C_{1-4}$ aliphatic residue (2$^{nd}$ generation substituent). This produces the functional group $R^1$ = ($C_{2-6}$ aliphatic residue-NH-$C_{1-4}$ aliphatic residue). The NH-$C_{1-4}$ aliphatic residue can then for its part be resubstituted, for example with Cl (3$^{rd}$ generation substituent). Overall, this produces the functional group $R^1$ = $C_{2-6}$ aliphatic residue-NH-$C_{1-4}$ aliphatic residue, wherein the $C_{1-4}$ aliphatic residue of the NH-$C_{1-4}$ aliphatic residue is substituted by Cl.

However, in a preferred embodiment, the 3$^{rd}$ generation substituents may not be resubstituted, i.e. there are then no 4$^{th}$ generation substituents.

In another preferred embodiment, the 2$^{nd}$ generation substituents may not be resubstituted, i.e. there are then not even any 3$^{rd}$ generation substituents. In other words, in this embodiment, in the case of general formula (I), for example, the functional groups for $R^1$ to $R^5$ can each if appropriate be substituted; however, the respective substituents may then for their part not be resubstituted.

In some cases, the compounds according to the invention are defined by substituents which are or carry an aryl or heteroaryl residue, respectively unsubstituted or mono- or polysubstituted, or which form together with the carbon atom(s) or heteroatom(s) connecting them, as the ring member or as the ring members, a ring, for example an aryl or heteroaryl, in each case unsubstituted or mono- or polysubstituted. Both these aryl or heteroaryl residues and the (hetero)aromatic ring systems formed in this way can if appropriate be condensed with a cycloaliphatic, preferably a $C_{3-6}$ cycloaliphatic residue, or heterocycloaliphatic residue, preferably a 3 to 6 membered heterocycloaliphatic residue, or with aryl or heteroaryl, e.g. with a $C_{3-6}$ cycloaliphatic residue such as cyclopentyl, or a 3 to 6 membered heterocycloaliphatic residue such as morpholinyl, or an aryl such as phenyl, or a heteroaryl such as pyridyl, wherein the cycloaliphatic or heterocycloaliphatic residues, aryl or heteroaryl residues condensed in this way can for their part be respectively unsubstituted or mono- or polysubstituted.

In some cases, the compounds according to the invention are defined by substituents which are or carry a cycloaliphatic residue or a heterocycloaliphatic residue, respectively, in each case unsubstituted or mono- or polysubstituted, or which form together with the carbon atom(s) or heteroatom(s) connecting them, as the ring member or as the ring members, a ring, for example a cycloaliphatic or a heterocycloaliphatic ring system. Both these cycloaliphatic or heterocycloaliphatic ring systems and the (hetero)cycloaliphatic ring systems formed in this manner can if appropriate be condensed with aryl or heteroaryl, preferably selected from the group consisting of phenyl, pyridyl and thienyl, or with a cycloaliphatic residue, preferably a $C_{3-6}$ cycloaliphatic residue, or a heterocycloaliphatic residue, preferably a 3 to 6 membered heterocycloaliphatic residue, e.g. with an aryl such as phenyl, or a heteroaryl such as pyridyl, or a cycloaliphatic residue such as cyclohexyl, or a heterocycloaliphatic residue such as morpholinyl, wherein the aryl or heteroaryl residues or cycloaliphatic or heterocycloaliphatic residues condensed in this way can for their part be respectively unsubstituted or mono- or polysubstituted.

Within the scope of the present invention, the symbol

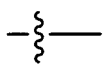

used in the formulae denotes a link of a corresponding residue to the respective superordinate general structure.

If a residue occurs multiply within a molecule, then this residue can have respectively different meanings for various substituents: if, for example, both $R^2$ and $R^3$ denote a $C_{1-4}$-aliphatic residue, then the $C_{1-4}$-aliphatic residue residue can e.g. represent methyl for $R^2$ and can represent ethyl for $R^3$.

In a preferred embodiment of the compound according to general formula (I) the particular radicals $R^2$-$R^5$ have the meanings described herein in connection with the compounds according to the invention and preferred embodiments thereof and

$R^1$ represents a $C_{2-6}$-aliphatic residue, unsubstituted or mono- or polysubstituted; or a $C_{3-6}$-cycloaliphatic residue, unsubstituted or mono- or polysubstituted, optionally bridged via a $C_{1-4}$ aliphatic group, which in turn may be unsubstituted or mono- or polysubstituted.

In another preferred embodiment of the compound according to general formula (I) the particular radicals $R^2$-$R^5$ have the meanings described herein in connection with the compounds according to the invention and preferred em-

bodiments thereof and

$R^1$ represents $OR^6$, wherein $R^6$ represents a $C_{1-6}$-aliphatic residue, unsubstituted or mono- or polysubstituted.

Preferred embodiments of the compound according to general formula (I) are compounds which have the general formulae **(I-a)** and/or **(I-b):**

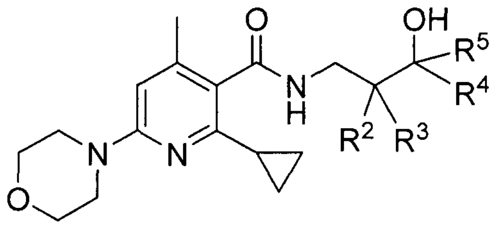

(**I-a**),

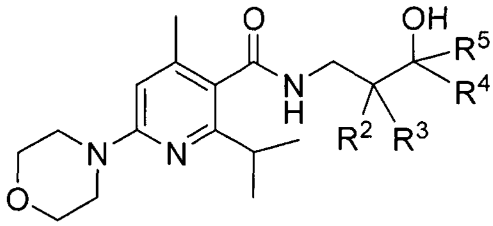

(**I-b**),

wherein the particular radicals $R^2$, $R^3$, $R^4$, and $R^5$, have the meanings described herein in connection with the compounds according to the invention and preferred embodiments thereof.

Another preferred embodiment of the compound according to general formula (I) is a compound which has the general formula **(I-c),**

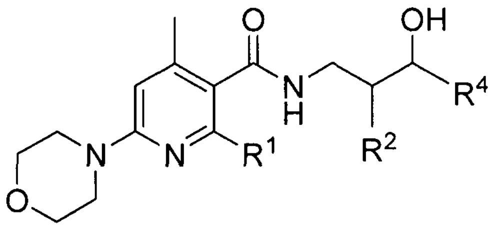

(**I-c**),

wherein the particular radicals $R^1$, $R^2$ and $R^4$ have the meanings described herein in connection with the compounds according to the invention and preferred embodiments thereof.

Further preferred embodiments of the compound according to general formula (I) are compounds which have the general formula **(I-d)** and/or **I-e),**

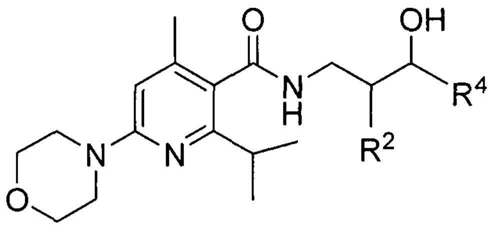

(**I-d**)

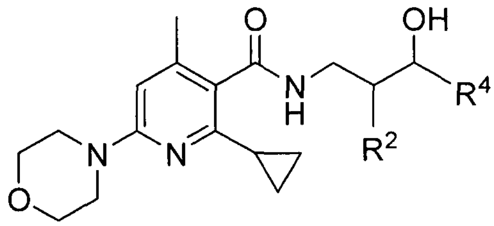

(**I-e**),

wherein the particular radicals $R^2$ and $R^4$ have the meanings described herein in connection with the compounds according to the invention and preferred embodiments thereof.

Further preferred embodiments of the compound according to general formula (I) are compounds which have the general formula (**I-f**) and/or **(I-g),**

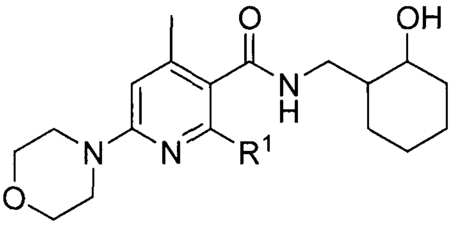

**(I-f)**

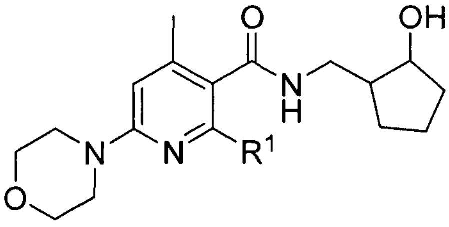

**(I-g)**,

wherein the particular radical $R^1$ has the meanings described herein in connection with the compounds according to the invention and preferred embodiments thereof.

Yet another preferred embodiment of present invention is a compound according to general formula (I), wherein

$R^1$ represents a $C_{2-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue, wherein the $C_{1-4}$-aliphatic residue in each case may be unsubstituted or mono-or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents a $C_{3-6}$-cycloaliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,
and wherein the $C_{3-6}$-cycloaliphatic residue may optionally be bridged via a $C_{1-4}$ aliphatic group, which in turn may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,
or represents $OR^6$, wherein
$R^6$ represents a $C_{1-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue;
wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,
$R^2$ and $R^3$ independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,
wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,
or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,
with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected

from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

on the condition that if $R^4$ and/or $R^5$ denote(s) a 3 to 6 membered heterocycloaliphatic residue, the 3 to 6 membered heterocycloaliphatic residue is linked via a carbon atom,

or

$R^2$ and $R^3$

and/or $R^4$ and $R^5$

in pairs, in each case independently of one another, together with the carbon atom connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the respective remaining substituents of $R^2$ and $R^3$ each independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected

from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

with the proviso that at least one of $R^2$ and $R^3$ denotes H or is linked via a carbon atom,

and the respective remaining substituents of $R^4$ and $R^5$ each independently of one another represent H; $CH_2F$; $CHF_2$; $CF_3$; a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

on the condition that if $R^4$ and/or $R^5$ denote(s) a 3 to 6 membered heterocycloaliphatic residue, the 3 to 6 membered heterocycloaliphatic residue is linked via a carbon atom,

or

$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the remaining substituent $R^3$ represents H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the remaining substituent $R^5$ represents H; $CH_2F$; $CHF_2$; $CF_3$; a $C_{1-4}$-aliphatic residue,

wherein the $C_{1-4}$-aliphatic residue may be unsubstituted or mono- or polysubstituted with at least one substituent selected from the group consisting of F, Cl, Br, I, OH,

$OCH_2F$, $OCHF_2$, $OCF_3$, $CH_2F$, $CHF_2$, $CF_3$ and an unsubstituted O-$C_{1-4}$-aliphatic residue,

or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

on the condition that if $R^5$ denotes a 3 to 6 membered heterocycloaliphatic residue, the 3 to 6 membered heterocycloaliphatic residue is linked via a carbon atom.

In another preferred embodiment of the compound according to general formula (I), the residue

$R^1$ represents a $C_{2-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono-or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents a $C_{3-6}$-cycloaliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono-or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents $OR^6$, wherein

$R^6$ represents a $C_{1-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, .$OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue.

Preferably,

$R^1$ represents a $C_{2-6}$-aliphatic residue, unsubstituted or mono- or polysubstituted with at least one substituent selected

from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue is in each case unsubstituted, or represents a $C_{3-6}$-cycloaliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,
wherein the $C_{1-4}$-aliphatic residue is in each case unsubstituted,
or represents $OR^6$, wherein
$R^6$ represents a $C_{1-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,
wherein the $C_{1-4}$-aliphatic residue is in each case unsubstituted.

More preferably,

$R^1$ represents a $C_{2-6}$-aliphatic residue, unsubstituted or mono- or polysubstituted with at least one substituent selected from the group consisting of F, Cl, Br, I, OH, and an unsubstituted O-$C_{1-4}$-aliphatic residue,

or represents a $C_{3-6}$-cycloaliphatic residue, unsubstituted or mono- or polysubstituted with at least one substituent selected from the group consisting of F, Cl, Br, I, OH, and an unsubstituted O-$C_{1-4}$-aliphatic residue,
or represents $OR^6$, wherein
$R^6$ represents a $C_{1-4}$-aliphatic residue, unsubstituted or mono- or polysubstituted with at least one substituent selected from the group consisting of F, Cl, Br, I, OH, and an unsubstituted O-$C_{1-4}$-aliphatic residue.

Even more preferably,
$R^1$ represents ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, ethenyl or propenyl (-$CH_2CH=CH_2$, -$CH=CH-CH_3$, -$C(=CH_2)-CH_3$),
or represents a $C_{3-6}$-cycloaliphatic residue, preferably selected from the group consisting of cyclopropyl, cyclobutyl, cyclopentyl, and cyclohexyl, unsubstituted or mono- or polysubstituted with at least one substituent selected from the group consisting of F, Cl, Br, I, OH, and an unsubstituted O-$C_{1-4}$-aliphatic residue, preferably unsubstituted,
or represents $OR^6$, wherein
$R^6$ represents methyl, ethyl, n-propyl, 2-propyl, n-butyl, isobutyl, sec.-butyl, tert.-butyl, $CH_2OH$, $CH_2OCH_3$, $CH_2CH_2OH$, $CH_2CH_2OCH_3$, and $CH(OH)CH_2OH$.

In particular,

$R^1$ represents ethyl, n-propyl, 2-propyl, n-butyl, isobutyl, sec.-butyl, tert.-butyl, cyclopropyl, cyclobutyl, cyclopentyl, or cyclohexyl,

or represents $OR^6$, wherein $R^6$ represents methyl, ethyl, n-propyl, 2-propyl, n-butyl, isobutyl, sec.-butyl, or tert.-butyl, preferably represents ethyl, n-propyl, 2-propyl, n-butyl, isobutyl, sec.-butyl, tert.-butyl, cyclopropyl, cyclobutyl, cyclopentyl, or cyclohexyl.

Most preferred,

$R^1$ represents ethyl, n-propyl, 2-propyl, tert.-butyl, or cyclopropyl.

In another preferred embodiment of the compound according to general formula (I), the residues
$R^2$ and $R^3$ independently of one another represent H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,

with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^3$
and/or $R^4$ and $R^5$, preferably $R^2$ and $R^3$ or $R^4$ and $R^5$,
in pairs, in each case independently of one another, together with the carbon atom connecting them, form a $C_{3-10}$-cycloaliphatic residue or a 3 to 10 membered heterocycloaliphatic residue, preferably a $C_{3-10}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,,
and the respective remaining substituents of $R^2$ and $R^3$ each independently of one another represent H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^4$ together with the carbon atoms connecting them, form a $C_{3-10}$-cycloaliphatic residue or a 3 to 10 membered heterocycloaliphatic residue, preferably a $C_{3-10}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
and the remaining substituent $R^3$ represents H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
or represents a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a

$C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
and the remaining substituent $R^5$ represents H; $FCH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue,
or represents a $C_{3-6}$-cycloaliphatic residue or a 3 to 6 membered heterocycloaliphatic residue, preferably a $C_{3-6}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono- or polysubstituted with at least one substituent selected from the group consisting of F, Cl, Br, I, OH, and an unsubstituted O-$C_{1-4}$-aliphatic residue,
on the condition that if $R^5$ denotes a 3 to 6 membered heterocycloaliphatic residue, the 3 to 6 membered heterocycloaliphatic residue is linked via a carbon atom.

Preferably,
$R^2$ and $R^3$ independently of one another represent H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each case unsubstituted,
or represent a $C_{3-6}$-cycloaliphatic residue or a 3 to 6 membered heterocycloaliphatic residue, preferably a $C_{3-6}$-cycloaliphatic residue, in each case unsubstituted,
with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue,
wherein the $C_{1-4}$-aliphatic residue is unsubstituted,
or represent a $C_{3-6}$-cycloaliphatic residue or a 3 to 6 membered heterocycloaliphatic residue, preferably a $C_{3-6}$-cycloaliphatic residue, in each case unsubstituted,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^3$
and/or $R^4$ and $R^5$, preferably $R^2$ and $R^3$ or $R^4$ and $R^5$,
in pairs, in each case independently of one another, together with the carbon atom connecting them, form a $C_{3-10}$-cycloaliphatic residue or a 3 to 10 membered heterocycloaliphatic residue, preferably a $C_{3-10}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, and a $C_{1-4}$-aliphatic residue,
wherein the $C_{1-4}$-aliphatic residue is in each case unsubstituted,
and the respective remaining substituents of $R^2$ and $R^3$ each independently of one another represent H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each unsubstituted,
or represent a $C_{3-6}$-cycloaliphatic residue or a 3 to 6 membered heterocycloaliphatic residue, preferably a $C_{3-6}$-cycloaliphatic residue, in each case unsubstituted,
with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue,
wherein the $C_{1-4}$-aliphatic residue is in each unsubstituted,
or represent a $C_{3-6}$-cycloaliphatic residue or a 3 to 6 membered heterocycloaliphatic residue, preferably a $C_{3-6}$-cycloaliphatic residue, in each case unsubstituted,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^4$ together with the carbon atoms connecting them, form a $C_{3-10}$-cycloaliphatic residue or a 3 to 10 membered heterocycloaliphatic residue, preferably a $C_{3-10}$-cycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, and a $C_{1-4}$-aliphatic residue,
wherein the $C_{1-4}$-aliphatic residue is in each case unsubstituted,
and the remaining substituent $R^3$ represents H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each case unsubstituted,
or represents a $C_{3-6}$-cycloaliphatic residue or a 3 to 6 membered heterocycloaliphatic residue, preferably a $C_{3-6}$-cy-

cloaliphatic residue, in each case unsubstituted, and the remaining substituent $R^5$ represents H; $CH_2F$; $CHF_2$; $CF_3$; a $C_{1-4}$-aliphatic residue, wherein the $C_{1-4}$-aliphatic residue is in each case unsubstituted, or represents a $C_{3-6}$-cycloaliphatic residue or a 3 to 6 membered heterocycloaliphatic residue, preferably a $C_{3-6}$-cycloaliphatic residue, in each case unsubstituted, on the condition that if $R^5$ denotes a 3 to 6 membered heterocycloaliphatic residue, the 3 to 6 membered heterocycloaliphatic residue is linked via a carbon atom.

More preferably,
$R^2$ and $R^3$ independently of one another represent H; OH; an unsubstituted $C_{1-4}$-aliphatic residue, or an unsubstituted O-$C_{1-4}$ aliphatic residue, with the proviso that at least one of $R^2$ and $R^3$ denotes H or is linked via a carbon atom,
$R^4$ and $R^5$ independently of one another represent H; $CH_2F$; $CHF_2$; $CF_3$; an unsubstituted $C_{1-4}$-aliphatic residue,
or
$R^2$ and $R^3$
or $R^4$ and $R^5$
in pairs, in each case independently of one another, together with the carbon atom connecting them, form a $C_{3-10}$-cycloaliphatic residue, preferably a $C_{3-6}$-cycloaliphatic residue, unsubstituted or mono- or polysubstituted with at least one substituent selected from the group consisting of F, Cl, Br, I, OH, $CH_2F$, $CHF_2$, $CF_3$, an unsubstituted O-$C_{1-4}$-aliphatic residue, and an unsubstituted $C_{1-4}$-aliphatic residue,
and the respective remaining substituents of $R^2$ and $R^3$ each independently of one another represent H; OH; an unsubstituted $C_{1-4}$-aliphatic residue, or an unsubstituted O-$C_{1-4}$ aliphatic residue, with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; an unsubstituted $C_{1-4}$-aliphatic residue,
or
$R^2$ and $R^4$ together with the carbon atoms connecting them, form a $C_{3-10}$-cycloaliphatic residue, preferably a $C_{3-6}$-cycloaliphatic residue, unsubstituted or mono- or polysubstituted with at least one substituent selected from the group consisting of F, Cl, Br, I, OH, an unsubstituted O-$C_{1-4}$-aliphatic residue, $CH_2F$, $CHF_2$, $CF_3$, and a an unsubstituted $C_{1-4}$-aliphatic residue,
and the remaining substituent $R^3$ represents H; F; Cl; Br; I; OH; an unsubstituted $C_{1-4}$-aliphatic residue, or an unsubstituted O-$C_{1-4}$ aliphatic residue,
and the remaining substituent $R^5$ represents H; $CH_2F$; $CHF_2$; $CF_3$; an unsubstituted $C_{1-4}$-aliphatic residue.

Even more preferably,
$R^2$ and $R^3$ independently of one another represent H; OH; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,; preferably represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,
$R^4$ and $R^5$ independently of one another represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$;,
or
$R^2$ and $R^3$
or $R^4$ and $R^5$
in pairs, in each case independently of one another, together with the carbon atom connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of F, Cl, Br, I, OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and the respective remaining substituents of $R^2$ and $R^3$ each independently of one another represents H; OH; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,preferably represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,
and the respective remaining substituents of $R^4$ and $R^5$ each independently of one another represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$;,
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least

one substituent selected from the group consisting of F, Cl, Br, I, OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and $R^3$ represents H; OH; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, preferably represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$,
and $R^5$ represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$.

Still more preferably,
$R^2$ and $R^3$ independently of one another represent H; OH; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,preferably represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,
$R^4$ and $R^5$ independently of one another represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$;,
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of F, Cl, Br, I, OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and the respective remaining substituent $R^3$ represents H; OH; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, preferably represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$,
and the respective remaining substituents $R^5$ represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$.

In another preferred embodiment of the compound according to general formula (I), at least one of $R^2$, $R^3$, $R^4$ and $R^5$ denotes H, preferably at least two of $R^2$, $R^3$, $R^4$ and $R^5$ denote H.

In yet another preferred embodiment of the compound according to general formula (I), at least one of $R^3$ and $R^5$ denotes H, preferably both $R^3$ and $R^5$ denote H.

In a further preferred embodiment of the compound according to general formula (I),

$R^1$ represents ethyl, n-propyl, 2-propyl, n-butyl, isobutyl, sec.-butyl, tert.-butyl, cyclopropyl, cyclobutyl, cyclopentyl, or cyclohexyl,

or represents $OR^6$, wherein
$R^6$ represents methyl, ethyl, n-propyl, 2-propyl, n-butyl, isobutyl, sec.-butyl, or tert.-butyl,
preferably represents ethyl, n-propyl, 2-propyl, n-butyl, isobutyl, sec.-butyl, tert.-butyl, cyclopropyl, cyclobutyl, cyclopentyl, or cyclohexyl,
$R^2$ and $R^3$ independently of one another represent H; methyl; ethyl, n-propyl, 2-propyl, n-butyl, isobutyl, sec.-butyl, or tert.-butyl,
preferably $R^2$ is selected from the group consisting of methyl; ethyl, n-propyl, 2-propyl, n-butyl, isobutyl, sec.-butyl, or tert.-butyl, and $R^3$ denotes H,
$R^4$ and $R^5$ independently of one another represent H; methyl; ethyl, n-propyl, 2-propyl, n-butyl, isobutyl, sec.-butyl, tert.-butyl, $CH_2F$, $CHF_2$, or $CF_3$;
preferably $R^4$ is selected from the group consisting of methyl; ethyl, n-propyl, 2-propyl, n-butyl, isobutyl, sec.-butyl, tert.-butyl, $CH_2F$, $CHF_2$, and $CF_3$; and $R^5$ denotes H,
or
$R^2$ and $R^3$ or $R^4$ and $R^5$
in pairs, in each case independently of one another, together with the carbon atom connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of OH, methyl; ethyl, n-propyl, 2-propyl, n-butyl, isobutyl, sec.-butyl, $CH_2F$, $CHF_2$, $CF_3$, $OCH_3$ and $OCH_2CH_3$,
and the respective remaining substituents of $R^2$ and $R^3$ in dependently of one another represent H; methyl; ethyl, n-propyl, 2-propyl, n-butyl, isobutyl, sec.-butyl, or tert.-butyl,
preferably $R^2$ is selected from the group consisting of methyl; ethyl, n-propyl, 2-propyl, n-butyl, isobutyl, sec.-butyl, or tert.-butyl, and $R^3$ denotes H,

and the respective remaining substituents of $R^4$ and $R^5$ independently of one another represent H; methyl; ethyl, n-propyl, 2-propyl, n-butyl, isobutyl, sec.-butyl, tert.-butyl, $CH_2F$, $CHF_2$, or $CF_3$;
preferably $R^4$ is selected from the group consisting of methyl; ethyl, n-propyl, 2-propyl, n-butyl, isobutyl, sec.-butyl, tert.-butyl, $CH_2F$, $CHF_2$, and $CF_3$; and $R^5$ denotes H,
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and $R^3$ represents H; methyl; ethyl, n-propyl, 2-propyl, n-butyl, isobutyl, sec.-butyl, or tert.-butyl,
preferably $R^3$ denotes H,
and $R^5$ represents H; methyl; ethyl, n-propyl, 2-propyl, n-butyl, isobutyl, sec.-butyl, tert.-butyl, $CH_2F$, $CHF_2$, or $CF_3$,
preferably $R^5$ denotes H.

In a further preferred embodiment of the compound according to general formula (I),

$R^1$ represents ethyl, n-propyl, 2-propyl, n-butyl, isobutyl, sec.-butyl, tert.-butyl, cyclopropyl, cyclobutyl, cyclopentyl, or cyclohexyl,

or represents $OR^6$, wherein
$R^6$ represents methyl, ethyl, n-propyl, 2-propyl, n-butyl, isobutyl, sec.-butyl, or tert.-butyl,
preferably represents ethyl, n-propyl, 2-propyl, n-butyl, isobutyl, sec.-butyl, tert.-butyl, cyclopropyl, cyclobutyl, cyclopentyl, or cyclohexyl,
$R^2$ and $R^3$ independently of one another represent H; methyl; ethyl, n-propyl, 2-propyl, n-butyl, isobutyl, sec.-butyl, or tert.-butyl,
preferably $R^2$ is selected from the group consisting of methyl; ethyl, n-propyl, 2-propyl, n-butyl, isobutyl, sec.-butyl, or tert.-butyl, and $R^3$ denotes H,
$R^4$ and $R^5$ independently of one another represent H; methyl; ethyl, n-propyl, 2-propyl, n-butyl, isobutyl, sec.-butyl, tert.-butyl, $CH_2F$, $CHF_2$, or $CF_3$;
preferably $R^4$ is selected from the group consisting of methyl; ethyl, n-propyl, 2-propyl, n-butyl, isobutyl, sec.-butyl, tert.-butyl, $CH_2F$, $CHF_2$, and $CF_3$, and $R^5$ denotes H.
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and the respective remaining substituent $R^3$ represents H; methyl; ethyl, n-propyl, 2-propyl, n-butyl, isobutyl, sec.-butyl, or tert.-butyl,
preferably $R^3$ denotes H,
and the respective remaining substituent $R^5$ represents H; methyl; ethyl, n-propyl, 2-propyl, n-butyl, isobutyl, sec.-butyl, tert.-butyl, $CH_2F$, $CHF_2$, and $CF_3$,
preferably $R^5$ denotes H.

Particularly preferred are compounds according to general formula (I) selected from the group comprising:

1 2-Cyclopropyl-N-(3-hydroxy-4,4-dimethyl-pentyl)-4-methyl-6-morpholin-4-yl-pyridine-3-carboxylic acid amide;
2 2-Cyclopropyl-N-[[(1S,2R)-2-hydroxy-cyclohexyl]-methyl]-4-methyl-6-morpholin-4-yl-pyridine-3-carboxylic acid amide;
3 2-Cyclopropyl-N-[[(1R,2S)-2-hydroxy-cyclohexyl]-methyl]-4-methyl-6-morpholin-4-yl-pyridine-3-carboxylic acid amide;
4 2-Cyclopropyl-N-([2-hydroxy-cyclopentyl]-methyl)-4-methyl-6-morpholin-4-yl-pyridine-3-carboxylic acid amide;
5 N-(3-Hydroxy-4,4-dimethyl-pentyl)-2-isopropyl-4-methyl-6-morpholin-4-yl-pyridine-3-carboxylic acid amide;
6 2-Isopropyl-4-methyl-6-morpholin-4-yl-N-(4,4,4-trifluoro-3-hydroxy-butyl)-pyridine-3-carboxylic acid amide;
7 N-[[(1S,2R)-2-Hydroxy-cyclohexyl]-methyl]-2-isopropyl-4-methyl-6-morpholin-4-yl-pyridine-3-carboxylic acid amide; and
8 2-Ethoxy-N-(3-hydroxy-4,4-dimethyl-pentyl)-4-methyl-6-morpholin-4-yl-pyridine-3-carboxylic acid amide,

optionally in the form of a single stereoisomer or a mixture of stereoisomers, in the form of the free compound and/or a physiologically acceptable salt thereof.

The substituted compounds according to the invention of the aforementioned general formula (I) and corresponding stereoisomers and also the respective corresponding salts and solvates are toxicologically safe and are therefore suitable as pharmaceutical active ingredients in pharmaceutical compositions.

The present invention therefore further relates to a pharmaceutical composition containing at least one compound according to general formula (I), in each case optionally in the form of a single stereoisomer or a mixture of stereoisomers, in the form of the free compound and/or a physiologically acceptable salt and/or a solvate, in particular hydrate, thereof, and also optionally one or more pharmaceutically acceptable auxiliaries.

These pharmaceutical compositions according to the invention are suitable in particular for the modulation of KCNQ2/3 $K^+$ channels, preferably for KCNQ2/3 $K^+$ channel inhibition and/or KCNQ2/3 $K^+$ channel stimulation, i.e. they exert an agonistic or antagonistic effect.

Likewise, the pharmaceutical compositions according to the invention are preferably suitable for the prophylaxis and/or treatment of disorders and/or diseases which are mediated, at least in part, by KCNQ2/3 $K^+$ channels.

The pharmaceutical composition according to the invention is suitable for administration to adults and children, including toddlers and babies.

The pharmaceutical composition according to the invention may be prepared as a liquid, semisolid or solid pharmaceutical form, for example in the form of injection solutions, drops, juices, syrups, sprays, suspensions, tablets, patches, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and also be administered as much.

In addition to at least one substituted compound of general formula (I), optionally in the form of a single stereoisomer or a mixture of stereoisomers, in the form of the free compound and/or a physiologically acceptable salt and/or a solvate, in particular hydrate, thereof, the pharmaceutical composition according to the invention conventionally may contain further physiologically acceptable pharmaceutical auxiliaries which, for example, can be selected from the group consisting of excipients, fillers, solvents, diluents, surface-active substances, dyes, preservatives, blasting agents, slip additives, lubricants, aromas and binders.

The selection of the physiologically acceptable auxiliaries and also the amounts thereof to be used depend on whether the pharmaceutical composition is to be applied orally, subcutaneously, parenterally, intravenously, intraperitoneally, intradermally, intramuscularly, intranasally, buccally, rectally or locally, for example to infections of the skin, the mucous membranes and of the eyes. Preparations in the form of tablets, dragées, capsules, granules, pellets, drops, juices and syrups are preferably suitable for oral application; solutions, suspensions, easily reconstitutable dry preparations and also sprays are preferably suitable for parenteral, topical and inhalative application. The substituted compounds according to the invention used in the pharmaceutical composition according to the invention in a repository, in a dissolved form or in a plaster, and further agents promoting skin penetration being added if appropriate, are suitable percutaneous application preparations. Orally or percutaneously applicable preparation forms can release the respective substituted compound according to the invention also in a delayed manner.

The pharmaceutical compositions according to the invention can be prepared with the aid of conventional means, devices, methods and process known in the art, such as are described for example in "Remington's Pharmaceutical Sciences", A.R. Gennaro (Editor), 17th edition, Mack Publishing Company, Easton, Pa, 1985, in particular in Part 8, Chapters 76 to 93. The corresponding description is introduced herewith by way of reference and forms part of the disclosure. The amount to be administered to the patient of the respective substituted compounds according to the invention of the above-indicated general formula (I) may vary and is for example dependent on the patient's weight or age and also on the type of application, the indication and the severity of the disorder. Conventionally, 0.001 to 100 mg/kg, preferably 0.05 to 75 mg/kg, particularly preferably 0.05 to 50 mg of at least one compound according to the invention are applied per kg of the patient's body weight.

The pharmaceutical composition according to the invention is preferably suitable for the prophylaxis and/or treatment of disorders and/or diseases which are mediated, at least in part, by KCNQ2/3 $K^+$ channels. The pharmaceutical composition according to the invention is more preferably suitable for the treatment and/or prophylaxis of one or more diseases and/or disorders selected from the group consisting of pain, in particular pain selected from the group consisting of acute pain, chronic pain, neuropathic pain, muscular pain, visceral pain and inflammatory pain, epilepsy, urinary incontinence, anxiety, dependency, mania, bipolar disorders, migraine, cognitive diseases and dystonia-associated dyskinesias.

The pharmaceutical composition according to the invention is suitable particularly preferably for the treatment of pain, more particularly preferably of acute pain, chronic pain, neuropathic pain, visceral pain, inflammatory pain and muscular pain, and most particularly for the treatment of neuropathic pain.

The pharmaceutical composition according to the invention is also preferably suitable for the treatment and/or prophylaxis of epilepsy.

The present invention further relates to at least one compound according to general formula (I), optionally in the form of a single stereoisomer or a mixture of stereoisomers, in the form of the free compound and/or a physiologically acceptable salt and/or a solvate, in particular hydrate, thereof, and also optionally of one or more pharmaceutically

acceptable auxiliaries for use in the modulation of KCNQ2/3 $K^+$ channels, preferably for use in KCNQ2/3 $K^+$ channel inhibition and/or stimulation.

The present invention therefore further relates to at least one compound according to general formula (I), optionally in the form of a single stereoisomer or a mixture of stereoisomers, in the form of the free compound and/or a physiologically acceptable salt and/or a solvate, in particular hydrate, thereof, and also optionally of one or more pharmaceutically acceptable auxiliaries for use in the prophylaxis and/or treatment of disorders and/or diseases which are mediated, at least in part, by KCNQ2/3 $K^+$ channels.

Preference is given to at least one compound according to general formula (I), optionally in the form of a single stereoisomer or a mixture of stereoisomers, in the form of the free compound and/or a physiologically acceptable salt and/or a solvate, in particular hydrate, thereof, and optionally one or more pharmaceutically acceptable auxiliaries for use in the prophylaxis and/or treatment of disorders and/or diseases selected from the group consisting of pain, in particular pain selected from the group consisting of acute pain, chronic pain, neuropathic pain, muscular pain, visceral pain and inflammatory pain, epilepsy, urinary incontinence, anxiety, dependency, mania, bipolar disorders, migraine, cognitive diseases and dystonia-associated dyskinesias.

Particular preference is given to at least one compound according to general formula (I) and optionally one or more pharmaceutically acceptable auxiliaries for use in the prophylaxis and/or treatment of disorders and/or diseases selected from the group consisting of pain, in particular pain selected from the group consisting of acute pain, chronic pain, neuropathic pain, muscular pain, visceral pain and inflammatory pain, most particularly neuropathic pain.

Particular preference is also given to at least one compound according to general formula (I) and optionally one or more pharmaceutically acceptable auxiliaries for use in the prophylaxis and/or treatment of epilepsy.

The present invention further relates to at least one compound according to general formula (I) and also optionally one or more pharmaceutically acceptable auxiliaries for the modulation of KCNQ2/3 $K^+$ channels, preferably for KCNQ2/3 $K^+$ channel inhibition and/or stimulation.

The present invention therefore further relates to at least one compound according to general formula (I) and also optionally of one or more pharmaceutically acceptable auxiliaries for the prophylaxis and/or treatment of disorders and/or diseases which are mediated, at least in part, by KCNQ2/3 $K^+$ channels.

Preference is given to at least one compound according to general formula (I) and optionally one or more pharmaceutically acceptable auxiliaries for the prophylaxis and/or treatment of disorders and/or diseases selected from the group consisting of pain, especially pain selected from the group consisting of acute pain, chronic pain, neuropathic pain, muscular pain, visceral pain and inflammatory pain, epilepsy, urinary incontinence, anxiety, dependency, mania, bipolar disorders, migraine, cognitive diseases and dystonia-associated dyskinesias.

Particular preference is given to at least one compound according to general formula (I) and optionally one or more pharmaceutically acceptable auxiliaries for the prophylaxis and/or treatment of disorders and/or diseases selected from the group consisting of pain, in particular pain selected from the group consisting of acute pain, chronic pain, neuropathic pain, muscular pain, visceral pain and inflammatory pain, most particularly neuropathic pain.

Particular preference is also given to at least one compound according to general formula (I) and optionally one or more pharmaceutically acceptable auxiliaries for the prophylaxis and/or treatment of epilepsy.

The present invention further relates to at least one compound according to general formula (I) and also optionally one or more pharmaceutically acceptable auxiliaries for use in the preparation of a medicament for prophylaxis and/or treatment of disorders and/or diseases which are mediated, at least in part, by KCNQ2/3 $K^+$ channels.

Preference is given to at least one compound according to general formula (I) and optionally one or more pharmaceutically acceptable auxiliaries for use in the preparation of a medicament for the prophylaxis and/or treatment of disorders and/or diseases selected from the group consisting of pain, in particular pain selected from the group consisting of acute pain, chronic pain, neuropathic pain, muscular pain, visceral pain and inflammatory pain, epilepsy, urinary incontinence, anxiety, dependency, mania, bipolar disorders, migraine, cognitive diseases and dystonia-associated dyskinesias.

Particular preference is given to at least one compound according to general formula (I) and optionally one or more pharmaceutically acceptable auxiliaries for use in the preparation of a medicament for the prophylaxis and/or treatment of disorders and/or diseases selected from the group consisting of pain, in particular pain selected from the group consisting of acute pain, chronic pain, neuropathic pain, muscular pain, visceral pain and inflammatory pain, most particularly neuropathic pain.

Particular preference is also given to at least one compound according to general formula (I) and optionally one or more pharmaceutically acceptable auxiliaries for use in the preparation of a medicament for the prophylaxis and/or treatment of epilepsy.

Another aspect of the present invention are the compounds of formula (I) for use in a method of treatment and/or prophylaxis of disorders and/or diseases, which are mediated, at least in part, by KCNQ2/3 $K^+$ channels, in a mammal, preferably of disorders and/or diseases selected from the group consisting of pain, preferably pain selected from the group consisting of acute pain, chronic pain, neuropathic pain, muscular pain, visceral pain and inflammatory

pain, epilepsy, urinary incontinence, anxiety, dependency, mania, bipolar disorders, migraine, cognitive diseases and dystonia-associated dyskinesias, which comprises administering an effective amount of at least one compound of general formula (I) to the mammal.

The effectiveness against pain can be shown, for example, in the *Bennett* or *Chung* model (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363), by tail flick experiments (e.g. according to D'Amour und Smith (J. Pharm. Exp. Ther. 72, 74 79 (1941)) or by the formalin test (e.g. according to D. Dubuisson et al., Pain 1977, 4, 161-174). The effectiveness against epilepsy can be demonstrated, for example, in the *DBA/2 mouse* model (De Sarro et al., Naunyn-Schmiedeberg's Arch. Pharmacol. 2001, 363, 330-336).

The compounds according to the invention preferably have a $EC_{50}$ value of not more than 10000 nM or not more than 8000 nM, more preferably not more than 7000 nM or not more than 6000 nM, yet more preferably not more than 5000 nM or not more than 3000 nM, even more preferably not more than 2000 nM or not more than 1000 nM, yet even more preferably not more than 800 nM or not more than 700 nM, still more preferably not more than 600 nM or not more than 500 nM, yet still more preferably not more than 400 nM or not more than 300 nM, most preferably not more than 200 nM or not more than 150 nM and especially not more than 120 nM or not more than 100 nM. Methods for determining the $EC_{50}$ value are known to the person skilled in the art. The $EC_{50}$ value is preferably determined by fluorimetry, particularly preferably as described below under "pharmacological **experiments".**

The invention further provides processes for the preparation of the substituted compounds according to the invention.

The chemicals and reaction components used in the reactions and schemes described below are available commercially or in each case can be prepared by conventional methods known to the person skilled in the art.

The reactions described can each be carried out under the conventional conditions with which the person skilled in the art is familiar, for example with regard to pressure or the order in which the components are added. If appropriate, the person skilled in the art can determine the optimum procedure under the respective conditions by carrying out simple preliminary tests. The intermediate and end products obtained using the reactions described hereinbefore can each be purified and/or isolated, if desired and/or required, using conventional methods known to the person skilled in the art. Suitable purifying processes are for example extraction processes and chromatographic processes such as column chromatography or preparative chromatography. All of the process steps described below, as well as the respective purification and/or isolation of intermediate or end products, can be carried out partly or completely under an inert gas atmosphere, preferably under a nitrogen atmosphere.

If the substituted compounds according to the invention of the aforementioned general formula (I) are obtained, after preparation thereof, in the form of a mixture of their stereoisomers, preferably in the form of their racemates or other mixtures of their various enantiomers and/or diastereomers, they can be separated and if appropriate isolated using conventional processes known to the person skilled in the art. Examples include chromatographic separating processes, in particular liquid chromatography processes under normal pressure or under elevated pressure, preferably MPLC and HPLC processes, and also fractional crystallisation processes. These processes allow individual enantiomers, for example diastereomeric salts formed by means of chiral stationary phase HPLC or by means of crystallisation with chiral acids, for example (+)-tartaric acid, (-)-tartaric acid or (+)-10-camphorsulphonic acid, to be separated from one another.

*General reaction scheme I*:

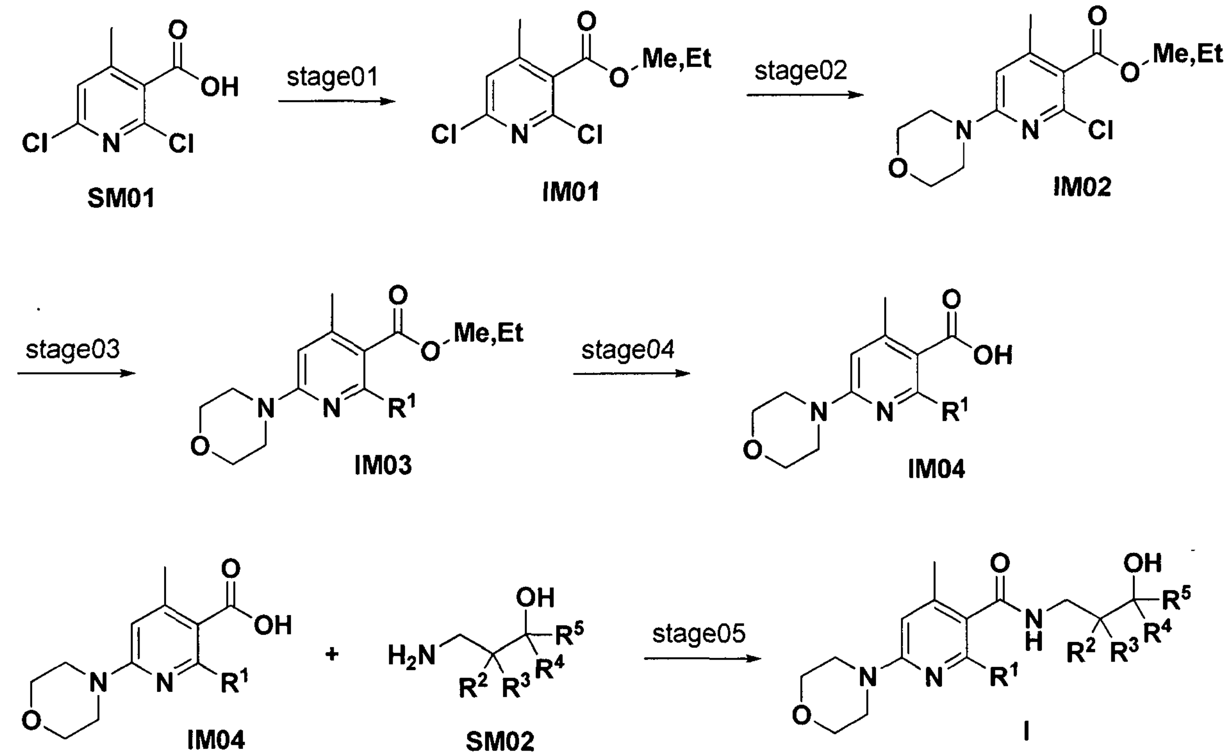


SM01 IM01 IM02 IM03 IM04 IM04 SM02 I

In **stage01,** 2,6-dichloro-nicotinic acids of the general formula **SM01** can be transformed into the corresponding esters of the general formula **IM01** according to methods known to the person skilled in the art, for example using a suitable alkylation reagent, for example methyl iodide or ethyl iodide.

In **stage02,** 2,6-dichloro-nicotinic acid esters of the general formula **IM01** can be transformed into 2-chhloro-6-morpholin-nicotinic acid esters of the general formula **IM02** using morpholine according to methods known to the person skilled in the art, for example by conventional or microwave heating, neat or in solution, for example in acetonitrile, dimethylformamide, dioxane, N-methyl-2-pyrrolidone or tetrahydrofuran, optionally in the presence of a suitable base, for example triethylamine, N,N-diisopropylethylamine, potassium carbonate, caesium carbonate, sodium tert-butoxide or potassium tert-butoxide, optionally by addition of a suitable coupling reagent, for example tetrakis(triphenylphosphin)-palladium, bis(dibenzylideneacetone)-palladium(0), or tris(dibenzylideneacetone)-dipalladium(0), optionally in presence of an additional ligand, for example (2-biphenyl)di-tert-butylphosphine or 2'-bis(diphenylphosphino)-1,1'-binaphthyl.

In **stage03** 2-chhloro-6-morpholin-nicotinic acid esters of the general formulae **IM02** can be transformed into the intermediates of the general formula **IM03** according to methods known to the person skilled in the art with compounds of the general formula Y-$R^1$, where Y denotes hydrogen, a metal or organometallic residue, for example sodium, magnesium bromide, magnesium chloride, tributyltin or boronic acid, or a residue to form an organometallic reagent, according to methods known to the person skilled in the art, for example by conventional or microwave heating, neat or in solution, for example in acetonitrile, dimethylformamide, dioxane, N-methyl-2-pyrrolidone, tetrahydrofuran, methanol or ethanol, optionally in the presence of a suitable base, for example triethylamine, N,N-diisopropylethylamine, potassium carbonate, caesium carbonate, sodium tert-butoxide or potassium tert-butoxide, optionally by addition of a suitable coupling reagent, for example tetrakis(triphenylphosphin)-palladium, bis(dibenzylideneacetone)-palladium(0), tris(di-benzylideneacetone)-dipalladium(0), [1,3-bis(diphenylphosphino)propane]-dichloronickel(II) or iron(III) acetylacetonate, optionally in presence of an additional ligand, for example (2-biphenyl)di-tert-butylphosphine or 2'-bis(diphenylphosphino)-1,1'-binaphthyl.

In **stage04** nicotinic acid esters of the general formula **IM03** can be transformed into the corresponding nicotinic acids of the general formula **IM04** according to methods known to the person skilled in the art, for example by employing a base, for example sodium hydroxide.

In **stage05** nicotinic acids of the general formula **IM04** can be converted to yield amides of the general formula I with amines of the general formula **SM02** according to methods known to the person skilled in the art, for example by

using a suitable coupling reagent, for example O-(7-azabenzotriazol-1-yl)-N,N,N',N'-tetramethyluronium hexafluorophosphate.

*General reaction scheme II*:

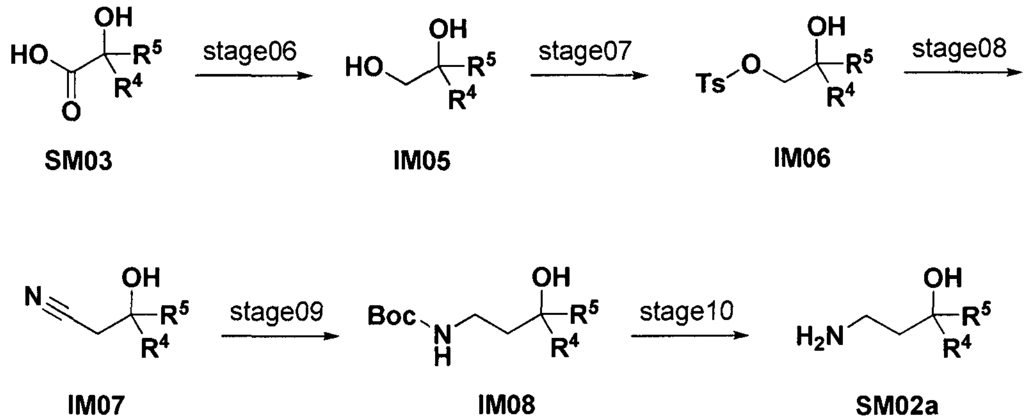


SM03 IM05 IM06

IM07 IM08 SM02a

Amines of the general formula **SM02,** in which $R^2$ and $R^3$ denote hydrogen, herein thereafter referred to as **SM02a** can be prepared from substituted 2-hydroxyacetic acids of the general formula **SM03,** according to methods known to the person skilled in the art. Enantiomerically pure amines of the general formula **SM02a** can be obtained from enantiomerically pure 2-hydroxyacetic acids of the general formula **SM03.** In **stage06,** 2-hydroxyacetic acids of the general formula **SM03** can be transformed into the corresponding diols of the general formula **IM05** according to methods known to the person skilled in the art, for example by employing a reducing agent, for example lithium aluminium hydride. In **stage07,** diols of the general formula **IM05** can be transformed into the corresponding p-toluenesulfonic acid esters of the general formula **IM06** according to methods known to the person skilled in the art, for example by treating with 4-toluenesulfonyl chloride. In **stage08,** p-toluenesulfonic acid esters of the general formula **IM06** can be transformed into the corresponding nitriles general formula **IM07** according to methods known to the person skilled in the art, for example by treating with sodium cyanide. In **stage09,** nitriles general formula **IM07** can be transformed into the corresponding tert-butyl carbamates of the general formula **IM08** according to methods known to the person skilled in the art, for example by treating with nickel(II) chloride and sodium borohydride in the presence of di-tert-butyl dicarbonate. In **stage10,** tert-butyl carbamates of the general formula **IM08** can be transformed into the corresponding amines of the general formula **SM02a** according to methods known to the person skilled in the art, for example by treating with hydrochloric acid.

*General reaction scheme III*:

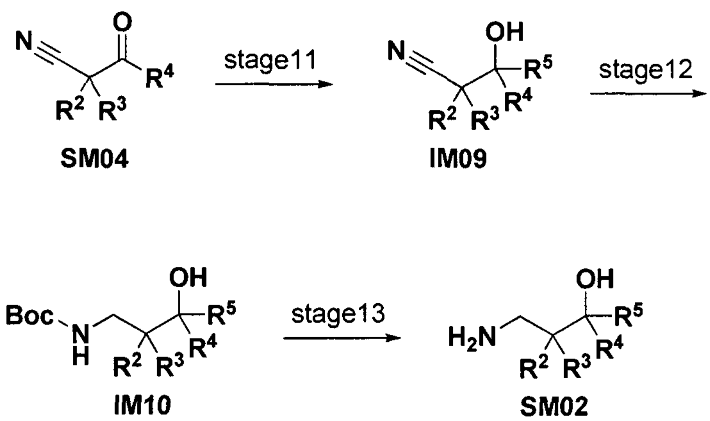


SM04 IM09

IM10 SM02

Alternatively, amines of the general formula **SM02** can be prepared from substituted 3-oxopropanenitriles of the general formula **SM04,** according to methods known to the person skilled in the art. In **stage11,** 3-oxopropanenitriles of the general formula **SM04** can be transformed into the corresponding 3-hydroxypropanenitriles of the general formula

**IM09** according to methods known to the person skilled in the art, for example by employing a reducing agent, for example sodium borohydride, borane dimethyl sulfide complex or borane tetrahydrofuran complex (affording compounds in which $R^5$ denotes a hydrogen), optionally in presence of a suitable ligand, for example CBS-oxazaborolidines, or alternatively by treating with compounds of the general formula Y-$R^5$, where Y denotes a metal or organometallic residue, for example sodium, magnesium bromide, magnesium chloride, or a residue to form an organometallic reagent, according to methods known to the person skilled in the art (affording compounds in which $R^5$ denotes a substituted carbon). In **stage12,** 3-hydroxypropanenitriles of the general formula **IM09** can be transformed into the corresponding tert-butyl carbamates of the general formula **IM10** according to methods known to the person skilled in the art, for example by treating with nickel(II) chloride and sodium borohydride in the presence of di-tert-butyl dicarbonate. In **stage13,** tert-butyl carbamates of the general formula **IM10** can be transformed into the corresponding amines of the general formula **SM02** according to methods known to the person skilled in the art, for example by treating with hydrochloric acid.

A plurality of additional synthesis paths to compounds of the general formulae **SM02, SM02a, SM03, SM04** with a very broad substitution pattern for residues $R^2$ to $R^5$ are known in the current specialist literature. Previously unknown intermediates of the general formulae **SM02, SM02a, SM03, SM04** with similar substitution patterns for residues $R^2$ to $R^5$ as outlined above and whose syntheses are not described in greater detail can be produced by the person skilled in the art according to these known methods or by combination of the known methods.

The invention will be described hereinafter with the aid of a number of examples. This description is intended merely by way of example and does not limit the general idea of the invention.

## Examples

The indication "M" are indications of concentration in mol/l, "d" means days, "brine" means saturated aqueous sodium chloride solution, "h" means hour(s), "MS" means mass spectrometry, "RT" means room temperature (23 ± 7 °C), "TLC" means thin layer chromatography, "v/v" means volume to volume.

The yields of the compounds prepared were not optimized. All temperatures are uncorrected.

The stationary phase used for the column chromatography was silica gel 60 (0.04 - 0.063 mm) from E. Merck, Darmstadt.

All starting materials which are not explicitly described were either commercially available (the details of suppliers such as for example Acros, Avocado, Aldrich, Bachem, Fluka, Lancaster, Maybridge, Merck, Sigma, TCI, Oakwood, etc. can be found in the Symyx® Available Chemicals Database of MDL, San Ramon, US or the SciFinder® Database of the ACS, Washington DC, US, respectively, for example) or the synthesis thereof has already been described precisely in the specialist literature (experimental guidelines can be found in the Reaxys® Database of Elsevier, Amsterdam, NL or the SciFinder® Database of the ACS, Washington DC, US, respectively, for example) or can be prepared using the conventional methods known to the person skilled in the art.

The mixing ratios of solvents or eluents for chromatography are specified in v/v.

All the intermediate products and exemplary compounds were analytically characterised by means of $^1$H-NMR spectroscopy. In addition, mass spectrometry tests (MS, m/z for $[M+H]^+$) were carried out for all the exemplary compounds and selected intermediate products.

## Synthesis of exemplary compounds

**Synthesis of example** 1: 2-Cyclopropyl-N-(3-hydroxy-4,4-dimethyl-pentyl)-4-methyl-6-morpholin-4-yl-pyridine-3-carboxylic acid amide

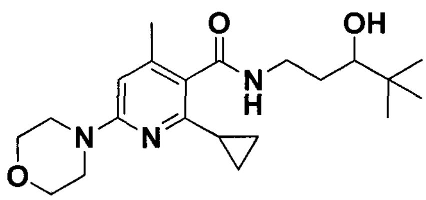

a) Synthesis of 2,6-dichloro-4-methyl-pyridine-3-carboxylic acid ethyl ester A mixture of 2,6-dichlor-4-methylpyridin-3-carboxylic acid (100 g, 485 mmol) and potassium carbonate (100 g, 728 mmol) in dimethylformamide (1.25 l) is stirred for 30 h at RT before ethyl iodide (59.5 ml, 728 mmol) is added and stirring is continued for 18 h. After completion of the reaction, water is added and the mixture is extracted with ethyl acetate (3x). The combined organic

layers are washed with water (2x) and brine (1x), dried over magnesium sulphate and evaporated to dryness to yield 2,6-dichloro-4-methyl-pyridine-3-carboxylic acid ethyl ester (112 g), which was used without further purification.

b) Synthesis of 2-chloro-4-methyl-6-morpholin-4-yl-pyridine-3-carboxylic acid ethyl ester
A mixture of 2,6-dichloro-4-methyl-pyridine-3-carboxylic acid ethyl ester (29.7 g, 127 mmol), potassium carbonate (26.3 g, 190 mmol), and morpholine (13.3 g, 152 mmol) in dimethylformamide (350 ml) is stirred for 18 h at 95 °C. After completion of the reaction, water is added and the mixture is extracted with diethyl ether (3x). The combined organic layers are washed with water (2x) and brine (1x), dried over magnesium sulphate and evaporated to dryness. The crude product is purified by flash chromatography (silica gel, 25% ethyl acetate/cyclohexane) to yield 2-chloro-4-methyl-6-morpholin-4-yl-pyridine-3-carboxylic acid ethyl ester (10.5 g, 36.9 mmol, 29%).

c) Synthesis of 2-cyclopropyl-4-methyl-6-morpholin-4-yl-pyridine-3-carboxylic acid ethyl ester To a solution 2-chloro-4-methyl-6-morpholin-4-yl-pyridine-3-carboxylic acid ethyl ester (7.0 g, 24.6 mmol) in tetrahydrofuran-N-methyl-2-pyrrolidone (2:3) (250 ml) is added iron(III) acetylacetonate (1.74 g, 4.92 mmol) and the mixture is cooled to -20 °C. At this temperature, a solution of cyclopropylmagnesium bromide (0.7 M in tetrahydrofuran) (105 ml, 73.8 mmol) is slowly added and the mixture is warmed to 0 °C within 30 min. After completion of the reaction, 10% aqueous ammonium chloride is added and the mixture is extracted with ethyl acetate (2x). The combined organic layers are washed with water and brine, dried over magnesium sulphate and evaporated to dryness. The reaction is repeated once and the combined crude products are purified by flash chromatography (silica gel, 75% ethyl acetate/cyclohexane) to yield 2-cyclopropyl-4-methyl-6-morpholin-4-yl-pyridine-3-carboxylic acid ethyl ester (15.1 g).

d) Synthesis of 2-cyclopropyl-4-methyl-6-morpholin-4-yl-pyridine-3-carboxylic acid
A solution of 2-cyclopropyl-4-methyl-6-morpholin-4-yl-pyridine-3-carboxylic acid ethyl ester (16.8 g, 57.8 mmol) in methanol-tetrahydrofuran (1:1) (90 ml) is treated for 3 days with a 2M aqueous solution of sodium hydroxide at reflux. The organic solvent is distilled off and the aqueous layer treated with 2M hydrochloric acid, and extracted with ethyl acetate. The combined organic layers are dried over magnesium sulphate and evaporated to dryness. The crude product is purified by flash chromatography (silica gel, 75% ethyl acetate/cyclohexane) to yield 2-cyclopropyl-4-methyl-6-morpholin-4-yl-pyridine-3-carboxylic acid (10.4 g, 39.6 mmol, 69%).

e) Synthesis of 2-cyclopropyl-N-(3-hydroxy-4,4-dimethyl-pentyl)-4-methyl-6-morpholin-4-yl-pyridine-3-carboxylic acid amide
To a solution of 2-cyclopropyl-4-methyl-6-morpholin-4-yl-pyridine-3-carboxylic acid (1.90 g, 7.24 mmol) in tetrahydrofuran (60 ml) are added triethylamine (3.0 ml, 21.7 mol) and O-(7-azabenzotriazol-1-yl)N,N,N',N'-tetramethyluronium hexafluorophosphate (2.75 g, 7.24 mmol). The mixture is stirred for 15 min at RT, before 1-amino-4,4-dimethylpentan-3-ol (1.05 g, 7.97 mmol) is added and stirring is continued for 18 h. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 50% ethyl acetate/cyclohexane) to yield 2-cyclopropyl-N-(3-hydroxy-4,4-dimethyl-pentyl)-4-methyl-6-morpholin-4-yl-pyridine-3-carboxylic acid amide **(example 1)** (1.74 g, 4.63 mmol, 64%). $[M+H]^+$ 376.4.

**Examples 1a and 1b:** 2-Cyclopropyl-N-(3-hydroxy-4,4-dimethyl-pentyl)-4-methyl-6-morpholin-4-yl-pyridine-3-carboxylic acid amide (1.64 g) was resolved into its corresponding enantiomers using chiral HPLC with the following conditions. Column, Chiralpak AD-H, 5 p, 250 x 20 mm; mobile phase, n-heptane/iso-propanol = 90:10 (v/v); flow rate, 19 ml/min; detection, UV (254 nm), 180 separations with 10 mg racemic material each. Analytical HPLC conditions. Column, Chiralpak AD-H, 250 x 4.6 mm; mobile phase, n-heptane/iso-propanol = 90:10 (v/v); flow rate, 1 ml/min; detection, UV (254 nm). Retention time (min): first eluting enantiomer, 8.59 (>99% ee); second eluting enantiomer, 10.45 (94% ee). Separation by chiral HPLC affords the first eluting enantiomer of 2-cyclopropyl-N-(3-hydroxy-4,4-dimethyl-pentyl)-4-methyl-6-morpholin-4-yl-pyridine-3-carboxylic acid amide (example 1a) (0.49 g; $[M+H]^+$ 376.4) and the second eluting enantiomer of 2-cyclopropyl-N-(3-hydroxy-4,4-dimethyl-pentyl)-4-methyl-6-morpholin-4-yl-pyridine-3-carboxylic acid amide **(example 1b)** (0.49 g; $[M+H]^+$ 376.4) as white solids.

**Synthesis of example 2:** 2-Cyclopropyl-N-[[(1S,2R)-2-hydroxy-cyclohexyl]-methyl]-4-methyl-6-morpholin-4-yl-pyridine-3-carboxylic acid amide

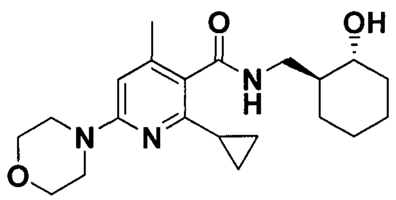

To a solution of 2-cyclopropyl-4-methyl-6-morpholin-4-yl-pyridine-3-carboxylic acid (synthesized according to the methods described in sections a) to d) of **example 1**) (0.15 g, 0.57 mmol) in dichloromethane (10 ml) are added N-ethyl-diisopropylamine (0.24 ml, 1.43 mmol), 1-ethyl-3-(3-dimethylaminopropyl) carbodiimide (0.13 g, 0.69 mmol), and 1-hydroxybenzotriazole hydrate (0.015 g, 0.11 mmol). The mixture is stirred for 15 min at RT, and then cooled to 0 °C, before (1R,2S)-2-(aminomethyl)cyclohexanol (0.07 g, 0.57 mmol) is added and stirring is continued for 3 days. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 30% ethyl acetate/cyclohexane) to yield 2-cyclopropyl-N-[[(1S,2R)-2-hydroxy-cyclohexyl]-methyl]-4-methyl-6-morpholin-4-yl-pyridine-3-carboxylic acid amide **(example 2)** (0.21 g, 0.56 mmol, 98%). $[M+H]^+$ 374.2.

**Synthesis of example 3:** 2-Cyclopropyl-N-[[(1 R,2S)-2-hydroxy-cyclohexyl]-methyl]-4-methyl-6-morpholin-4-yl-pyridine-3-carboxylic acid amide

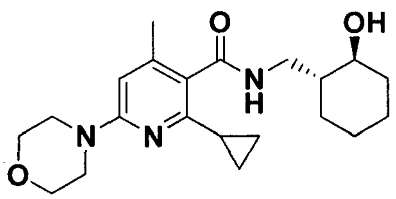

To a solution of 2-cyclopropyl-4-methyl-6-morpholin-4-yl-pyridine-3-carboxylic acid (synthesized according to the methods described in sections a) to d) **of example 1**) (0.15 g, 0.57 mmol) in dichloromethane (10 ml) are added N-ethyl-diisopropylamine (0.24 ml, 1.43 mmol), 1-ethyl-3-(3-dimethylaminopropyl) carbodiimide (0.13 g, 0.69 mmol), and 1-hydroxybenzotriazole hydrate (0.015 g, 0.11 mmol). The mixture is stirred for 15 min at RT, and then cooled to 0 °C, before (1S,2R)-2-(aminomethyl)cyclohexanol (0.07 g, 0.57 mmol) is added and stirring is continued for 3 days. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 60% ethyl acetate/cyclohexane) to yield 2-cyclopropyl-N-[[(1R,2S)-2-hydroxy-cyclohexyl]-methyl]-4-methyl-6-morpholin-4-yl-pyridine-3-carboxylic acid amide **(example 3)** (0.17 g, 0.45 mmol, 80%). $[M+H]^+$ 374.3.

**Synthesis of example 4:** 2-Cyclopropyl-N-([2-hydroxy-cyclopentyl]-methyl)-4-methyl-6-morpholin-4-yl-pyridine-3-carboxylic acid amide

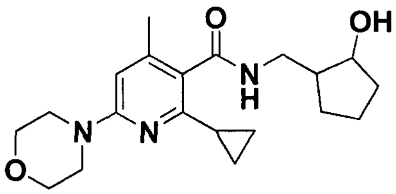

To a solution of 2-cyclopropyl-4-methyl-6-morpholin-4-yl-pyridine-3-carboxylic acid (synthesized according to the methods described in sections a) to d) of **example 1)** (0.50 g, 1.91 mmol) in dichloromethane (20 ml) are added N-ethyl-diisopropylamine (0.81 ml, 4.77 mmol), 1-ethyl-3-(3-dimethylaminopropyl) carbodiimide hydrochloride (0.42 g, 2.29 mmol), and 1-hydroxybenzotriazole hydrate (0.05 g, 0.38 mmol). The mixture is stirred for 15 min at RT, and then cooled to 0 °C, before 2-(aminomethyl)cyclopentanol (0.22 g, 1.91 mmol) is added and stirring is continued for 3 days. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness affording **example 4** as crude product. The crude product is purified by flash chromatography (silica gel, 50% ethyl acetate/cyclohexane) to yield the first eluting diastereomer of 2-cyclopropyl-N-([2-hydroxy-cyclopentyl]-methyl)-4-methyl-6-morpholin-4-yl-pyridine-3-carboxylic acid amide **(example 4a)** (0.34 g, 0.95 mmol, $[M+H]^+$ 360.2) and the second eluting diastereomer of 2-cyclopropyl-N-([2-hydroxy-cyclopentyl]-methyl)-4-methyl-6-morpholin-4-yl-pyridine-3-carboxylic acid amide **(example 4b)** (0.27 g, 0.75 mmol, $[M+H]^+$ 360.2) as white solids.

**Synthesis of example 5:** N-(3-Hydroxy-4,4-dimethyl-pentyl)-2-isopropyl-4-methyl-6-morpholin-4-yl-pyridine-3-carboxylic acid amide

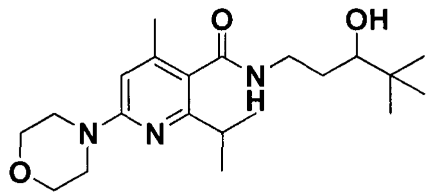

To a solution of 2-isopropyl-4-methyl-6-morpholin-4-yl-pyridine-3-carboxylic acid (synthesized similar to the methods described in sections a) to d) of **example 1**) (1.86 g, 7.05 mmol) in tetrahydrofuran (60 ml) are added triethylamine (2.9 ml, 21.1 mol) and O-(7-azabenzotriazol-1-yl)N,N,N',N'-tetramethyluronium hexafluorophosphate (2.69 g, 7.05 mmol). The mixture is stirred for 15 min at RT, before 1-amino-4,4-dimethylpentan-3-ol (1.02 g, 7.76 mmol) is added and stirring is continued for 18 h. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 30% ethyl acetate/cyclohexane) to yield N-(3-hydroxy-4,4-dimethyl-pentyl)-2-isopropyl-4-methyl-6-morpholin-4-yl-pyridine-3-carboxylic acid amide **(example 5)** (1.30 g, 3.44 mmol, 49%). $[M+H]^+$ 378.3.

**Examples 5a and 5b:** N-(3-Hydroxy-4,4-dimethyl-pentyl)-2-isopropyl-4-methyl-6-morpholin-4-yl-pyridine-3-carboxylic acid amide (1.1 g) was resolved into its corresponding enantiomers using chiral HPLC with the following conditions. Column, Chiralpak AD-H, 5 $\mu$, 250 x 20 mm; mobile phase, n-heptane/iso-propanol = 90:10 (v/v); flow rate, 19 ml/min; detection, UV (254 nm), 80 separations with 15 mg racemic material each. Analytical HPLC conditions. Column, Chiralpak AD-H, 250 x 4.6 mm; mobile phase, n-heptane/iso-propanol = 90:10 (v/v); flow rate, 1 ml/min; detection, UV (254 nm). Retention time (min): first eluting enantiomer, 6.07 (94% ee); second eluting enantiomer, 8.40 (99% ee). Separation by chiral HPLC affords the first eluting enantiomer of N-(3-hydroxy-4,4-dimethyl-pentyl)-2-isopropyl-4-methyl-6-morpholin-4-yl-pyridine-3-carboxylic acid amide **(example 5a)** (0.44 g; $[M+H]^+$ 378.3) and the second eluting enantiomer of N-(3-hydroxy-4,4-dimethyl-pentyl)-2-isopropyl-4-methyl-6-morpholin-4-yl-pyridine-3-carboxylic acid amide **(example 5b)** (0.42 g; $[M+H]^+$ 378.3) as white solids.

**Synthesis of example 6:** 2-Isopropyl-4-methyl-6-morpholin-4-yl-N-(4,4,4-trifluoro-3-hydroxy-butyl)-pyridine-3-carboxylic acid amide

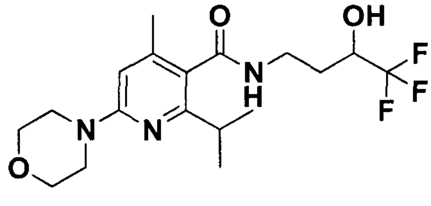

To a solution of 2-isopropyl-4-methyl-6-morpholin-4-yl-pyridine-3-carboxylic acid (synthesized similar to the

methods described in sections a) to d) of **example 1**) (1.40 g, 5.30 mmol) in tetrahydrofuran (40 ml) are added triethylamine (2.9 ml, 21.2 mol) and O-(7-azabenzotriazol-1-yl)N,N,N',N'-tetramethyluronium hexafluorophosphate (2.9 g, 5.3 mmol). The mixture is stirred for 15 min at RT, before 4-amino-1,1,1-trifluorobutan-2-ol hydrochloride (1.05 g, 5.8 mmol) is added and stirring is continued for 18 h. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 50% ethyl acetate/cyclohexane) to yield 2-Isopropyl-4-methyl-6-morpholin-4-yl-N-(4,4,4-trifluoro-3-hydroxy-butyl)-pyridine-3-carboxylic acid amide **(example 6)** (1.24 g, 3.18 mmol, 60%). $[M+H]^+$ 390.2.

**Examples 6a and 6b:** 2-Isopropyl-4-methyl-6-morpholin-4-yl-N-(4,4,4-trifluoro-3-hydroxy-butyl)-pyridine-3-carboxylic acid amide was resolved into its corresponding enantiomers using chiral HPLC with the following conditions. Column, Chiralpak AD-H, 5 μ, 250 x 20 mm; mobile phase, n-heptane/iso-propanol = 90:10 (v/v); flow rate, 19 ml/min; detection, UV (254 nm). Analytical HPLC conditions. Column, Chiralpak AD-H, 250 x 4.6 mm; mobile phase, n-heptane/ethanol = 95:5 (v/v); flow rate, 1 ml/min; detection, UV (254 nm). Retention time (min): first eluting enantiomer, 9.60 **(example 6a);** second eluting enantiomer, 12.21 **(example 6b).**

**Synthesis of example 7:** N-[[(1S,2R)-2-Hydroxy-cyclohexyl]-methyl]-2-isopropyl-4-methyl-6-morpholin-4-yl-pyridine-3-carboxylic acid amide

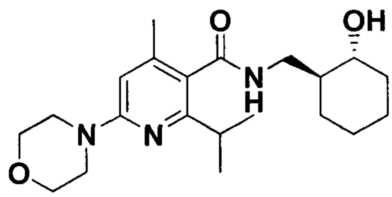

To a solution of 2-isopropyl-4-methyl-6-morpholin-4-yl-pyridine-3-carboxylic acid (synthesized similar to the methods described in sections a) to d) of **example 1)** (0.20 g, 0.76 mmol) in dichloromethane (15 ml) are added N-ethyldiisopropylamine (0.32 ml, 0.89 mmol), 1-ethyl-3-(3-dimethylaminopropyl) carbodiimide hydrochloride (0.17 g, 0.91 mmol), and 1-hydroxybenzotriazole hydrate (0.02 g, 0.15 mmol). The mixture is stirred for 15 min at RT, and then cooled to 0 °C, before (1R,2S)-2-(aminomethyl)cyclohexanol (0.09 g, 0.76 mmol) is added and stirring is continued for 18 h. After completion of the reaction, the mixture is diluted with ethyl acetate and 10% aqueous ammonium chloride. The organic layer is separated and washed with saturated sodium hydrogencarbonate solution and brine, dried over magnesium sulphate and evaporated to dryness. The crude product is purified by flash chromatography (silica gel, 50% ethyl acetate/cyclohexane) to yield N-([(1S,2R)-2-hydroxycyclohexyl]-methyl]-2-isopropyl-4-methyl-6-morpholin-4-yl-pyridine-3-carboxylic acid amide **(example 7)** (0.18 g, 0.48 mmol, 63%). $[M+H]^+$ 376.3.

**Synthesis of example 8:** 2-Ethoxy-N-(3-hydroxy-4,4-dimethyl-pentyl)-4-methyl-6-morpholin-4-yl-pyridine-3-carboxylic acid amide

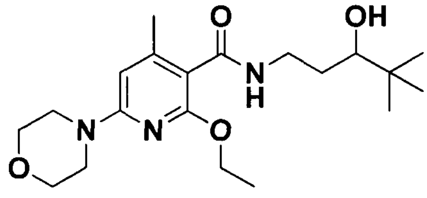

a) Synthesis of 6-chloro-2-ethoxy-4-methyl-pyridine-3-carboxylic acid

To a suspension of sodium hydride (60% dispersion in mineral oil) (4.2 g, 175 mmol) in tetrahydrofuran (200 ml) is added ethanol (4.08 ml, 70 mmol) at 10 °C. The mixture is allowed to warm to RT and stirring is continued for 1 h before 2,6-dichloro-4-methyl-pyridine-3-carboxylic acid (15.1 g, 73.5 mmol) in tetrahydrofuran (300 ml) is added and the mixture is stirred for additional 18 h at RT. Upon completion, the mixture is acidified with 2M hydrochloric acid and extracted with ethyl acetate (2 x 200 ml). The combined organic layers are washed with water (2 x 100 ml)

and brine (2 x 100 ml), dried over magnesium sulfate and evaporated to dryness. The crude product is purified by flash chromatography (silica gel, 5% methanol/ ethyl acetate) to yield 6-chloro-2-ethoxy-4-methyl-pyridine-3-carboxylic acid (13.8 g, 64.0 mmol, 87%).

b) Synthesis of 6-chloro-2-ethoxy-4-methyl-pyridine-3-carboxylic acid ethyl ester
A mixture of 6-chloro-2-ethoxy-4-methyl-pyridine-3-carboxylic acid (11.6 g, 53.8 mmol) in dimethylformamide (140 ml) is treated with potassium carbonate (11.1 g, 80.7 mmol) for 30 min, before ethyl iodide (6.6 ml, 80.7 mmol) is added and stirring is continued for 18 h. Upon completion, water is added and the mixture is extracted with ethyl acetate (2 x 50 ml). The combined organic layers are washed with brine, dried over magnesium sulfate and evaporated to dryness. The crude product is purified by flash chromatography (silica gel, 50% ethyl acetate/cyclohexane) to yield 6-chloro-2-ethoxy-4-methyl-pyridine-3-carboxylic acid ethyl ester (11.2 g, 45.9 mmol, 85%).

c) Synthesis of 2-ethoxy-4-methyl-6-morpholin-4-yl-pyridine-3-carboxylic acid ethyl ester
A mixture of 6-chloro-2-ethoxy-4-methyl-pyridine-3-carboxylic acid ethyl ester (11.2 g, 45.9 mmol), morpholine (4.36 ml, 50.5 mmol) and triethylamine (12.7 ml, 91.9 mmol) in 1-methyl-2-pyrrolidon (45 ml) is heated at 90 °C for 2d. After completion of the reaction, the solvent is distilled off and the residue is taken up in 1 M aqueous sodium hydrogen carbonate and extracted with ethyl acetate (3 x 50 ml). The combined organic layers are dried over magnesium sulfate and evaporated to dryness. The crude product is purified by flash chromatography (silica gel, 25% ethyl acetate/cyclohexane) to yield 2-ethoxy-4-methyl-6-morpholin-4-yl-pyridine-3-carboxylic acid ethyl ester (7.36 g, 25.0 mmol, 55%).

d) Synthesis of 2-ethoxy-4-methyl-6-morpholin-4-yl-pyridine-3-carboxylic acid
A solution of 2-ethoxy-4-methyl-6-morpholin-4-yl-pyridine-3-carboxylic acid ethyl ester (7.34 g, 24.9 mmol) in tetrahydrofuran/methanol (1:1) (120 ml) is treated with a solution of lithium chloride (3.56 h, 149.7 mmol) in water (75 ml) and stirred at 75°C for 18 h. After completion of the reaction, the mixture is concentrated in vacuo, acidified with 2M hydrochloric acid, and extracted with ethyl acetate (3 x 50 ml). The combined organic layers are dried over magnesium sulfate and evaporated to dryness. The crude product is purified by flash chromatography (silica gel, 30% ethyl acetate/cyclohexane) to yield 2-ethoxy-4-methyl-6-morpholin-4-yl-pyridine-3-carboxylic acid (2.47 g, 9.28 mmol, 37%).

e) Synthesis of 2-ethoxy-N-(3-hydroxy-4,4-dimethyl-pentyl)-4-methyl-6-morpholin-4-yl-pyridine-3-carboxylic acid amide
To a solution of 2-ethoxy-4-methyl-6-morpholin-4-yl-pyridine-3-carboxylic acid (0.23 g, 0.86 mmol) in dichloromethane (15 ml) are added diisopropylethylamine (0.37 ml, 2.16 mmol), 1-ethyl-3-(3-dimethylaminopropyl) carbodiimide (0.2 g, 1.04 mmol) and 1-hydroxybenzotriazol hydrate (23 mg, 0.17 mmol). The mixture is stirred for 15 min at RT, before 1-amino-4,4-dimethylpentan-3-ol (0.11 g, 0.86 mmol) is added and stirring is continued for 18 h. After completion of the reaction, the mixture is diluted with ethyl acetate and 10% aqueous ammonium chloride. The organic layer is separated and washed with saturated sodium hydrogencarbonate solution and brine, dried over magnesium sulfate and evaporated to dryness. The crude product is purified by flash chromatography (silica gel, 30% ethyl acetate/cyclohexane) to yield 2-ethoxy-N-(3-hydroxy-4,4-dimethyl-pentyl)-4-methyl-6-morpholin-4-yl-pyridine-3-carboxylic acid amide **(example 8)** (0.22 g, 0.58 mmol, 67%). $[M+H]^+$ 380.3.

**Pharmacological experiments**

**Method I. Fluorescence assay using a voltage sensitive dye (fluorimetry)**

The modulation of the KCNQ opening state (and consequently the voltage potential of a cell) by test compounds such as compounds according to the present invention results in an increased or reduced amount of a voltage-sensitive dye in the cytoplasm of the cells tested. These voltage-sensitive dyes are fluorescent dyes and, therefore, are forming the link between cell potential influenced by KCNQ modulation and fluorescence intensity. A KCNQ agonist leads to an opening of the channel, potassium and dye efflux, a following hyperpolarization and a reduction of the inner fluorescence intensity in a kinetic protocol. If applying an ion jump by KCl depolarization, KCNQ agonists increase the $\Delta F/F$ value. An antagonist performs vice versa, respectively.

The 'fluorimetry $EC_{50}$' is the half maximum effective concentration ($EC_{50}$), where the concentration of a drug/compound induces a response halfway between the baseline and maximum plateau effect. In other words, said value represents the concentration of a compound, where 50% of its maximal effect in the fluorimetric assay is observed if the substance concentration is graphed against the corresponding $\Delta F/F$ values (for which the calculation method is described below). Therefore, 'fluorimetry $EC_{50}$' is a measure for the compound potency. The compound efficacy is

mirrored by 'fluorimetry % efficacy' ('%Efficacy') and refers to the maximum response achievable by the test compound, i.e. the plateau effect. This drug's plateau effect is related to the plateau effect, which can be achieved by applying a saturated concentration of a reference compound, e.g. Retigabine (50 μM), in independent wells of the same experimental plate or series. A compound showing 100% efficacy is as efficacious as the reference compound (Retigabine) with a saturated concentration. This calculation '%Efficacy' method was introduced in order to normalize the ΔF/F values of different experiments to a common comparator compound, and make the test drug effects comparable.

Human CHO-K1 cells expressing KCNQ2/3 channels are cultivated adherently at 37°C, 5% $CO_2$ and 95% humidity in cell culture bottles (e.g. 80 $cm^2$ TC flasks, Nunc) with DMEM-high glucose (Sigma Aldrich, D7777) including 10% FCS (PAN Biotech, e.g. 3302-P270521) or alternatively MEM Alpha Medium (1x, liquid, Invitrogen, #22571), 10% fetal calf serum (FCS) (Invitrogen, #10270-106, heat-inactivated) and the necessary selection antibiotics.

Before being sown out for the measurements, the cells are washed with 1 x DPBS buffer $Ca^{2+}$/$Mg^{2+}$-free (e.g. Invitrogen, #14190-094) and detached from the bottom of the culture vessel by using Accutase (PAA Laboratories, #L11-007) (incubation with Accutase for 15 min at 37°C). The cell number is determined using a CASY™ cell counter (TCC, Schärfe System). Depending on the optimal density for each individual cell line, 20,000-30,000 cells/well/100 μl are seeded onto 96-well Corning™ CellBIND™ assay plates (Flat Clear Bottom Black Polystyrene Microplates, #3340). Freshly seeded cells are then left to settle for one hour at room temperature, followed by incubation for 24 hours at 37°C, 5% $CO_2$ and 95% humidity.

The voltage-sensitive fluorescent dye from the Membrane Potential Assay Kit (Red™ Bulk format part R8123 for FLIPR, MDS Analytical Technologies™) is prepared by dissolving the contents of one vessel *Membrane Potential Assay Kit Red Component A* in 200 ml of extracellular buffer (ES buffer, 120 mM NaCl, 1 mM KCl, 10 mM HEPES, 2 mM $CaCl_2$, 2 mM $MgCl_2$, 10 mM glucose; pH 7.4). After removal of the nutrient medium, the cells are washed once with 200 μl of ES buffer, then loaded for 45 min at room temperature in 100 μl of dye solution in the dark.

Fluorescence measurements are carried out in a BMG Labtech FLUOstar™, BMG Labtech NOVOstar™ or BMG Labtech POLARstar™ instrument (525 nm excitation, 560 nm emission, Bottom Read mode). After incubation with the dye, 50 μl of the test substances in the desired concentrations, or 50 μl of ES buffer for control purposes, are applied to the wells of the assay plate and incubated for 30 min at room temperature while being shielded from light. The fluorescence intensity of the dye is then measured for 5 min and the fluorescence value $F_1$ of each well is thus determined at a given, constant time. 15 μl of a KCl solution are then added to each well (final concentration of potassium ions 92 mM). The change in fluorescence intensity is subsequently monitored until all the relevant values have been obtained (mainly 5-30 min). At a given time post KCl application, a fluorescence value $F_2$ is determined, in this case at the time of the fluorescence peak.

For calculation, the fluorescence intensity $F_2$ is corrected for the fluorescence intensity $F_1$, and the activity (ΔF/F) of the target compound on the potassium channel is determined as follows:

$$\left(\frac{F_2 - F_1}{F_1}\right) \times 100 = \frac{\Delta F}{F} (\%)$$

In order to determine whether a substance has agonistic activity, $\frac{\Delta F}{F}$ can be related to $\left(\frac{\Delta F}{F}\right)_K$ of control wells. $\left(\frac{\Delta F}{F}\right)_K$ is determined by adding to the well only the buffer solution instead of the test substance, determining the value $F_{1K}$ of the fluorescence intensity, adding the potassium ions as described above, and measuring a value $F_{2K}$ of the fluorescence intensity. $F_{2K}$ and $F_{1K}$ are then calculated as follows:

$$\left(\frac{F_{2K} - F_{1K}}{F_{1K}}\right) \times 100 = \left(\frac{\Delta F}{F}\right)_K (\%)$$

A substance has an agonistic activity on the potassium channel if $\frac{\Delta F}{F}$ is greater than $\left(\frac{\Delta F}{F}\right)_K$:

$$\frac{\Delta F}{F} \rangle \left(\frac{\Delta F}{F}\right)_K$$

Independently of the comparison of $\frac{\Delta F}{F}$ with $\left(\frac{\Delta F}{F}\right)_K$ it is possible to conclude that a target compound has agonistic activity if $\frac{\Delta F}{F}$ increases dose dependently.

Calculations of $EC_{50}$ and $IC_{50}$ values are carried out with the aid of 'Prism v4.0' software (GraphPad Software™).

**Method II. Low-intensity tail flick test (rat)**

In the low-intensity tail flick test, the determination of the antinociceptive effect of the compounds according to the invention towards an acute noxious thermal stimulus is carried out by measuring the withdrawal reflex of the rat tail (tail flick) in response to a radiant heat beam (analgesia meter; model 2011 of the company Rhema Labortechnik, Hofheim, Germany) according to the method described by D'Amour and Smith (J. Pharm. Exp. Ther. 72, 74 79 (1941). To this end, the rats were placed in a plexiglas restrainer, and a low-intensity radiant heat beam (48°C) was focused onto the dorsal surface of the tail root. The stimulus intensity was adjusted to result in a mean pre-drug control withdrawal latency of about 7 s, thus also allowing a supraspinal modulation of the spinally mediated acute nociceptive reflex. A cutoff time of 30 s was applied to avoid tissue damage. Male Sprague-Dawley rats (Janvier, Le Genest St. Isle, Frankreich) with weights of 200-250 g were used. 10 rats were used per group. Before administration of a compound according to the invention, the animals were pre-tested twice in the course of five minutes and the mean of these measurements was calculated as the pre-test mean. The antinociceptive effect was determined at 20, 40 and 60 min after peroral compound administration. The antinociceptive effect was calculated based on the increase in the tail withdrawal latency according to the following formula and is expressed as percentage of the maximum possible effect (MPE [%]):

$$MPE = [(T_1-T_0)/(T_2-T_0)]*100$$

In this, $T_0$ is the control latency time before and $T_1$ the latency time after administration of the compound, $T_2$ is the cutoff time and MPE is the maximum possible effect. Employing variant analysis (repeated measures ANOVA) allowed testing of statistically significant differences between the compounds according to the invention and the vehicle group. The significance level was set to $p \leq 0.05$. To determine the dose dependency, the particular compound according to the invention was administered in 3-5 logarithmically increasing doses, including a threshold dose and a maximum effective dose, and the $ED_{50}$ values were determined with the aid of regression analysis. The $ED_{50}$ calculation was performed at the time of maximum efficacy (usually 20 min after administration of the compounds).

**Pharmacological data**

The pharmacological effects of the compounds according to the invention were determined as described hereinbefore **(pharmacological experiments,** methods I and II respectively).

The corresponding pharmacological data are summarized in *Table 1.*

*Table 1*:

| **Example** | **Fluorimetry % efficacy (retigabine = 100%)** | **Fluorimetry $EC_{50}$ / $IC_{50}$ [nM]** | **Low intensity tail flick, rat, peroral, $ED_{50}$ or MPE (dose) [mg/kg]** |
|---|---|---|---|
| **1** | 237 | 529 | |
| **1a** | 242 | 961 | |
| **1b** | 224 | 318 | 100 (10) |
| **2** | 245 | 483 | 67 (10) |
| **3** | 99 | 8545 | |
| **5** | 230 | 156 | |

(continued)

| Example | Fluorimetry % efficacy (retigabine = 100%) | Fluorimetry $EC_{50}$ / $IC_{50}$ [nM] | Low intensity tail flick, rat, peroral, $ED_{50}$ or MPE (dose) [mg/kg] |
|---|---|---|---|
| **5a** | 239 | 431 | |
| **5b** | 239 | 94 | |
| **6** | 150 | 1987 | |
| **6a** | 206 | 1093 | |
| **6b** | 149 | 3915 | |
| **7** | 243 | 185 | |
| **8** | 231 | 252 | |

## Claims

1. A substituted compound of general formula (I)

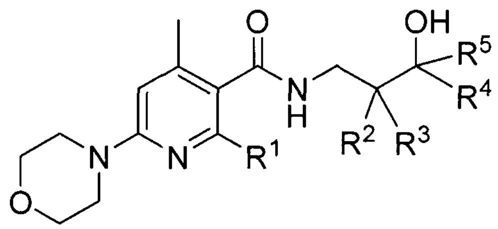

**(I)**,

wherein

$R^1$ represents a $C_{2-6}$-aliphatic residue, unsubstituted or mono- or polysubstituted; or a $C_{3-6}$-cycloaliphatic residue or a 3 to 6 membered heterocycloaliphatic residue, in each case unsubstituted or mono- or polysubstituted, and in each case optionally bridged via a $C_{1-4}$ aliphatic group, which in turn may be unsubstituted or mono- or polysubstituted;

or represents $OR^6$, wherein
$R^6$ represents a $C_{1-6}$-aliphatic residue, unsubstituted or mono- or polysubstituted; or a $C_{3-6}$-cycloaliphatic residue, unsubstituted or mono- or polysubstituted;
$R^2$ and $R^3$ independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted;
with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue, wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^3$
and/or $R^4$ and $R^5$
in pairs, in each case independently of one another, together with the carbon atom connecting them, form a $C_{3-10}$-cycloaliphatic residue or a 3 to 10 membered heterocycloaliphatic residue, in each case unsubstituted or mono- or polysubstituted,

and the respective remaining substituents of $R^2$ and $R^3$ each independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted,
with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue, wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted,
and $R^3$ represents H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue, wherein the $C_{1-4}$ aliphatic residue may in each case be unsubstituted or mono- or polysubstituted; or a $C_{3-6}$-cycloaliphatic residue or a 3 to 6 membered heterocycloaliphatic residue, in each case unsubstituted or mono- or polysubstituted;
and $R^5$ represents H; $CH_2F$; $CHF_2$; $CF_3$; a $C_{1-4}$-aliphatic residue, , wherein the $C_{1-4}$ aliphatic residue may be unsubstituted or mono- or polysubstituted; or a $C_{3-6}$-cycloaliphatic residue or a 3 to 6 membered heterocycloaliphatic residue, in each case unsubstituted or mono- or polysubstituted;
on the condition that if $R^5$ denotes a 3 to 6 membered heterocycloaliphatic residue, the 3 to 6 membered heterocycloaliphatic residue is linked via a carbon atom,
in which an "aliphatic group" and an "aliphatic residue" can in each case be branched or unbranched, saturated or unsaturated,
in which a "cycloaliphatic residue" and a "heterocycloaliphatic residue" can in each case be saturated or unsaturated,
in which "mono- or polysubstituted" with respect to an "aliphatic group" and an "aliphatic residue" relates, with respect to the corresponding residues or groups, to the substitution of one or more hydrogen atoms each independently of one another by at least one substituent selected from the group consisting of F, Cl, Br, I, $NO_2$, $NH_2$, an NH($C_{1-4}$ aliphatic residue), an N($C_{1-4}$ aliphatic residue)$_2$, a NH-C(=O)-$C_{1-4}$ aliphatic residue, a N($C_{1-4}$-aliphatic residue)-C(=O)-$C_{1-4}$ aliphatic residue, a NH-S(=O)$_2$-$C_{1-4}$ aliphatic residue, a N($C_{1-4}$-aliphatic residue)-S(=O)$_2$-$C_{1-4}$ aliphatic residue, =O, OH, $OCH_2F$, $OCHF_2$, $OCF_3$, a O-$C_{1-4}$-aliphatic residue, a O-C(=O)-$C_{1-4}$-aliphatic residue, SH, $SCF_3$, a S-$C_{1-4}$-aliphatic residue, S(=O)$_2$OH, a S(=O)-$C_{1-4}$-aliphatic residue, a S(=O)$_2$-$C_{1-4}$-aliphatic residue, a S(=O)$_2$-O-$C_{1-4}$-aliphatic residue, a S(=O)$_2$-NH-$C_{1-4}$-aliphatic residue, a S(=O)$_2$-N($C_{1-4}$-aliphatic residue)$_2$, CN, $CH_2F$, $CHF_2$, $CF_3$, CHO, COOH, a $C_{1-4}$-aliphatic residue, $CH_2OH$, $CH_2$-$OCH_3$, $C_2H_4$-OH, $C_2H_4$-$OCH_3$ $CH_2$-$CF_3$, a C(=O)-$C_{1-4}$-aliphatic residue, a C(=O)-O-$C_{1-4}$-aliphatic residue, a $C_{3-6}$-cycloaliphatic residue, a 3 to 6 membered heterocycloaliphatic residue, C(=O)-$NH_2$, a C(=O)-NH($C_{1-4}$ aliphatic residue), and a C(=O)-N($C_{1-4}$ aliphatic residue)$_2$;
in which "mono- or polysubstituted" with respect to a "cycloaliphatic residue" and a "heterocycloaliphatic residue" relates, with respect to the corresponding residues, to the substitution of one or more hydrogen atoms each independently of one another by at least one substituent selected from the group consisting of F, Cl, Br, I, $NO_2$, $NH_2$, an NH($C_{1-4}$ aliphatic residue), an N($C_{1-4}$ aliphatic residue)$_2$, a NH-C(=O)-$C_{1-4}$ aliphatic residue, a N($C_{1-4}$-aliphatic residue)-C(=O)-$C_{1-4}$ aliphatic residue, a NH-S(=O)$_2$-$C_{1-4}$ aliphatic residue, a N($C_{1-4}$-aliphatic residue)-S(=O)$_2$-$C_{1-4}$ aliphatic residue, =O, OH, $OCH_2F$, $OCHF_2$, $OCF_3$, a O-$C_{1-4}$-aliphatic residue, a O-C(=O)-$C_{1-4}$-aliphatic residue, SH, $SCF_3$, a S-$C_{1-4}$-aliphatic residue, S(=O)$_2$OH, a S(=O)-$C_{1-4}$-aliphatic residue, a S(=O)$_2$-$C_{1-4}$-aliphatic residue, a S(=O)$_2$-O-$C_{1-4}$-aliphatic residue, a S(=O)$_2$-NH-$C_{1-4}$-aliphatic residue, a S(=O)$_2$-N($C_{1-4}$-aliphatic residue)$_2$, CN, $CH_2F$, $CHF_2$, $CF_3$, CHO, COOH, a $C_{1-4}$-aliphatic residue, $CH_2OH$, $CH_2$-$OCH_3$, $C_2H_4$-OH, $C_2H_4$-$OCH_3$ $CH_2$-$CF_3$, a C(=O)-$C_{1-4}$-aliphatic residue, a C(=O)-O-$C_{1-4}$-aliphatic residue, a $C_{3-6}$-cycloaliphatic residue, a 3 to 6 membered heterocycloaliphatic residue, C(=O)-$NH_2$, a C(=O)-NH($C_{1-4}$ aliphatic residue), and a C(=O)-N($C_{1-4}$ aliphatic residue)$_2$;
optionally in the form of a single stereoisomer or a mixture of stereoisomers, in the form of the free compound and/or a physiologically acceptable salt thereof.

**2.** The compound according to claim 1, **characterized in that**

$R^1$ represents a $C_{2-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-atiphatic residue,

or represents a $C_{3-6}$-cycloaliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and wherein the $C_{3-6}$-cycloaliphatic residue may optionally be bridged via a $C_{1-4}$ aliphatic group, which in turn may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents $OR^6$, wherein

$R^6$ represents a $C_{1-6}$-aliphatic residue, unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue;

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

$R^2$ and $R^3$ independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

with the proviso that at least one of $R^2$ and $R^3$ denotes H or is linked via a carbon atom,

$R^4$ and $R^5$ independently of one another represent H; $CH_2F$; $CHF_2$; $CF_3$; or a $C_{1-4}$-aliphatic residue,

wherein the $C_{1-4}$-aliphatic residue may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

on the condition that if $R^4$ and/or $R^5$ denote(s) a 3 to 6 membered heterocycloaliphatic residue, the 3 to 6 membered heterocycloaliphatic residue is linked via a carbon atom,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or

$R^2$ and $R^3$

and/or $R^4$ and $R^5$

in pairs, in each case independently of one another, together with the carbon atom connecting them, form a $C_{3-10}$-cycloaliphatic residue or a 3 to 10 membered heterocycloaliphatic residue, in each case unsubstituted or mono-or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the respective remaining substituents of $R^2$ and $R^3$ each independently of one another represent H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

with the proviso that at least one of $R^2$ and $R^3$ denotes H or is linked via a carbon atom,

and the respective remaining substituents of $R^4$ and $R^5$ each independently of one another represent H; $CH_2F$; $CHF_2$; $CF_3$; a $C_{1-4}$-aliphatic residue,

wherein the $C_{1-4}$-aliphatic residue may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

on the condition that if $R^4$ and/or $R^5$ denote(s) a 3 to 6 membered heterocycloaliphatic residue, the 3 to 6 membered heterocycloaliphatic residue is linked via a carbon atom,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or

$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the remaining substituent $R^3$ represents H; F; Cl; Br; I; CN; $CH_2F$; $CHF_2$; $CF_3$; OH; $OCH_2F$; $OCHF_2$; $OCF_3$; $SCF_3$; a $C_{1-4}$-aliphatic residue, or an O-$C_{1-4}$ aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an NH($C_{1-4}$ aliphatic residue), an N($C_{1-4}$ aliphatic residue)$_2$, OH, =O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a S(=O)$_2$-$C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted O-$C_{1-4}$-aliphatic residue,

and the remaining substituent $R^5$ represents H; $CH_2F$; $CHF_2$; $CF_3$; $OCH_2F$; $OCHF_2$; $OCF_3$; $SCF_3$; a $C_{1-4}$-aliphatic residue,

wherein the $C_{1-4}$-aliphatic residue may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted

$O-C_{1-4}$-aliphatic residue,
or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,
on the condition that if $R^5$ denotes a 3 to 6 membered heterocycloaliphatic residue, the 3 to 6 membered heterocycloaliphatic residue is linked via a carbon atom,
wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted $O-C_{1-4}$-aliphatic residue.

**3.** The compound according to claim 1 or 2, **characterized in that**

$R^1$ represents a $C_{2-6}$-aliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted $O-C_{1-4}$-aliphatic residue,

or represents a $C_{3-6}$-cycloaliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted $O-C_{1-4}$-aliphatic residue,

or represents $OR^6$, wherein

$R^6$ represents a $C_{1-6}$-aliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,

wherein the $C_{1-4}$-aliphatic residue in each case may be unsubstituted or mono- or polysubstituted with at least one substituent selected from the group consisting of F, Cl, Br, I, OH, $OCH_2F$, $OCHF_2$, $OCF_3$, $CH_2F$, $CHF_2$, $CF_3$ and an unsubstituted $O-C_{1-4}$-aliphatic residue.

**4.** The compound according to any of the preceding claims, **characterized in that**

$R^1$ represents a $C_{2-6}$-aliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,

wherein the $C_{1-4}$-aliphatic residue is in each case unsubstituted,
or represents a $C_{3-6}$-cycloaliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH, =O, an $O-C_{1-4}$-aliphatic residue, $OCH_2F$, $OCHF_2$, $OCF_3$, SH, $SCF_3$, a $S-C_{1-4}$-aliphatic residue, a $S(=O)-C_{1-4}$-aliphatic residue, a $S(=O)_2-C_{1-4}$-aliphatic residue, CN, and a $C_{1-4}$-aliphatic residue,
wherein the $C_{1-4}$-aliphatic residue is in each case unsubstituted, or represents $OR^6$, wherein
$R^6$ represents a $C_{1-6}$-aliphatic residue, unsubstituted or mono- or polysubstituted with at least one substituent selected from the group consisting of F, Cl, Br, I, $NO_2$, $NH_2$, an $NH(C_{1-4}$ aliphatic residue), an $N(C_{1-4}$ aliphatic residue$)_2$, OH,

=O, an O-$C_{1-4}$-aliphatic residue, $OCH_2F$, $OCHF_2$, $OCF_3$, SH, $SCF_3$, a S-$C_{1-4}$-aliphatic residue, a S(=O)-$C_{1-4}$-aliphatic residue, a $S(=O)_2$-$C_{1-4}$-aliphatic residue, CN, and a $C_{1-4}$-aliphatic residue,
wherein the $C_{1-4}$-aliphatic residue is in each case unsubstituted.

**5.** The compound according to any of the preceding claims, **characterized in that**

$R^1$ represents a $C_{2-6}$-aliphatic residue, unsubstituted or mono- or polysubstituted with at least one substituent selected from the group consisting of F, Cl, Br, I, OH, and an unsubstituted O-$C_{1-4}$-aliphatic residue,

or represents a $C_{3-6}$-cycloaliphatic residue, unsubstituted or mono- or polysubstituted with at least one substituent selected from the group consisting of F, Cl, Br, I, OH, and an unsubstituted O-$C_{1-4}$-aliphatic residue,
or represents $OR^6$, wherein
$R^6$ represents a $C_{1-4}$-aliphatic residue, unsubstituted or mono- or polysubstituted with at least one substituent selected from the group consisting of F, Cl, Br, I, OH, and an unsubstituted O-$C_{1-4}$-aliphatic residue.

**6.** The compound-according to any of the preceding claims, **characterized in that**

$R^1$ represents ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, ethenyl or propenyl (-$CH_2CH=CH_2$, -CH=CH-$CH_3$, -C(=$CH_2$)-$CH_3$),

cyclopropyl, cyclobutyl, cyclopentyl, or cyclohexyl,
or represents $OR^6$, wherein
$R^6$ represents methyl, ethyl, n-propyl, 2-propyl, n-butyl, isobutyl, sec.-butyl, tert.-butyl, $CH_2OH$, $CH_2OCH_3$, $CH_2CH_2OH$, $CH_2CH_2OCH_3$, and $CH(OH)CH_2OH$.

**7.** The compound according to any of the preceding claims, **characterized in that**
$R^2$ and $R^3$ independently of one another represent H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue,
with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue,
or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^3$
and/or $R^4$ and $R^5$
in pairs, in each case independently of one another, together with the carbon atom connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue,,
and the respective remaining substituents of $R^2$ and $R^3$ each independently of one another represent H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,

wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue, or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue, wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue, with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue, or represent a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue, on the condition that if $R^4$ and/or $R^5$ denote(s) a 3 to 6 membered heterocycloaliphatic residue, the 3 to 6 membered heterocycloaliphatic residue is linked via a carbon atom,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue,
or
$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue, and the remaining substituent $R^3$ represents H; F; Cl; Br; I; $CH_2F$; $CHF_2$; $CF_3$; OH; $OCH_2F$; $OCHF_2$; $OCF_3$; a $C_{1-4}$-aliphatic residue, or an O-$C_{1-4}$ aliphatic residue,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue, or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue, wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue, and the remaining substituent $R^5$ represents H; $CH_2F$; $CHF_2$; $CF_3$; a $C_{1-4}$-aliphatic residue,
wherein the $C_{1-4}$-aliphatic residue may be unsubstituted or mono- or polysubstituted with at least one substituent selected from the group consisting of F, Cl, Br, I, OH, and an unsubstituted O-$C_{1-4}$-aliphatic residue, or represents a $C_{3-6}$-cycloaliphatic residue or a 3 to 6 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, $OCH_2F$, $OCHF_2$, $OCF_3$, and a $C_{1-4}$-aliphatic residue, on the condition that if $R^5$ denotes a 3 to 6 membered heterocycloaliphatic residue, the 3 to 6 membered heterocycloaliphatic residue is linked via a carbon atom,
wherein the $C_{1-4}$-aliphatic residue in each case may be unsubstituted or mono-or polysubstituted with at least one substituent selected from the group consisting of F, Cl, Br, I, OH, and an unsubstituted O-$C_{1-4}$-aliphatic residue.

**8.** The compound according to any of the preceding claims, **characterized in that**
$R^2$ and $R^3$ independently of one another represent H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each case unsubstituted,
or represent a $C_{3-6}$-cycloaliphatic residue or a 3 to 6 membered heterocycloaliphatic residue, in each case unsubstituted,
with the proviso that at least one of $R^2$ and $R^3$ denotes H or is linked via a carbon atom,
$R^4$ and $R^5$ independently of one another represent H; $CH_2F$; $CHF_2$; $CF_3$; a $C_{1-4}$-aliphatic residue,
wherein the $C_{1-4}$-aliphatic residue is unsubstituted,
or represent a $C_{3-6}$-cycloaliphatic residue or a 3 to 6 membered heterocycloaliphatic residue, in each case unsubstituted,
on the condition that if $R^4$ and/or $R^5$ denote(s) a 3 to 6 membered heterocycloaliphatic residue, the 3 to 6 membered

heterocycloaliphatic residue is linked via a carbon atom,
or
$R^2$ and $R^3$
and/or $R^4$ and $R^5$
in pairs, in each case independently of one another, together with the carbon atom connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, and a $C_{1-4}$-aliphatic residue,
wherein the $C_{1-4}$-aliphatic residue is in each case unsubstituted,
and the respective remaining substituents of $R^2$ and $R^3$ each independently of one another represent H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each unsubstituted,
or represent a $C_{3-6}$-cycloaliphatic residue or a 3 to 6 membered heterocycloaliphatic residue, in each case unsubstituted,
with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; a $C_{1-4}$-aliphatic residue,
wherein the $C_{1-4}$-aliphatic residue is unsubstituted,
or represent a $C_{3-6}$-cycloaliphatic residue or a 3 to 6 membered heterocycloaliphatic residue, in each case unsubstituted,
on the condition that if $R^4$ and/or $R^5$ denote(s) a 3 to 6 membered heterocycloaliphatic residue, the 3 to 6 membered heterocycloaliphatic residue is linked via a carbon atom,
or
$R^2$ and $R^4$ together with the carbon atoms connecting them, form a $C_{3-10}$-cycloaliphatic residue or a 3 to 10 membered heterocycloaliphatic residue, in each case unsubstituted or mono- or polysubstituted with at least one substituent selected from the group consisting of F, Cl, Br, I, OH, an O-$C_{1-4}$-aliphatic residue, $CH_2F$, $CHF_2$, $CF_3$, and a $C_{1-4}$-aliphatic residue,
wherein the $C_{1-4}$-aliphatic residue is in each case unsubstituted,
and the remaining substituent $R^3$ represents H; F; Cl; Br; I; $CH_2F$; $CHF_2$; $CF_3$; OH; a $C_{1-4}$-aliphatic residue, or an O-$C_{1-4}$ aliphatic residue,
wherein the $C_{1-4}$-aliphatic residue is in each case unsubstituted,
or represents a $C_{3-6}$-cycloaliphatic residue or a 3 to 6 membered heterocycloaliphatic residue, in each case unsubstituted,
and the remaining substituent $R^5$ represents H; $CH_2F$; $CHF_2$; $CF_3$; a $C_{1-4}$-aliphatic residue,
wherein the $C_{1-4}$-aliphatic residue is unsubstituted,
or represents a $C_{3-6}$-cycloaliphatic residue or a 3 to 6 membered heterocycloaliphatic residue, in each case unsubstituted,
on the condition that if $R^5$ denotes a 3 to 6 membered heterocycloaliphatic residue, the 3 to 6 membered heterocycloaliphatic residue is linked via a carbon atom.

**9.** The compound according to any of the preceding claims, **characterized in that**
$R^2$ and $R^3$ independently of one another represent H; OH; an unsubstituted $C_{1-4}$-aliphatic residue, or an unsubstituted O-$C_{1-4}$ aliphatic residue, with the proviso that at least one of $R^2$ and $R^3$ denotes H or is linked via a carbon atom,
$R^4$ and $R^5$ independently of one another represent H; $CH_2F$; $CHF_2$; $CF_3$; an unsubstituted $C_{1-4}$-aliphatic residue,
or
$R^2$ and $R^3$
or $R^4$ and $R^5$
in pairs, in each case independently of one another, together with the carbon atom connecting them, form a $C_{3-10}$-cycloaliphatic residue, unsubstituted or mono- or polysubstituted with at least one substituent selected from the group consisting of F, Cl, Br, I, OH, $CH_2F$, $CHF_2$, $CF_3$, an unsubstituted O-$C_{1-4}$-aliphatic residue, and an unsubstituted $C_{1-4}$-aliphatic residue,
and the respective remaining substituents of $R^2$ and $R^3$ each independently of one another represent H; OH; an unsubstituted $C_{1-4}$-aliphatic residue, or an unsubstituted O-$C_{1-4}$ aliphatic residue, with the proviso that at least one of $R^2$ and $R^3$ denotes H or is linked via a carbon atom,
and the respective remaining substituents of $R^4$ and $R^5$ each independently of one another represent H; $CH_2F$; $CHF_2$; $CF_3$; an unsubstituted $C_{1-4}$-aliphatic residue,
or
$R^2$ and $R^4$ together with the carbon atoms connecting them, form a $C_{3-10}$-cycloaliphatic residue, unsubstituted or

mono- or polysubstituted with at least one substituent selected from the group consisting of F, Cl, Br, I, OH, an unsubstituted O-$C_{1-4}$-aliphatic residue, $CH_2F$, $CHF_2$, $CF_3$, and a an unsubstituted $C_{1-4}$-aliphatic residue,
and the remaining substituent $R^3$ represents H; F; Cl; Br; I; OH; an unsubstituted $C_{1-4}$-aliphatic residue, or an unsubstituted O-$C_{1-4}$ aliphatic residue,
and the remaining substituent $R^5$ represents H; $CH_2F$; $CHF_2$; $CF_3$; an unsubstituted $C_{1-4}$-aliphatic residue.

**10.** The compound according to any of the preceding claims, **characterized in that**
$R^2$ and $R^3$ independently of one another represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,
$R^4$ and $R^5$ independently of one another represent H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$;
or
$R^2$ and $R^3$
or $R^4$ and $R^5$
in pairs, in each case independently of one another, together with the carbon atom connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of F, Cl, Br, I, OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and the respective remaining substituents of $R^2$ and $R^3$ each independently of one another represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$, with the proviso that at least one of $R^2$ and $R^3$ denotes H or is linked via a carbon atom,
and the respective remaining substituents of $R^4$ and $R^5$ each independently of one another represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$;
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of F, Cl, Br, I, OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and the respective remaining substituent of $R^3$ represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $OCH_3$ or $OCH_2CH_3$,
and the respective remaining substituent $R^5$ represents H; methyl; ethyl, n-propyl, 2-propyl, n-butyl, isobutyl, sec.-butyl, tert.-butyl, n-pentyl, isopentyl, neopentyl, n-hexyl, $CH_2$-$CH(CH_3)(C_2H_5)$, $C(CH_3)_2(C_2H_5)$, $CH_2F$; $CHF_2$; $CF_3$.

**11.** The compound according to any of the preceding claims, **characterized in that**

$R^1$ represents ethyl, n-propyl, 2-propyl, n-butyl, isobutyl, sec.-butyl, tert.-butyl, cyclopropyl, cyclobutyl, cyclopentyl, or cyclohexyl,

or represents $OR^6$, wherein
$R^6$ represents methyl, ethyl, n-propyl, 2-propyl, n-butyl, isobutyl, sec.-butyl, or tert.-butyl,
$R^2$ and $R^3$ in dependently of one another represent H; methyl; ethyl, n-propyl, 2-propyl, n-butyl, isobutyl, sec.-butyl, or tert.-butyl,
$R^4$ and $R^5$ independently of one another represent H; methyl; ethyl, n-propyl, 2-propyl, n-butyl, isobutyl, sec.-butyl, tert.-butyl, $CH_2F$, $CHF_2$, or $CF_3$;
or
$R^2$ and $R^3$
or $R^4$ and $R^5$
in pairs, in each case independently of one another, together with the carbon atom connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of OH, methyl; ethyl, n-propyl, 2-propyl, n-butyl, isobutyl, sec.-butyl, $CH_2F$, $CHF_2$, $CF_3$, $OCH_3$ and $OCH_2CH_3$,
and the respective remaining substituents of $R^2$ and $R^3$ in dependently of one another represent H; methyl; ethyl,

n-propyl, 2-propyl, n-butyl, isobutyl, sec.-butyl, or tert.-butyl,
and the respective remaining substituents of $R^4$ and $R^5$ independently of one another represent H; methyl; ethyl, n-propyl, 2-propyl, n-butyl, isobutyl, sec.-butyl, tert.-butyl, $CH_2F$, $CHF_2$, or $CF_3$;
or
$R^2$ and $R^4$ together with the carbon atoms connecting them, form a $C_{3-6}$-cycloaliphatic residue selected from the group consisting of cyclopropyl, cyclobutyl, cyclopentyl and cyclohexyl, unsubstituted or mono- or polysubstituted with at least one substituent selected from the group consisting of OH, methyl; ethyl, n-propyl, 2-propyl, n-butyl, isobutyl, sec.-butyl, tert.-butyl, $CH_2F$, $CHF_2$, $CF_3$, $OCH_3$ and $OCH_2CH_3$,
and $R^3$ represents H; methyl; ethyl, n-propyl, 2-propyl, n-butyl, isobutyl, sec.-butyl, or tert.-butyl,
and $R^5$ represents H; methyl; ethyl, n-propyl, 2-propyl, n-butyl, isobutyl, sec.-butyl, tert.-butyl, $CH_2F$, $CHF_2$, or $CF_3$.

**12.** The compound according to any of the preceding claims, **characterized in that** the compound is selected from the group comprising

1 2-Cyclopropyl-N-(3-hydroxy-4,4-dimethyl-pentyl)-4-methyl-6-morpholin-4-yl-pyridine-3-carboxylic acid amide;
2 2-Cyclopropyl-N-[[(1S,2R)-2-hydroxy-cyclohexyl]-methyl]-4-methyl-6-morpholin-4-yl-pyridine-3-carboxylic acid amide;
3 2-Cyclopropyl-N-[[(1R,2S)-2-hydroxy-cyclohexyl]-methyl]-4-methyl-6-morpholin-4-yl-pyridine-3-carboxylic acid amide;
4 2-Cyclopropyl-N-([2-hydroxy-cyclopentyl]-methyl)-4-methyl-6-morpholin-4-yl-pyridine-3-carboxylic acid amide;
5 N-(3-Hydroxy-4,4-dimethyl-pentyl)-2-isopropyl-4-methyl-6-morpholin-4-yl-pyridine-3-carboxylic acid amide;
6 2-Isopropyl-4-methyl-6-morpholin-4-yl-N-(4,4,4-trifluoro-3-hydroxy-butyl)-pyridine-3-carboxylic acid amide;
7 N-[[(1S,2R)-2-Hydroxy-cyclohexyl]-methyl]-2-isopropyl-4-methyl-6-morpholin-4-yl-pyridine-3-carboxylic acid amide; and
8 2-Ethoxy-N-(3-hydroxy-4,4-dimethyl-pentyl)-4-methyl-6-morpholin-4-yl-pyridine-3-carboxylic acid amide,

optionally in the form of a single stereoisomer or a mixture of stereoisomers, in the form of the free compound and/or a physiologically acceptable salt thereof.

**13.** A pharmaceutical composition comprising at least one compound according to any of the preceding claims, optionally in the form of a single stereoisomer or a mixture of stereoisomers, in the form of the free compound and/or a physiologically acceptable salt thereof, and optionally at least one pharmaceutically acceptable auxiliary.

**14.** The compound according to any of claims 1 to 12 for use in the treatment and/or prophylaxis of disorders and/or diseases which are mediated, at least in part, by KCNQ2/3 $K^+$ channels.

**15.** The composition according to claim 13 for use in the treatment and/or prophylaxis of disorders and/or diseases selected from the group consisting of pain, preferably pain selected from the group consisting of acute pain, chronic pain, neuropathic pain, muscular pain, visceral pain and inflammatory pain, epilepsy, urinary incontinence, anxiety, dependency, mania, bipolar disorders, migraine, cognitive diseases and dystonia-associated dyskinesias.

## Patentansprüche

**1.** Substituierte Verbindung der allgemeinen Formel (I)

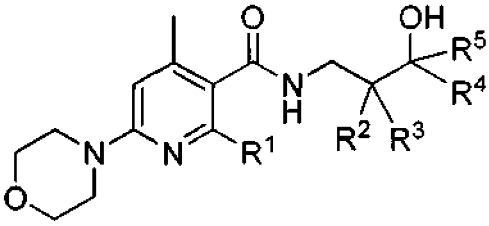

(I),

wobei

$R^1$ für einen aliphatischen $C_{2-6}$-Rest, unsubstituiert oder mono- oder polysubstituiert; oder einen cycloaliphatischen $C_{3-6}$-Rest oder einen 3- bis 6-gliedrigen heterocycloaliphatischen Rest steht, in jedem Fall unsubstituiert oder mono- oder polysubstituiert und in jedem Fall gegebenenfalls über eine aliphatische $C_{1-4}$-Gruppe verbrückt, die wiederum unsubstituiert oder mono- oder polysubstituiert sein kann;

oder für $OR^6$ steht, wobei
$R^6$ für einen aliphatischen $C_{1-6}$-Rest, unsubstituiert oder mono- oder polysubstituiert; oder einen cycloaliphatischen $C_{3-6}$-Rest, unsubstituiert oder mono- oder polysubstituiert, steht;
$R^2$ und $R^3$ unabhängig voneinander für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen aliphatischen O-$C_{1-4}$-Rest stehen, wobei der aliphatische $C_{1-4}$-Rest in jedem Fall unsubstituiert oder mono- oder polysubstituiert sein kann; oder für einen cycloaliphatischen $C_{3-6}$-Rest oder einen 3- bis 6-gliedrigen heterocycloaliphatischen Rest, in jedem Fall unsubstituiert oder mono- oder polysubstituiert, steht;
unter der Maßgabe, dass mindestens einer von $R^2$ und $R^3$ H bezeichnet oder über ein Kohlenstoffatom verbunden ist,
$R^4$ und $R^5$ unabhängig voneinander für H; $CH_2F$; $CHF_2$; $CF_3$; einen aliphatischen $C_{1-4}$-Rest,
wobei der aliphatische $C_{1-4}$-Rest unsubstituiert oder mono- oder polysubstituiert sein kann; oder einen cycloaliphatischen $C_{3-6}$-Rest oder einen 3- bis 6-gliedrigen heterocycloaliphatischen Rest, in jedem Fall unsubstituiert oder mono- oder polysubstituiert, stehen;
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
oder
$R^2$ und $R^3$
und/oder $R^4$ und $R^5$
paarweise, in jedem Fall unabhängig voneinander, zusammen mit dem Kohlenstoffatom, das sie verbindet, einen cycloaliphatischen $C_{3-10}$-Rest oder einen 3- bis 10-gliedrigen heterocycloaliphatischen Rest bilden, in jedem Fall unsubstituiert oder mono- oder polysubstituiert,
und die entsprechenden übrigen Substituenten von $R^2$ und $R^3$ jeweils unabhängig voneinander für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen O-aliphatischer-$C_{1-4}$-Rest, wobei der aliphatische $C_{1-4}$-Rest in jedem Fall unsubstituiert oder mono- oder polysubstituiert sein kann; oder für einen cycloaliphatischen $C_{3-6}$-Rest oder einen 3- bis 6-gliedrigen heterocycloaliphatischen Rest, in jedem Fall unsubstituiert oder mono- oder polysubstituiert, stehen,
unter der Maßgabe, dass mindestens einer von $R^2$ und $R^3$ H bezeichnet oder über ein Kohlenstoffatom verbunden ist,
und die entsprechenden übrigen Substituenten von $R^4$ und $R^5$ jeweils unabhängig voneinander für H; $CH_2F$; $CHF_2$; $CF_3$; einen aliphatischen $C_{1-4}$-Rest, wobei der aliphatische Rest unsubstituiert oder mono- oder polysubstituiert sein kann; oder einen cycloaliphatischen $C_{3-6}$-Rest oder einen 3- bis 6-gliedrigen heterocycloaliphatischen Rest, in jedem Fall unsubstituiert oder mono- oder polysubstituiert, stehen;
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
oder
$R^2$ und $R^4$ zusammen mit den Kohlenstoffatomen, die sie verbinden, einen cycloaliphatischen $C_{3-10}$-Rest oder einen 3- bis 10-gliedrigen heterocycloaliphatischen Rest bilden, in jedem Fall unsubstituiert oder mono- oder polysubstituiert,
und $R^3$ für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen aliphatischen O-$C_{1-4}$-Rest stehen, wobei der aliphatische $C_{1-4}$-Rest in jedem Fall unsubstituiert oder

mono- oder polysubstituiert sein kann; oder für einen cycloaliphatischen $C_{3-6}$-Rest oder einen 3- bis 6-gliedrigen heterocycloaliphatischen Rest, in jedem Fall unsubstituiert oder mono- oder polysubstituiert, steht;
und $R^5$ für H; $CH_2F$; $CHF_2$; $CF_3$; einen aliphatischen $C_{1-4}$-Rest, wobei der aliphatische $C_{1-4}$-Rest unsubstituiert oder mono- oder polysubstituiert sein kann; oder einen cycloaliphatischen $C_{3-6}$-Rest oder einen 3- bis 6-gliedrigen heterocycloaliphatischen Rest, in jedem Fall unsubstituiert oder mono- oder polysubstituiert, steht;
unter der Bedingung, dass, wenn $R^5$ einen 3- bis 6-gliedrigen heterocycloaliphatischen Rest bezeichnet, der 3- bis 6-gliedrige heterocycloaliphatische Rest über ein Kohlenstoffatom verbunden ist,
wobei eine "aliphatische Gruppe" und ein "aliphatischer Rest" in jedem Fall verzweigt oder unverzweigt, gesättigt oder ungesättigt sein können,
wobei eine "cycloaliphatische Gruppe" und ein "heterocycloaliphatischer Rest" in jedem Fall gesättigt oder ungesättigt sein können,
wobei sich "mono- oder polysubstituiert" in Bezug auf eine "aliphatische Gruppe" und einen "aliphatischen Rest" in Bezug auf die entsprechenden Reste oder Gruppen auf die Substitution eines oder mehrerer Wasserstoffatome jeweils unabhängig voneinander durch mindestens einen Substituenten bezieht, der unabhängig ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, einem NH-C(=O)-aliphatischer-$C_{1-4}$-Rest, einem N(aliphatischer-$C_{1-4}$-Rest)-C(=O)-aliphatischer-$C_{1-4}$-Rest, einem NH-S(=O)$_2$-aliphatischer-$C_{1-4}$ -Rest, einem N(aliphatischer-$C_{1-4}$-Rest)-S(=O)$_2$-aliphatischer-$C_{1-4}$-Rest, =O, OH, $OCH_2F$, $OCHF_2$, $OCF_3$, einem O-aliphatischer-$C_{1-4}$-Rest, einem O-C(=O)-aliphatischer-$C_{1-4}$-Rest, SH, $SCF_3$, einem S-aliphatischer-$C_{1-4}$-Rest, S(=O)$_2$OH, einem S(=O)-aliphatischer-$C_{1-4}$-Rest, einem S(=O)$_2$-aliphatischer-$C_{1-4}$-Rest, einem S(=O)$_2$-O-aliphatischer-$C_{1-4}$-Rest, einem S(=O)$_2$-NH-aliphatischer-$C_{1-4}$-Rest, einem S(=O)$_2$-N(aliphatischer-$C_{1-4}$-Rest)$_2$, CN, $CH_2F$, $CHF_2$, $CF_3$, CHO, COOH, einem aliphatischer-$C_{1-4}$-Rest, $CH_2OH$, $CH_2$-$OCH_3$, $C_2H_4$-OH, $C_2H_4$-$OCH_3$ $CH_2$-$CF_3$, einem C(=O)-aliphatischer-$C_{1-4}$-Rest, einem C(=O)-O-aliphatischer-$C_{1-4}$-Rest, einem cycloaliphatischen $C_{3-6}$-Rest, einem 3- bis 6-gliedrigen heterocycloaliphatischen Rest, C(=O)-$NH_2$, einem C(=O)-NH(aliphatischer-$C_{1-6}$-Rest) und einem C(=O)-N(aliphatischer-$C_{1-4}$-Rest)$_2$;
wobei sich "mono- oder polysubstituiert" in Bezug auf einen "cycloaliphatischen Rest" und einen "heterocycloaliphatischen Rest" in Bezug auf die entsprechenden Reste auf die Substitution von einem oder mehreren Wasserstoffatomen jeweils unabhängig voneinander durch mindestens einen Substituenten bezieht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, einem NH-C(=O)-aliphatischer-$C_{1-4}$-Rest, einem N(aliphatischer-$C_{1-4}$-Rest)-C(=O)-aliphatischer-$C_{1-4}$-Rest, einem NH-S(=O)$_2$-aliphatischer-$C_{1-4}$-Rest, einem N(aliphatischer-$C_{1-4}$-Rest)-S(=O)$_2$-aliphatischer-$C_{1-4}$-Rest, =O, OH, $OCH_2F$, $OCHF_2$, $OCF_3$, einem O-aliphatischer-$C_{1-4}$-Rest, einem O-C(=O)-aliphatischer-$C_{1-4}$-Rest, SH, $SCF_3$, einem S-aliphatischer-$C_{1-4}$-Rest, S(=O)$_2$OH, einem S(=O)-aliphatischer-$C_{1-4}$-Rest, einem S(=O)$_2$-aliphatischer-$C_{1-4}$-Rest, einem S(=O)$_2$-O-aliphatischer-$C_{1-4}$-Rest, einem S(=O)$_2$-NH-aliphatischer-$C_{1-4}$-Rest, einem S(=O)$_2$-N(aliphatischer-$C_{1-4}$-Rest)$_2$, CN, $CH_2F$, $CHF_2$, $CF_3$, CHO, COOH, einem aliphatischen $C_{1-4}$-Rest, $CH_2OH$, $CH_2$-$OCH_3$, $C_2H_4$-OH, $C_2H_4$-$OCH_3$ $CH_2$-$CF_3$, einem C(=O)-aliphatischen-$C_{1-4}$-Rest, einem C(=O)-O-aliphatischer-$C_{1-4}$-Rest, einem cycloaliphatischen $C_{3-6}$ Rest, einem 3- bis 6-gliedrigen heterocycloaliphatischen Rest, C(=O)-$NH_2$, einem C(=O)-NH(aliphatischen-$C_{1-4}$-Rest) und einem C(=O)-N(aliphatischen-$C_{1-4}$-Rest)$_2$;
gegebenenfalls in Form eines einzelnen Stereoisomers oder eines Gemischs von Stereoisomeren, in Form der freien Verbindung und/oder eines physiologisch unbedenklichen Salzes davon.

**2.** Verbindung nach Anspruch 1, **dadurch gekennzeichnet, dass**
$R^1$ für einen aliphatischen $C_{2-6}$-Rest, unsubstituiert oder mono- oder polysubstituiert mit
mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder für einen cycloaliphatischen $C_{3-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
und wobei der cycloaliphatische $C_{3-6}$-Rest gegebenenfalls über eine aliphatische $C_{1-4}$-Gruppe verbrückt sein kann,

die wiederum unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

oder für $OR^6$ steht, wobei

$R^6$ für einen aliphatischen $C_{1-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)-aliphatischer-$C_{1-4}$-Rest, CN und einem aliphatischen $C_{1-4}$-Rest;

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

$R^2$ und $R^3$ unabhängig voneinander für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen O-aliphatischer-$C_{1-4}$-Rest stehen,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

oder einen cycloaliphatischen $C_{3-6}$-Rest oder einen 3- bis 6-gliedrigen heterocycloaliphatischen Rest, in jedem Fall unsubstituiert oder mono- oder polysubstituiert mit mindestens einem Substituenten, stehen, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

unter der Maßgabe, dass mindestens einer von $R^2$ und $R^3$ H bezeichnet oder über ein Kohlenstoffatom verbunden ist,

$R^4$ und $R^5$ unabhängig voneinander für H; $CH_2F$; $CHF_2$; $CF_3$; oder einen aliphatischen $C_{1-4}$-Rest stehen,

wobei der aliphatische $C_{1-4}$-Rest unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

oder einen cycloaliphatischen $C_{3-6}$-Rest oder einen 3- bis 6 -gliedrigen heterocycloaliphatischen Rest, in jedem Fall unsubstituiert oder mono- oder polysubstituiert mit mindestens einem Substituenten, stehen, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=0)-aliphatischer-$C_{1-4}$-Rest, einem S(=O)$_2$-aliphatischer-$C_{1-4}$-Rest, CN und einem aliphatischen $C_{1-4}$-Rest, unter der Bedingung, dass, wenn $R^4$ und/oder $R^5$ einen 3- bis 6-gliedrigen heterocycloaliphatischen Rest bezeichnet/n, der 3- bis 6-gliedrige heterocycloaliphatische Rest über ein Kohlenstoffatom verbunden sind,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

oder

$R^2$ und $R^3$

und/oder $R^4$ und $R^5$

paarweise in jedem Fall unabhängig voneinander zusammen mit dem Kohlenstoffatom, das sie verbindet, einen cycloaliphatischen $C_{3-10}$-Rest oder einen 3- bis 10-gliedrigen heterocycloaliphatischen Rest, in jedem Fall unsubstituiert oder mono- oder polysubstituiert mit mindestens einem Substituenten bilden, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,

und die entsprechenden übrigen Substituenten aus $R^2$ und $R^3$ jeweils unabhängig voneinander für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen O-aliphatischer-$C_{1-4}$-Rest,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder einen cycloaliphatischen $C_{3-6}$-Rest oder einen 3- bis 6-gliedrigen heterocycloaliphatischen Rest, in jedem Fall unsubstituiert oder mono- oder polysubstituiert mit mindestens einem Substituenten, stehen, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer- $C_{1-4}$-Rest,
unter der Maßgabe, dass mindestens einer von $R^2$ und $R^3$ H bezeichnet oder über ein Kohlenstoffatom verbunden ist, und die entsprechenden übrigen Substituenten von $R^4$ und $R^5$ jeweils unabhängig voneinander für H; $CH_2F$; $CHF_2$; $CF_3$; einen aliphatischen $C_{1-4}$-Rest stehen,
wobei der aliphatische $C_{1-4}$-Rest unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder einen cycloaliphatischen $C_{3-6}$-Rest oder einen 3- bis 6-gliedrigen heterocycloaliphatischen Rest, in jedem Fall unsubstituiert oder mono- oder polysubstituiert mit mindestens einem Substituenten, stehen, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder
$R^2$ und $R^4$ zusammen mit den Kohlenstoffatomen, die sie verbinden, einen cycloaliphatischen $C_{3-10}$-Rest oder einen 3- bis 10-gliedrigen heterocycloaliphatischen Rest, in jedem Fall unsubstituiert oder mono- oder polysubstituiert mit mindestens einem Substituenten, stehen, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
und der übrige Substituent $R^3$ für H; F; Cl; Br; I; CN; $CH_2F$; $CHF_2$; $CF_3$; OH; $OCH_2F$; $OCHF_2$; $OCF_3$; $SCF_3$; einen aliphatischen $C_{1-4}$-Rest oder einen O-aliphatischer-$C_{1-4}$-Rest steht,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder für einen cycloaliphatischen $C_{3-6}$-Rest oder einen 3- bis 6-gliedrigen heterocycloaliphatischen Rest, in jedem Fall unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
und der übrige Substituent $R^5$ für H; $CH_2F$; $CHF_2$; $CF_3$; $OCH_2F$; $OCHF_2$; $OCF_3$; $SCF_3$; einen aliphatischen $C_{1-4}$-Rest steht,
wobei der aliphatische $C_{1-4}$-Rest unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder für einen cycloaliphatischen $C_{3-6}$-Rest oder einen 3- bis 6 -gliedrigen heterocycloaliphatischen Rest, in jedem

Fall unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
unter der Bedingung, dass, wenn $R^5$ einen 3- bis 6-gliedrigen heterocycloaliphatischen Rest bezeichnet, der 3- bis 6-gliedrige heterocycloaliphatische Rest über ein Kohlenstoffatom verbunden ist,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest.

3. Verbindung nach Anspruch 1 oder 2, **dadurch gekennzeichnet, dass**

$R^1$ für einen aliphatischen $C_{2-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,

wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder für einen cycloaliphatischen $C_{3-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest,
oder für $OR^6$ steht, wobei
$R^6$ für einen aliphatischen $C_{1-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN, und einem aliphatischen $C_{1-4}$-Rest;
wobei der aliphatische $C_{1-4}$-Rest in jedem Fall unsubstituiert oder mono- oder polysubstituiert sein kann, mit mindestens einem Substituenten, der ausgewählt ist aus der Gruppe, bestehend aus F, Cl, Br, I, OH, $OCH_2F$, $OCHF_2$, $OCF_3$, $CH_2F$, $CHF_2$, $CF_3$ und einem unsubstituierten O-aliphatischer-$C_{1-4}$-Rest.

4. Verbindung nach einem der vorstehenden Ansprüche, **dadurch gekennzeichnet, dass**

$R^1$ für einen aliphatischen $C_{2-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,

wobei der aliphatische $C_{1-4}$-Rest in jedem Fall unsubstituiert ist, oder für einen cycloaliphatischen $C_{3-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$, SH, $SCF_3$, einem S-aliphatischer-$C_{1-4}$-Rest, einem S(=O)-aliphatischer-$C_{1-4}$-Rest, einem S(=O)$_2$-aliphatischer-$C_{1-4}$-Rest, CN und einem aliphatischen $C_{1-4}$-Rest,
wobei der aliphatische $C_{1-4}$-Rest in jedem Fall unsubstituiert ist, oder für $OR^6$ steht, wobei
$R^6$ für einen aliphatischen $C_{1-6}$-Rest, unsubstituiert oder mono- oder polysubstituiert mit mindestens einem Substituenten, steht, der ausgewählt ist aus der Gruppe, bestehend aus F, Cl, Br, I, $NO_2$, $NH_2$, einem NH(aliphatischer-$C_{1-4}$-Rest), einem N(aliphatischer-$C_{1-4}$-Rest)$_2$, OH, =O, einem O-aliphatischer-$C_{1-4}$-Rest, $OCH_2F$, $OCHF_2$, $OCF_3$,

SH, $SCF_3$, einem S-aliphatischer-$C_{1-4}$-Rest, einem S(=O)-aliphatischer-$C_{1-4}$-Rest, einem $S(=O)_2$-aliphatischer-$C_{1-4}$-Rest, CN und einem aliphatischen $C_{1-4}$-Rest, wobei der aliphatische $C_{1-4}$-Rest in jedem Fall unsubstituiert ist.

**5.** Verbindung nach einem der vorstehenden Ansprüche, **dadurch gekennzeichnet, dass**

$R^1$ für einen aliphatischen $C_{2-6}$-Rest, unsubstituiert oder mono- oder polysubstituiert mit mindestens einem Substituenten, steht, der ausgewählt ist aus der Gruppe, bestehend aus F, Cl, Br, I, OH, und einem unsubstituierten O-aliphatischer-$C_{1-4}$-Rest, oder für einen cycloaliphatischen $C_{3-6}$-Rest, unsubstituiert oder mono- oder polysubstituiert mit mindestens einem Substituenten, steht, der ausgewählt ist aus der Gruppe, bestehend aus F, Cl, Br, I, OH und einem unsubstituierten O-aliphatischer-$C_{1-4}$-Rest,

oder für $OR^6$ steht, wobei $R^6$ für einen aliphatischen $C_{1-4}$-Rest, unsubstituiert oder mono- oder polysubstituiert mit mindestens einem Substituenten, steht, der ausgewählt ist aus der Gruppe, bestehend aus F, Cl, Br, I, OH und einem unsubstituierten O-aliphatischer-$C_{1-4}$-Rest.

**6.** Verbindung nach einem der vorstehenden Ansprüche, **dadurch gekennzeichnet, dass**

$R^1$ für Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, Ethenyl oder Propenyl ($-CH_2CH=CH_2$, $-CH=CH-CH_3$, $-C(=CH_2)-CH_3$),

Cyclopropyl, Cyclobutyl, Cyclopentyl oder Cyclohexyl steht oder für $OR^6$ steht, wobei $R^6$ für Methyl, Ethyl, n-Propyl, 2-Propyl, n-Butyl, Isobutyl, sec-Butyl, tert-Butyl, $CH_2OH$, $CH_2OCH_3$, $CH_2CH_2OH$, $CH_2CH_2OCH_3$ und $CH(OH)CH_2OH$ steht.

**7.** Verbindung nach einem der vorstehenden Ansprüche, **dadurch gekennzeichnet, dass**
$R^2$ und $R^3$ unabhängig voneinander für H; F; Cl; Br; I; $CH_2F$; $CHF_2$; $CF_3$; OH; $OCH_2F$; $OCHF_2$; $OCF_3$; einen aliphatischen $C_{1-4}$-Rest oder einen O-aliphatischer-$C_{1-4}$-Rest stehen,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder für einen aliphatischen $C_{3-6}$-Rest oder einen 3- bis 6 gliedrigen heterocycloaliphatischen Rest stehen, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
unter der Maßgabe, dass mindestens einer von $R^2$ und $R^3$ H bezeichnet oder über ein Kohlenstoffatom verbunden ist,
$R^4$ und $R^5$ unabhängig voneinander für H; $CH_2F$; $CHF_2$; $CF_3$; einen aliphatischen $C_{1-4}$-Rest stehen,
wobei der aliphatische $C_{1-4}$-Rest unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder für einen cycloaliphatischen $C_{3-6}$-Rest oder einen 3- bis 6-gliedrigen heterocycloaliphatischen Rest, stehen, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
oder
$R^2$ und $R^3$
und/oder $R^4$ und $R^5$
paarweise oder jeweils unabhängig voneinander zusammen mit dem Kohlenstoffatom, das sie verbindet, einen cycloaliphatischen $C_{3-10}$-Rest oder einen 3- bis 10-gliedrigen heterocycloaliphatischen Rest bilden, in jedem Fall

unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
und die entsprechenden übrigen Substituenten aus $R^2$ und $R^3$ jeweils unabhängig voneinander für H; F; Cl; Br; I; $CH_2F$; $CHF_2$; $CF_3$; OH; $OCH_2F$; $OCHF_2$; $OCF_3$; einen aliphatischen $C_{1-4}$-Rest oder einen O-aliphatischer-$C_{1-4}$-Rest stehen,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder für einen cycloaliphatischen $C_{3-6}$-Rest oder einen 3- bis 6-gliedrigen heterocycloaliphatischen Rest stehen, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
unter der Maßgabe, dass mindestens einer von $R^2$ und $R^3$ H bezeichnet oder über ein Kohlenstoffatom verbunden ist, und die entsprechenden übrigen Substituenten von $R^4$ und $R^5$ jeweils unabhängig voneinander für H; $CH_2F$; $CHF_2$; $CF_3$; einen aliphatischen $C_{1-4}$-Rest stehen,
wobei der aliphatische $C_{1-4}$-Rest unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder für einen cycloaliphatischen $C_{3-6}$-Rest oder einen 3- bis 6-gliedrigen heterocycloaliphatischen Rest stehen, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder
$R^2$ und $R^4$ zusammen mit den Kohlenstoffatomen, die sie verbinden, einen cycloaliphatischen $C_{3-10}$-Rest oder einen 3- bis 10-gliedrigen heterocycloaliphatischen Rest bilden, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
und der übrige Substituent $R^3$ für H; F; Cl; Br; I; $CH_2F$; $CHF_2$; $CF_3$; OH; $OCH_2F$; $OCHF_2$; $OCF_3$; einen aliphatischen $C_{1-4}$-Rest oder einen O-aliphatischer-$C_{1-4}$-Rest steht,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
oder für einen cycloaliphatischen $C_{3-6}$-Rest oder einen 3- bis 6-gliedrigen heterocycloaliphatischen Rest steht, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,
und der übrige Substituent $R^5$ für H; $CH_2F$; $CHF_2$; $CF_3$; einen aliphatischen $C_{1-4}$-Rest steht,
wobei der aliphatische $C_{1-4}$-Rest unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest,

oder für einen cycloaliphatischen $C_{3-6}$-Rest oder einen 3- bis 6-gliedrigen heterocycloaliphatischen Rest steht, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$, $OCH_2F$, $OCHF_2$, $OCF_3$ und einem aliphatischen $C_{1-4}$-Rest,
unter der Bedingung, dass, wenn $R^5$ einen 3- bis 6-gliedrigen heterocycloaliphatischen Rest bezeichnet, der 3- bis 6-gliedrige heterocycloaliphatische Rest über ein Kohlenstoffatom verbunden ist,
wobei der aliphatische $C_{1-4}$-Rest in jedem Fall unsubstituiert oder mono- oder polysubstituiert mit mindestens einem Substituenten sein kann, der ausgewählt ist aus der Gruppe, bestehend aus F, Cl, Br, I, OH und einem unsubstituierten O-aliphatischer-$C_{1-4}$-Rest.

**8.** Verbindung nach einem der vorstehenden Ansprüche, **dadurch gekennzeichnet, dass**
$R^2$ und $R^3$ unabhängig voneinander für H; F; Cl; Br; I; $CH_2F$; $CHF_2$; $CF_3$; OH; einen aliphatischen $C_{1-4}$-Rest oder einen O-aliphatischer-$C_{1-4}$-Rest stehen,
wobei der aliphatische $C_{1-4}$-Rest in jedem Fall unsubstituiert ist,
oder für einen cycloaliphatischen $C_{3-6}$-Rest oder einen 3- bis 6-gliedrigen heterocycloaliphatischen Rest stehen, in jedem Fall unsubstituiert,
unter der Maßgabe, dass mindestens einer von $R^2$ und $R^3$ H bezeichnet oder über ein Kohlenstoffatom verbunden ist,
$R^4$ und $R^5$ unabhängig voneinander für H; $CH_2F$; $CHF_2$; $CF_3$; einen aliphatischen $C_{1-4}$-Rest stehen,
wobei der aliphatische $C_{1-4}$-Rest unsubstituiert ist,
oder für einen cycloaliphatischen $C_{3-6}$-Rest oder einen 3- bis 6-gliedrigen heterocycloaliphatischen Rest stehen, in jedem Fall unsubstituiert,
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
oder
$R^2$ und $R^3$
und/oder $R^4$ und $R^5$
paarweise oder jeweils unabhängig voneinander zusammen mit dem Kohlenstoffatom, das sie verbindet, einen cycloaliphatischen $C_{3-10}$-Rest oder einen 3- bis 10-gliedrigen heterocycloaliphatischen Rest bilden, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$ und einem aliphatischen $C_{1-4}$-Rest,
wobei der aliphatische $C_{1-4}$-Rest in jedem Fall unsubstituiert ist,
und die entsprechenden übrigen Substituenten von $R^2$ und $R^3$ jeweils unabhängig voneinander für H; F; Cl; Br; I; $CH_2F$; $CHF_2$; $CF_3$; OH; einen aliphatischen $C_{1-4}$-Rest oder einen O-aliphatischer- $C_{1-4}$-Rest stehen,
wobei der aliphatische $C_{1-4}$-Rest in jedem Fall unsubstituiert ist,
oder für einen cycloaliphatischen $C_{3-6}$-Rest oder einen 3- bis 6-gliedrigen heterocycloaliphatischen Rest stehen, in jedem Fall unsubstituiert,
mit der Maßgabe, dass mindestens einer von $R^2$ und $R^3$ H bezeichnet oder über ein Kohlenstoffatom verbunden ist und die entsprechenden übrigen Substituenten von $R^4$ und $R^5$ jeweils unabhängig für H; $CH_2F$; $CHF_2$; $CF_3$; einen aliphatischen $C_{1-4}$-Rest stehen,
wobei der aliphatische $C_{1-4}$-Rest unsubstituiert ist,
oder für einen cycloaliphatischen $C_{3-6}$-Rest oder einen 3- bis 6-gliedrigen heterocycloaliphatischen Rest stehen, in jedem Fall unsubstituiert,
unter der Bedingung, dass, wenn $R^4$ und/oder $R^5$ einen 3- bis 6 gliedrigen heterocycloaliphatischen Rest bezeichnet/n, der 3- bis 6-gliedrige heterocycloaliphatische Rest über ein Kohlenstoffatom verbunden ist,
oder
$R^2$ und $R^4$ zusammen mit den Kohlenstoffatomen, die sie verbinden, einen cycloaliphatischen $C_{3-10}$-Rest oder einen 3- bis 10-gliedrigen heterocycloaliphatischen Rest bilden, in jedem Fall unsubstituiert oder mono- oder polysubstituiert mit mindestens einem Substituenten, der ausgewählt ist aus der Gruppe, bestehend aus F, Cl, Br, I, OH, einem O-aliphatischer-$C_{1-4}$-Rest, $CH_2F$, $CHF_2$, $CF_3$ und einem aliphatischen $C_{1-4}$-Rest,
wobei der aliphatische $C_{1-4}$-Rest in jedem Fall unsubstituiert ist,
und der übrige Substituent $R^3$ für H; F; Cl; Br; I; $CH_2F$; $CHF_2$; $CF_3$; OH; einen aliphatischen $C_{1-4}$-Rest oder einen O-aliphatischer-$C_{1-4}$ -Rest steht,
wobei der aliphatische $C_{1-4}$-Rest in jedem Fall unsubstituiert ist,
oder für einen cycloaliphatischen $C_{3-6}$-Rest oder einen 3- bis 6-gliedrigen heterocycloaliphatischen Rest steht, in jedem Fall unsubstituiert,
und der übrige Substituent $R^5$ für H; $CH_2F$; $CHF_2$; $CF_3$; einen aliphatischen $C_{1-4}$-Rest steht,
wobei der aliphatische $C_{1-4}$-Rest unsubstituiert ist,

oder für einen cycloaliphatischen $C_{3-6}$-Rest oder einen 3- bis 6-gliedrigen heterocycloaliphatischen Rest steht, in jedem Fall unsubstituiert,
unter der Bedingung, dass, wenn $R^5$ einen 3- bis 6-gliedrigen heterocycloaliphatischen Rest bezeichnet, der 3- bis 6-gliedrige heterocycloaliphatische Rest über ein Kohlenstoffatom verbunden ist.

**9.** Verbindung nach einem der vorstehenden Ansprüche, **dadurch gekennzeichnet, dass**
$R^2$ und $R^3$ unabhängig voneinander für H; OH; einen unsubstituierten aliphatischen $C_{1-4}$-Rest oder einen unsubstituierten O-aliphatischer-$C_{1-4}$-Rest stehen, unter der Maßgabe, dass mindestens einer von $R^2$ und $R^3$ H bezeichnet oder über ein Kohlenstoffatom verbunden ist,
$R^4$ und $R^5$ unabhängig voneinander für H; $CH_2F$; $CHF_2$; $CF_3$; einen unsubstituierten aliphatischen $C_{1-4}$-Rest stehen,
oder
$R^2$ und $R^3$
oder $R^4$ und $R^5$
paarweise oder jeweils unabhängig voneinander zusammen mit dem Kohlenstoffatom, das sie verbindet, einen cycloaliphatischen $C_{3-10}$-Rest bilden, unsubstituiert oder mono- oder polysubstituiert mit mindestens einem Substituenten, der ausgewählt ist aus der Gruppe, bestehend aus F, Cl, Br, I, OH, $CH_2F$, $CHF_2$, $CF_3$ einem O-aliphatischer-$C_{1-4}$-Rest und einem unsubstituierten aliphatischen $C_{1-4}$-Rest,
und die entsprechenden übrigen Substituenten von $R^2$ und $R^3$ jeweils unabhängig voneinander für H; OH; einen unsubstituierten aliphatischen $C_{1-4}$-Rest oder einen unsubstituierten O-aliphatischer-$C_{1-4}$-Rest stehen, unter der Maßgabe, dass mindestens einer von $R^2$ und $R^3$ H bezeichnet oder über ein Kohlenstoffatom verbunden ist,
und die entsprechenden übrigen Substituenten von $R^4$ und $R^5$ jeweils unabhängig voneinander für H; $CH_2F$; $CHF_2$; $CF_3$; einen unsubstituierten aliphatischen $C_{1-4}$-Rest stehen,
oder
$R^2$ und $R^4$ zusammen mit den Kohlenstoffatomen, die sie verbinden, einen cycloaliphatischen $C_{3-10}$-Rest bilden, unsubstituiert oder mono- oder polysubstituiert mit mindestens einem Substituenten, der ausgewählt ist aus der Gruppe, bestehend aus F, Cl, Br, I, OH, einem unsubstituierten O-aliphatischer-$C_{1-4}$-Rest, $CH_2F$, $CHF_2$, $CF_3$ und einem unsubstituierten aliphatischen $C_{1-4}$-Rest,
und der übrige Substituent $R^3$ für H; F; Cl; Br; I; OH; einen unsubstituierten aliphatischen $C_{1-4}$-Rest oder einen unsubstituierten O-aliphatischer-$C_{1-4}$-Rest steht,
und der übrige Substituent $R^5$ für H; $CH_2F$; $CHF_2$; $CF_3$; einen unsubstituierten aliphatischen $C_{1-4}$-Rest steht.

**10.** Verbindung nach einem der vorstehenden Ansprüche, **dadurch gekennzeichnet, dass**
$R^2$ und $R^3$ unabhängig voneinander für H; Methyl; Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, $OCH_3$ oder $OCH_2CH_3$ stehen, unter der Maßgabe, dass mindestens einer von $R^2$ und $R^3$ H bezeichnet oder über ein Kohlenstoffatom verbunden ist,
$R^4$ und $R^5$ unabhängig voneinander für H; Methyl; Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, $CH_2F$; $CHF_2$; $CF_3$ stehen;
oder
$R^2$ und $R^3$
oder $R^4$ und $R^5$
paarweise, in jedem Fall unabhängig voneinander, zusammen mit dem Kohlenstoffatom, das sie verbindet, einen cycloaliphatischen $C_{3-6}$-Rest bilden, der ausgewählt ist aus der Gruppe, bestehend aus Cyclopropyl, Cyclobutyl, Cyclopentyl und Cyclohexyl, unsubstituiert oder mono- oder polysubstituiert mit mindestens einem Substituenten, der ausgewählt ist aus der Gruppe, bestehend aus F, Cl, Br, I, OH, Methyl; Ethyl, n-Propyl, 2-Propyl, n-Butyl, Isobutyl, sec-Butyl, tert-Butyl, $CH_2F$, $CHF_2$, $CF_3$, $OCH_3$ und $OCH_2CH_3$,
und die entsprechenden übrigen Substituenten von $R^2$ und $R^3$ jeweils unabhängig voneinander für H; Methyl; Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, $OCH_3$ oder $OCH_2CH_3$ stehen, mit der Maßgabe, dass mindestens einer von $R^2$ und $R^3$ H bezeichnet oder über ein Kohlenstoffatom verbunden ist, und die entsprechenden übrigen Substituenten von $R^4$ und $R^5$ jeweils unabhängig voneinander für H; Methyl; Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, $CH_2F$; $CHF_2$; $CF_3$ stehen;
oder
$R^2$ und $R^4$ zusammen mit den Kohlenstoffatomen, die sie verbinden, einen cycloaliphatischen $C_{3-6}$-Rest bilden, der ausgewählt ist aus der Gruppe, bestehend aus Cyclopropyl, Cyclobutyl, Cyclopentyl und Cyclohexyl, unsubstituiert oder mono- oder polysubstituiert mit mindestens einem Substituenten, der ausgewählt ist aus der Gruppe, bestehend aus F, Cl, Br, I, OH, Methyl; Ethyl, n-Propyl, 2-Propyl, n-Butyl, Isobutyl, sec-Butyl, tert-Butyl, $CH_2F$, $CHF_2$, $CF_3$, $OCH_3$ und $OCH_2CH_3$,
und der entsprechende übrige Substituent $R^3$ für H; Methyl; Ethyl, n-Propyl, 2-Propyl, n-Butyl, Isobutyl, sec-Butyl,

tert-Butyl, n-Pentyl, Isopentyl, Neopentyl, n-Hexyl, $CH_2$-$CH(CH_3)(C_2H_5)$, $C(CH_3)_2(C_2H_5)$, $OCH_3$ oder $OCH_2CH_3$ steht,
und der entsprechende übrige Substituent $R^5$ für H; Methyl; Ethyl, n-Propyl, 2-Propyl, n-Butyl, Isobutyl, sec-Butyl, tert-Butyl, n-Pentyl, Isopentyl, Neopentyl, n-Hexyl, $CH_2$-$CH(CH_3)(C_2H_5)$, $C(CH_3)_2(C_2H_5)$, $CH_2F$; $CHF_2$; $CF_3$ steht.

**11.** Verbindung nach einem der vorstehenden Ansprüche, **dadurch gekennzeichnet, dass**

$R^1$ für Ethyl, n-Propyl, 2-Propyl, n-Butyl, Isobutyl, sec-Butyl, tert-Butyl, Cyclopropyl, Cyclobutyl, Cyclopentyl, oder Cyclohexyl steht,

oder für $OR^6$ steht, wobei
$R^6$ für Methyl, Ethyl, n-Propyl, 2-Propyl, n-Butyl, Isobutyl, sec-Butyl oder tert-Butyl steht,
R' und $R^3$ unabhängig voneinander für H; Methyl; Ethyl, n-Propyl, 2-Propyl, n-Butyl, Isobutyl, sec-Butyl oder tert-Butyl stehen,
$R^4$ und $R^5$ unabhängig voneinander für H; Methyl; Ethyl, n-Propyl, 2-Propyl, n-Butyl, Isobutyl, sec-Butyl, tert-Butyl, $CH_2F$, $CHF_2$ oder $CF_3$ stehen;
oder
$R^2$ und $R^3$
oder $R^4$ und $R^5$
paarweise, in jedem Fall unabhängig voneinander, zusammen mit dem Kohlenstoffatom, das sie verbindet, einen cycloaliphatischen $C_{3-6}$-Rest bilden, der ausgewählt ist aus der Gruppe, bestehend aus Cyclopropyl, Cyclobutyl, Cyclopentyl und Cyclohexyl, unsubstituiert oder mono- oder polysubstituiert mit mindestens einem Substituenten, der ausgewählt ist aus der Gruppe, bestehend aus OH, Methyl; Ethyl, n-Propyl, 2-Propyl, n-Butyl, Isobutyl, sec-Butyl, $CH_2F$, $CHF_2$, $CF_3$, $OCH_3$ und $OCH_2CH_3$,
und die entsprechenden übrigen Substituenten von $R^2$ und $R^3$ unabhängig voneinander für H, Methyl, Ethyl, n-Propyl, 2-Propyl, n-Butyl, Isobutyl, sec-Butyl oder tert-Butyl stehen,
und die entsprechenden übrigen Substituenten von $R^4$ und $R^5$ unabhängig voneinander für H; Methyl; Ethyl, n-Propyl, 2-Propyl, n-Butyl, Isobutyl, sec-Butyl, tert-Butyl, $CH_2F$, $CHF_2$ oder $CF_3$ stehen;
oder
$R^2$ und $R^4$ zusammen mit den Kohlenstoffatomen, die sie verbinden, einen cycloaliphatischen $C_{3-6}$-Rest bilden, der ausgewählt ist aus der Gruppe, bestehend aus Cyclopropyl, Cyclobutyl, Cyclopentyl und Cyclohexyl, unsubstituiert oder mono- oder polysubstituiert mit mindestens einem Substituenten, der ausgewählt ist aus der Gruppe, bestehend aus OH, Methyl; Ethyl, n-Propyl, 2-Propyl, n-Butyl, Isobutyl, sec-Butyl, tert-Butyl, $CH_2F$, $CHF_2$, $CF_3$, $OCH_3$ und $OCH_2CH_3$,
und $R^3$ für H; Methyl; Ethyl, n-Propyl, 2-Propyl, n-Butyl, Isobutyl, sec-Butyl oder tert-Butyl steht,
und $R^5$ für H; Methyl; Ethyl, n-Propyl, 2-Propyl, n-Butyl, Isobutyl, sec-Butyl, tert-Butyl, $CH_2F$, $CHF_2$ oder $CF_3$ steht.

**12.** Verbindung nach einem der vorstehenden Ansprüche, **dadurch gekennzeichnet, dass** die Verbindung aus der Gruppe ausgewählt ist, die Folgendes umfasst:

1. 2-Cyclopropyl-N-(3-hydroxy-4,4-dimethylpentyl)-4-methyl-6-morpholin-4-ylpyridin-3-carbonsäureamid;
2. 2-Cyclopropyl-N-[[(1S,2R)-2-hydroxycyclohexyl]-methyl]-4-methyl-6-morpholin-4-yl-pyridin-3-carbonsäureamid;
3. 2-Cyclopropyl-N-[[(1R,2S)-2-hydroxycyclohexyl]-methyl]-4-methyl-6-morpholin-4-yl-pyridin-3-carbonsäureamid;
4. 2-Cyclopropyl-N-([2-hydroxycyclopentyl]-methyl)-4-methyl-6-morpholin-4-ylpyridin-3-carbonsäureamid;
5. N-(3-Hydroxy-4,4-dimethylpentyl)-2-isopropyl-4-methyl-6-morpholin-4-ylpyridin-3-carbonsäureamid;
6. 2-Isopropyl-4-methyl-6-morpholin-4-yl-N-(4,4,4-trifluor-3-hydroxybutyl)-pyridin-3-carbonsäureamid;
7. N-[[(1S,2R)-2-Hydroxycyclohexyl]-methyl]-2-isopropyl-4-methyl-6-morpholin-4-yl-pyridin-3-carbonsäureamid; und
8. 2-Ethoxy-N-(3-hydroxy-4,4-dimethylpentyl)-4-methyl-6-morpholin-4-yl-pyridin-3-carbonsäureamid,

gegebenenfalls in Form eines einzelnen Stereoisomers oder eines Gemischs von Stereoisomeren, in Form der freien Verbindung und/oder eines physiologisch unbedenklichen Salzes davon.

**13.** Pharmazeutische Zusammensetzung, umfassend mindestens eine Verbindung nach einem der vorstehenden Ansprüche, gegebenenfalls in Form eines einzelnen Stereoisomers oder eines Gemischs von Stereoisomeren, in Form der freien Verbindung und/oder eines physiologisch unbedenklichen Salzes, und gegebenenfalls mindestens einen

pharmazeutisch unbedenklichen Hilfsstoff.

**14.** Verbindung nach einem der Ansprüche 1 bis 12 zur Verwendung bei der Behandlung und/oder Prophylaxe von Störungen und/oder Krankheiten, die zumindest teilweise über KCNQ2/3-$K^+$-Kanäle vermittelt werden.

**15.** Zusammensetzung nach Anspruch 13 zur Verwendung bei der Behandlung und/oder Prophylaxe von Störungen und/oder Erkrankungen, die ausgewählt sind aus der Gruppe bestehend aus Schmerz, vorzugsweise Schmerz, der ausgewählt ist aus der Gruppe bestehend aus akutem Schmerz, chronischem Schmerz, neuropathischem Schmerz, Muskelschmerz, viszeralem Schmerz und entzündlichem Schmerz, Epilepsie, Harninkontinenz, Angst, Abhängigkeit, Manie, bipolaren Störungen, Migräne, kognitiven Erkrankungen und dystonieassoziierten Dyskinesien.

## Revendications

**1.** Composé substitué de formule générale (I)

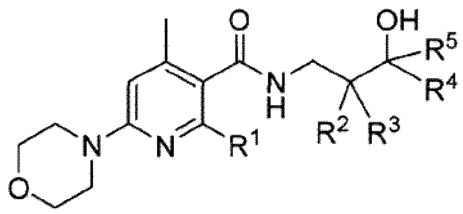

**(I)**,

dans laquelle :

$R^1$ représente un résidu aliphatique en $C_2$ à $C_6$, non substitué ou mono- ou polysubstitué ; ou un résidu cycloaliphatique en $C_3$ à $C_6$ ou bien un résidu hétérocycloaliphatique tri- à hexagonal, dans chaque cas non substitué ou mono- ou polysubstitué, et dans chaque cas éventuellement ponté par l'intermédiaire d'un groupe aliphatique en $C_1$ à $C_4$, qui peut lui-même être non substitué ou mono- ou polysubstitué ;

ou représente un groupe $OR^6$, dans lequel :

$R^6$ représente un résidu aliphatique en $C_1$ à $C_6$,
non substitué ou mono- ou polysubstitué ; ou un résidu cycloaliphatique en $C_3$ à $C_6$, non substitué ou mono- ou polysubstitué ;
$R^2$ et $R^3$, indépendamment l'un de l'autre, représentent H ; F ; Cl ; Br ; I ; CN ; $CH_2F$ ; $CHF_2$ ; $CF_3$ ; OH ; $OCH_2F$ ; $OCHF_2$ ; $OCF_3$ ; $SCF_3$ ; un résidu aliphatique en $C_1$ à $C_4$ ou un résidu O-aliphatique en $C_1$ à $C_4$, ledit résidu aliphatique en $C_1$ à $C_4$ pouvant dans chaque cas être non substitué ou mono- ou polysubstitué ; ou un résidu cycloaliphatique en $C_3$ à $C_6$, ou bien un résidu hétérocycloaliphatique tri- à hexagonal, dans chaque cas non substitué ou mono- ou polysubstitué ;
sous réserve qu'au moins un de $R^2$ et $R^3$ représente H ou soit lié par l'intermédiaire d'un atome de carbone,
$R^4$ et $R^5$, indépendamment l'un de l'autre, représentent H ; $CH_2F$ ; $CHF_2$ ; $CF_3$ ; un résidu aliphatique en $C_1$ à $C_4$, ledit résidu aliphatique en $C_1$ à $C_4$ pouvant être non substitué ou mono- ou polysubstitué ; ou un résidu cycloaliphatique en $C_3$ à $C_6$ ou bien un résidu hétérocycloaliphatique tri- à hexagonal, dans chaque cas non substitué ou mono- ou polysubstitué ;
à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,
ou
$R^2$ et $R^3$
et/ou $R^4$ et $R^5$,
par paires, dans chaque cas indépendamment l'un de l'autre, conjointement avec l'atome de carbone les connectant, forment un résidu cycloaliphatique en $C_3$ à $C_{10}$ ou un résidu hétérocycloaliphatique tri- à décagonal, dans chaque cas non substitué ou mono- ou polysubstitué,
et les substituants respectifs restant de $R^2$ et $R^3$ chacun indépendamment l'un de l'autre, représentent H ; F ;

Cl ; Br ; I ; CN ; $CH_2F$ ; $CHF_2$ ; $CF_3$ ; OH ; $OCH_2F$ ; OCHF ; $OCF_3$ ; $SCF_3$ ; un résidu aliphatique en $C_1$ à $C_4$, ou un résidu O-aliphatique en $C_1$ à $C_4$, ledit résidu aliphatique en $C_1$ à $C_4$ pouvant dans chaque cas être non substitué ou mono- ou polysubstitué ; ou un résidu cycloaliphatique en $C_3$ à $C_6$, ou bien un résidu hétérocycloaliphatique tri- à hexagonal, dans chaque cas non substitué ou mono- ou polysubstitué,
sous réserve qu'au moins un de $R^2$ et $R^3$ représente H ou soit lié par l'intermédiaire d'un atome de carbone,
et les substituants respectifs restant de $R^4$ et $R^5$, chacun indépendamment l'un de l'autre, représentent H ; $CH_2F$ ; $CHF_2$ ; $CF_3$ ; un résidu aliphatique en $C_1$ à $C_4$, ledit résidu aliphatique en $C_1$ à $C_4$ pouvant être non substitué ou mono- ou polysubstitué ; ou un résidu cycloaliphatique en $C_3$ à $C_6$ ou bien un résidu hétérocycloaliphatique tri- à hexagonal, dans chaque cas non substitué ou mono- ou polysubstitué ;
à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,
ou
$R^2$ et $R^4$, conjointement avec les atomes de carbone les connectant, forment un résidu cycloaliphatique en $C_3$ à $C_{10}$ ou un résidu hétérocycloaliphatique tri- à décagonal, dans chaque cas non substitué ou mono- ou polysubstitué,
et $R^3$ représente H ; F ; Cl ; Br ; I ; CN ; $CH_2F$ ; $CHF_2$ ; $CF_3$ ; OH ; $OCH_2F$ ; $OCHF_2$ ; $OCHF_3$ ; $SCF_3$ ; un résidu aliphatique en $C_1$ à $C_4$, ou un résidu O-aliphatique en $C_1$ à $C_4$, ledit résidu aliphatique en $C_1$ à $C_4$ pouvant dans chaque cas être non substitué ou mono- ou polysubstitué ; ou un résidu cycloaliphatique en $C_3$ à $C_6$ ou bien un résidu hétérocycloaliphatique tri- à hexagonal, dans chaque cas non substitué ou mono- ou polysubstitué ;
et $R^5$ représente H ; $CH_2F$ ; $CHF_2$ ; $CF_3$; un résidu aliphatique en $C_1$ à $C_4$, le résidu aliphatique en $C_1$ à $C_4$ pouvant être non substitué ou mono- ou polysubstitué ; ou un résidu cycloaliphatique en $C_3$ à $C_6$ ou bien un résidu hétérocycloaliphatique tri- à hexagonal, dans chaque cas non substitué ou mono- ou polysubstitué ;
à condition que, si $R^5$ représente un résidu hétérocycloaliphatique tri- à hexagonal, le résidu hétérocycloaliphatique tri- à hexagonal soit lié par l'intermédiaire d'un atome de carbone,
où un "groupe aliphatique" et un "résidu aliphatique" peuvent dans chaque cas être ramifiés ou un non ramifiés, saturés ou insaturés,
où un "résidu cycloaliphatique" et un "résidu hétérocycloaliphatique" peuvent dans chaque cas être saturés ou insaturés,
où l'expression "mono- ou polysubstitué" en rapport avec un "groupe aliphatique" et un "résidu aliphatique" désigne, par rapport aux résidus ou groupes correspondants, la substitution d'un ou plusieurs atomes d'hydrogène, chacun indépendamment l'un de l'autre, par au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4)_2$, un NH-C(=O)-(résidu aliphatique en $C_1$ à $C_4$), un N (résidu aliphatique en $C_1$ à $C_4$)-C(=O)-(résidu aliphatique en $C_1$ à $C_4$), un NH-S(=O)$_2$-(résidu aliphatique en $C_1$ à $C_4$), un N(résidu aliphatique en $C_1$ à $C_4$)-S(=O)$_2$-(résidu aliphatique en $C_1$ à $C_4$), =0, OH, $OCH_2F$, $OCHF_2$, $OCF_3$, un O-(résidu aliphatique en $C_1$ à $C_4$), un O-C(=O)-(résidu aliphatique en $C_1$ à $C_4$), SH, $SCF_3$, un S-(résidu aliphatique en $C_1$ à $C_4$), S(=O)$_2$OH, un S(=O)-(résidu aliphatique en $C_1$ à $C_4$), un S (=O)$_2$-(résidu aliphatique en $C_1$ à $C_4$), un S(=O)$_2$-O-(résidu aliphatique en $C_1$ à $C_4$), un S(=O)$_2$-NH-(résidu aliphatique en $C_1$ à $C_4$), un S(=O)$_2$-N(résidu aliphatique en $C_1$ à $C_4)_2$, CN, $CH_2F$, $CHF_2$, $CF_3$, CHO, COOH, un résidu aliphatique en $C_1$ à $C_4$, $CH_2OH$, $CH_2$-$OCH_3$, $C_2H_4$-OH, $C_2H_4$-$OCH_3$, $CH_2$-$CF_3$, un C(=O)-(résidu aliphatique en $C_1$ à $C_4$), un C(=O)-O-(résidu aliphatique en $C_1$ à $C_4$), un résidu cycloaliphatique en $C_3$ à $C_6$, un résidu hétérocycloaliphatique tri- à hexagonal, C(=O)-$NH_2$, un C(=O)-NH(résidu aliphatique en $C_1$ à $C_4$), et un C(=O)-N(résidu aliphatique en $C_1$ à $C_4$) ;
où l'expression "mono- ou polysubstitué" en rapport avec un "résidu cycloaliphatique" et un "résidu hétérocycloaliphatique" désigne, par rapport aux résidus correspondants, la substitution d'un ou plusieurs atomes d'hydrogène, chacun indépendamment l'un de l'autre, par au moins un substituant choisi dans le groupe consistant en F, Cl, Br, I, $NO_2$, $NH_2$, un NH (résidu aliphatique en $C_1$ à $C_4$), un N(résidu aliphatique en $C_1$ à $C_4)_2$, un NH-C(=O)-(résidu aliphatique en $C_1$ à $C_4$), un N (résidu aliphatique en $C_1$ à $C_4$)-C(=O)-(résidu aliphatique en $C_1$ à $C_4$), un NH-S(=O)$_2$-(résidu aliphatique en $C_1$ à $C_4$), un N(résidu aliphatique en $C_1$ à $C_4$)-S(=O)$_2$-(résidu aliphatique en $C_1$ à $C_4$), =0, OH, $OCH_2F$, $OCHF_2$, $OCF_3$, un O-(résidu aliphatique en $C_1$ à $C_4$), un O-C(=O)-(résidu aliphatique en $C_1$ à $C_4$), SH, $SCF_3$, un S-(résidu aliphatique en $C_1$ à $C_4$), S(=O)$_2$OH, S(=O)-(résidu aliphatique en $C_1$ à $C_4$), un S(=O)$_2$-(résidu aliphatique en $C_1$ à $C_4$), un S(=O)$_2$-O-(résidu aliphatique en $C_1$ à $C_4$), un S(=O)$_2$-NH-(résidu aliphatique en $C_1$ à $C_4$), un S(=O)$_2$-N (résidu aliphatique en $C_1$ à $C_4)_2$, CN, $CH_2F$, $CHF_2$, $CF_3$, CHO, COOH, un résidu aliphatique en $C_1$ à $C_4$, $CH_2OH$, $CH_2$-$OCH_3$, $C_2H_4$-OH, $C_2H_4$-$OCH_3$, $CH_2$-$CF_3$, un C(=O)-(résidu aliphatique en $C_1$ à $C_4$), un C(=O)O-(résidu aliphatique en $C_1$ à $C_4$), un résidu cycloaliphatique en $C_3$ à $C_6$, un résidu hétérocycloaliphatique tri- à hexagonal, C(=O)-$NH_2$, un C(=O)-NH-(résidu aliphatique en $C_1$ à $C_4$), et un C(=O)-N (résidu aliphatique en $C_1$ à $C_4)_2$ ;
éventuellement sous forme d'un stéréoisomère unique ou d'un mélange de stéréoisomères, sous forme du composé libre et/ou d'un de ses sels physiologiquement acceptables.

**2.** Composé suivant la revendication 1, **caractérisé en ce que** :

$R^1$ représente un résidu aliphatique en $C_2$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique an $C_1$ à $C_4$), $OCH_2F$, $OCHF_2$, $OCF_3$, SH, $SCF_3$, un S-(résidu aliphatique en $C_1$ à $C_4$), un S(=O)(résidu aliphatique en $C_1$ à $C_4$), un S(=O)$_2$(résidu aliphatique en $C_1$ à $C_4$), CN, et un résidu aliphatique en $C_1$ à $C_4$,

où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou représente un résidu cycloaliphatique en $C_3$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)(résidu aliphatique en $C_1$ à $C_4$), un S(=O)$_2$(résidu aliphatique en $C_1$ à $C_4$), CN, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
et où le résidu cycloaliphatique en $C_3$ à $C_6$ peut être éventuellement ponté par l'intermédiaire d'un groupe aliphatique en $C_1$ à $C_4$, qui peut lui-même être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou représente un groupe $OR^6$, dans lequel :
$R^6$ représente un résidu aliphatique en $C_1$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$ ;
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$. $CH_2F$, $CHF_2$, $CF_3$ et un O-(résidu aliphatique en $C_1$ à $C_4$) non substitué,
$R^2$ et $R^3$, indépendamment l'un de l'autre, représentent H ; un groupe F ; Cl ; Br ; I ; CN ; $CH_2F$ ; $CHF_2$ ; $CF_3$ ; OH ; $OCH_2F$ ; $OCHF_2$ ; $OCF_3$ ; $SCF_3$ ; un résidu aliphatique en $C_1$ à $C_4$, ou un résidu O-aliphatique en $C_1$ à $C_4$,
où le résidu aliphatique en $C_1$ à $C_4$ peut, dans chaque cas, être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S(=0)(résidu aliphatique en $C_1$ à $C_4$), un S(=O)$_2$-(résidu aliphatique en $C_1$ à $C_4$), résidu aliphatique, CN, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, sous réserve qu'au moins un de $R^2$ et $R^3$ représente H ou soit lié par l'intermédiaire d'un atome de carbone,
$R^4$ et $R^5$, indépendamment l'un de l'autre, représentent H ; un groupe $CH_2F$ ; $CHF_2$ ; $CF_3$ ; ou un résidu aliphatique en $C_1$ à $C_4$,
où le résidu aliphatique peut être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,
à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, ou
$R^2$ et $R^3$
et/ou $R^4$ et $R^5$
par paires, dans chaque cas indépendamment l'un de l'autre, conjointement avec l'atome de carbone les connectant, forment un résidu cycloaliphatique en $C_3$ à $C_{10}$ ou un résidu hétérocycloaliphatique tri- à décagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, et les substituants respectifs restants de $R^2$ et $R^3$, chacun indépendamment l'un de l'autre, représentent H ; F ; Cl ; Br ; I ; CN ; $CH_2F$ ; $CHF_2$ ; $CF_3$; OH ; $OCH_2F$ ; $OCHF_2$ ; $OCF_3$ ; $SCF_3$ ; un résidu aliphatique en $C_1$ à $C_4$, ou O-(résidu aliphatique en $C_1$ à $C_4$),
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, ou représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, sous réserve qu'au moins un de $R^2$ et $R^3$ représente H ou soit lié par l'intermédiaire d'un atome de carbone,
et les substituants respectifs restants de $R^4$ et $R^5$, chacun indépendamment l'un de l'autre, représentent H ; $CH_2F$; $CHF_2$ ; $CF_3$ ; ou un résidu aliphatique en $C_1$ à $C_4$,
où le résidu aliphatique en $C_1$ à $C_4$ peut être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,
à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un 0 (résidu aliphatique en $C_1$ à $C_4$) non substitué,
ou bien
$R^2$ et $R^4$, conjointement avec les atomes de carbone les connectant, forment un résidu cycloaliphatique en $C_3$ à $C_{10}$ ou un résidu hétérocycloaliphatique tri- à décagonal, dans chaque cas non substitué ou mono- ou polysubstitué avec au moins un substituant choisi dans le groupe consistant en F, Cl, Br, I, $NO_2$, NH, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
et les substituants de $R^3$ restants représentent H ; F ; Cl ; Br ; I ; CN ; $CH_2F$ ; $CHF_2$ ; $CF_3$ ; OH ; $OCH_2F$ ; $OCHF_2$ ; $OCF_3$; $SCF_3$; un résidu aliphatique en $C_1$ à $C_4$, ou un O- (résidu aliphatique en $C_1$ à $C_4$),
où le résidu aliphatique en $C_1$ à $C_4$ peut dans chaque cas être non substitué ou mono- ou polysubstitué avec au

moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, ou bien représente un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué, et les substituants de $R^5$ restants représentent H ; $CH_2F$ ; $CHF_2$ ; $CF_3$; $OCH_2F$ ; $OCHF_2$ ; $OCF_3$ ; $SCF_3$ ; ou un résidu aliphatique en $C_1$ à $C_4$,
où le résidu aliphatique en $C_1$ à $C_4$ peut être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou bien représente un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S- (résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
à condition que, si $R^5$ représente un résidu hétérocycloaliphatique tri- à hexagonal, le résidu hétérocycloaliphatique tri- à hexagonal soit lié par l'intermédiaire d'un atome de carbone,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué.

**3.** Composé suivant la revendication 1 ou 2, **caractérisé en ce que** :

$R^1$ représente un résidu aliphatique en $C_2$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$,

où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou représente un résidu cycloaliphatique en $C_3$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué,
ou bien représente un groupe $OR^6$, dans lequel :
$R^6$ représente un résidu aliphatique en $C_1$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, $OCH_2F$, $OCHF_2$, $OCF_3$, $CH_2F$, $CHF_2$, $CF_3$ et un O-(résidu aliphatique en $C_1$ à $C_4$) non substitué.

**4.** Composé suivant l'une quelconque des revendications précédentes, **caractérisé en ce que** :

$R^1$ représente un résidu aliphatique en $C_2$ à $C_6$, non substitué ou mono- ou polysubstitué avec au moins un

substituant choisi dans le groupe consistant en F, Cl, Br, I, $NO_2$, NH, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN, et un résidu aliphatique en $C_1$ à $C_4$),

où le résidu aliphatique en $C_1$ à $C_4$ est dans chaque cas non substitué,
ou bien représente un résidu cycloaliphatique en $C_3$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N (résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
où le résidu aliphatique en $C_1$ à $C_4$ est dans chaque cas non substitué,
ou bien représente un groupe $OR^6$, dans lequel :
$R^6$ représente un résidu aliphatique en $C_1$ à $C_6$, non substitué ou mono- ou polysubstitué avec au moins un substituant choisi dans le groupe consistant en F, Cl, Br, I, $NO_2$, $NH_2$, un NH (résidu aliphatique en $C_1$ à $C_4$), un N(résidu aliphatique en $C_1$ à $C_4$)$_2$, OH, =0, un O-(résidu aliphatique en $C_1$ à $C_4$), $OCH_2F$, $OCHF_2$, $OCF_3$, SH, $SCF_3$, un S-(résidu aliphatique en $C_1$ à $C_4$), un S(=O)-(résidu aliphatique en $C_1$ à $C_4$), un S(=O)$_2$-(résidu aliphatique en $C_1$ à $C_4$), CN et un résidu aliphatique en $C_1$ à $C_4$,
où le résidu aliphatique en $C_1$ à $C_4$ est dans chaque cas non substitué.

5. Composé suivant l'une quelconque des revendications précédentes, **caractérisé en ce que** :

$R^1$ représente un résidu aliphatique en $C_2$ à $C_6$, non substitué ou mono- ou polysubstitué avec au moins un substituant choisi dans le groupe consistant en F, Cl, Br, I, OH, et un O-(résidu aliphatique en $C_1$ à $C_4$) non substitué, ou représente un résidu cycloaliphatique en $C_3$ à $C_6$, non substitué ou mono- ou polysubstitué avec au moins un substituant choisi dans le groupe consistant en F, Cl, Br, I, OH, et un O-(résidu aliphatique en $C_1$ à $C_4$) non substitué,

ou bien représente un groupe $OR^6$, dans lequel :

$R^6$ représente un résidu aliphatique en $C_1$ à $C_4$,
non substitué ou mono- ou polysubstitué avec au moins un substituant choisi dans le groupe consistant en F, Cl, Br, I, OH, et un O-(résidu aliphatique en $C_1$ à $C_4$) non substitué.

6. Composé suivant l'une quelconque des revendications précédentes, **caractérisé en ce que** :

$R^1$ représente un groupe éthyle, n-propyle, 2-propyle, n-butyle, isobutyle, sec.-butyle, tertiobutyle, n-pentyle, isopentyle, néopentyle, n-hexyle, $CH_2$-CH($CH_3$)($C_2H_5$), C($CH_3$)$_2$($C_2H_5$), éthényle ou propényle (-$CH_2CH=CH_2$, -CH=CH-$CH_3$, -C(=$CH_2$)-$CH_3$), cyclopropyle, cyclobutyle, cyclopentyle, ou cyclohexyle,

ou représente un groupe $OR^6$, dans lequel :

$R^6$ représente un groupe méthyle, éthyle, n-propyle, 2-propyle, n-butyle, isobutyle, sec.-butyle, tertiobutyle, $CH_2OH$, $CH_2OCH_3$, $CH_2CH_2OH$, $CH_2CH_2OCH_3$ et CH(OH)$CH_2OH$.

7. Composé suivant l'une quelconque des revendications précédentes, **caractérisé en ce que** :

$R^2$ et $R^3$, indépendamment l'un de l'autre, représentent H ; F ; Cl ; Br ; I ; $CH_2F$ ; $CHF_2$ ; $CF_3$ ; OH ; $OCH_2F$ ; $OCHF_2$ ; $OCF_3$; un résidu aliphatique en $C_1$ à $C_4$, ou un groupe O-(résidu aliphatique en $C_1$ à $C_4$),
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,
ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu

aliphatique en $C_1$ à $C_4$) non substitué, sous réserve qu'au moins un de $R^2$ et $R^3$ représente H ou soit lié par l'intermédiaire d'un atome de carbone,

$R^4$ et $R^5$ indépendamment l'un de l'autre, représentent H ; $CH_2F$ ; $CHF_2$ ; $CF_3$ ; ou un résidu aliphatique en $C_1$ à $C_4$,

où le résidu aliphatique en $C_1$ à $C_4$ peut être non substitué ou mono- ou polysubstitué avec au moins un substituant choisi dans le groupe consistant en F, Cl, Br, I, OH, et un O-(résidu aliphatique en $C_1$ à $C_4$) non substitué,

ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,

où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,

à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,

ou

$R^2$ et $R^3$

et/ou $R^4$ et $R^5$

par paires, dans chaque cas indépendamment l'un de l'autre, conjointement avec l'atome de carbone les connectant, forment un résidu cycloaliphatique en $C_3$ à $C_6$ ou un résidu hétérocycloaliphatique tri- à décagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,

où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,

et les substituants respectifs restants de $R^2$ et $R^3$, chacun indépendamment l'un de l'autre, représentent H ; F ; Cl ; Br ; I ; $CH_2F$ ; $CHF_2$ ; $CF_3$ ; OH ; $OCH_2F$ ; $OCHF_2$ ; $OCF_3$ ; un résidu aliphatique en $C_1$ à $C_4$, ou un O-(résidu aliphatique en $C_1$ à $C_4$),

où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,

ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,

où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,

sous réserve qu'au moins un de $R^2$ et $R^3$ représente H ou soit lié par l'intermédiaire d'un atome de carbone,

et les substituants respectifs restants de $R^4$ et $R^5$, chacun indépendamment l'un de l'autre, représentent H ; $CH_2F$ ; $CHF_2$ ; $CF_3$ ; ou un résidu aliphatique en $C_1$ à $C_4$,

où le résidu aliphatique en $C_1$ à $C_4$ peut être non substitué ou mono- ou polysubstitué avec au moins un substituant choisi dans le groupe consistant en F, Cl, Br, I, OH, et un O-(résidu aliphatique en $C_1$ à $C_4$) non substitué,

ou bien représentent un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,

à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,

où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,

ou bien

$R^2$ et $R^4$, conjointement avec les atomes de carbone les connectant, forment un résidu cycloaliphatique en $C_3$ à $C_{10}$ ou un résidu hétérocycloaliphatique tri- à décagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$ et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en C1 à C4) non substitué,
et le substituant de $R^3$ restant représente H ; F ; Cl ; Br ; I ; $CH_2F$ ; $CHF_2$ ; $CF_3$ ; OH ; $OCH_2F$ ; $OCHF_2$ ; $OCF_3$ ; un résidu aliphatique en $C_1$ à $C_4$, ou un O-(résidu aliphatique en $C_1$ à $C_4$),
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,
ou bien représente un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué,
et le substituant de $R^5$ restant représente H ; $CH_2F$ ; CHF ; $CF_3$ ; ou un résidu aliphatique en $C_1$ à $C_4$ ;
où le résidu aliphatique en $C_1$ à $C_4$ peut être non substitué ou mono- ou polysubstitué avec au moins un substituant choisi dans le groupe consistant en F, Cl, Br, I, OH, et un O-(résidu aliphatique en $C_1$ à $C_4$) non substitué,
ou bien représente un résidu cycloaliphatique en $C_3$ à $C_6$ ou un résidu hétérocycloaliphatique tri- à hexagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, $OCH_2F$, $OCHF_2$, $OCF_3$, et un résidu aliphatique en $C_1$ à $C_4$,
à condition que, si $R^5$ représente un résidu hétérocycloaliphatique tri- à hexagonal, le résidu hétérocycloaliphatique tri- à hexagonal soit lié par l'intermédiaire d'un atome de carbone,
où le résidu aliphatique en $C_1$ à $C_4$ peut dans chaque cas être non substitué ou mono- ou polysubstitué avec au moins un substituant choisi dans le groupe consistant en F, Cl, Br, I, OH, et un O-(résidu aliphatique en $C_1$ à $C_4$) non substitué.

8. Composé suivant l'une quelconque des revendications précédentes, **caractérisé en ce que** :

$R^2$ et $R^3$ indépendamment l'un de l'autre, représentent H ; F ; Cl ; Br ; I ; $CH_2F$ ; $CHF_2$ ; $CF_3$ ; OH ; un résidu aliphatique en $C_1$ à $C_4$, ou un O-(résidu aliphatique en $C_1$ à $C_4$),
où le résidu aliphatique en $C_1$ à $C_4$ est dans chaque
cas non substitué,
ou bien représentent un résidu cycloaliphatique en $C_3$ à $C_6$ ou un résidu hétérocycloaliphatique tri- à hexagonal, dans chaque cas non substitué,
sous réserve qu'au moins un de $R^2$ et $R^3$ représente H ou soit lié par l'intermédiaire d'un atome de carbone,
$R^4$ et $R^5$, indépendamment l'un de l'autre, représentent H ; ; CHF ; $CHF_2$ ; $CF_3$ ; ou un résidu aliphatique en $C_1$ à $C_4$,
où le résidu aliphatique en $C_1$ à $C_4$ est non substitué, ou bien représentent un résidu cycloaliphatique en $C_3$ à $C_6$ ou un résidu hétérocycloaliphatique tri- à hexagonal, dans chaque cas non substitué,
à condition que, si $R^4$ et/ou $R^5$ représente(nt) un résidu hétérocycloaliphatique tri- à hexagonal, le résidu hétérocycloaliphatique tri- à hexagonal soit lié par l'intermédiaire d'un atome de carbone,
ou
$R^2$ et $R^3$
et/ou $R^4$ et $R^5$
par paires, dans chaque cas indépendamment l'un de l'autre, conjointement avec l'atome de carbone les connectant, forment un résidu cycloaliphatique en $C_3$ à $C_{10}$ ou un résidu hétérocycloaliphatique tri- à décagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$, et un résidu aliphatique en $C_1$ à $C_4$,
où le résidu aliphatique en $C_1$ à $C_4$ est dans chaque cas non substitué,
et les substituants respectifs restants de $R^2$ et $R^3$, chacun indépendamment l'un de l'autre, représentent H ; F ; Cl ; Br ; I ; $CH_2F$ ; $CHF_2$ ; $CF_3$ ; OH ; un résidu aliphatique en $C_1$ à $C_4$, ou un O-(résidu aliphatique en $C_1$ à $C_4$),
où le résidu aliphatique en $C_1$ à $C_4$ est dans chaque cas non substitué,

ou bien représentent un résidu cycloaliphatique en $C_3$ à $C_6$ ou un résidu hétérocycloaliphatique tri- à hexagonal, dans chaque cas non substitué,
sous réserve qu'au moins un de $R^2$ et $R^3$ représente H ou soit lié par l'intermédiaire d'un atome de carbone,
et les substituants respectifs restants de $R^4$ et $R^5$, chacun indépendamment l'un de l'autre, représentent H ; $CH_2F$ ; $CHF_2$ ; $CF_3$ ; ou un résidu aliphatique en $C_1$ à $C_4$,
où le résidu aliphatique en $C_1$ à $C_4$ est non substitué, ou représentent un résidu cycloaliphatique en $C_3$ à $C_6$ ou un résidu hétérocycloaliphatique tri- à hexagonal, dans chaque cas non substitué,
à condition que, si $R^4$ et/ou $R^5$ représente (nt) un résidu hétérocycloaliphatique tri- à hexagonal, le résidu hétérocycloaliphatique tri- à hexagonal soit lié par l'intermédiaire d'un atome de carbone,
ou
$R^2$ et $R^4$ conjointement avec les atomes de carbone les connectant, forment un résidu cycloaliphatique en $C_3$ à $C_{10}$ ou un résidu hétérocycloaliphatique tri- à décagonal, dans chaque cas non substitué ou mono- ou polysubstitué avec au moins un substituant choisi dans le groupe consistant en F, Cl, Br, I, OH, un O-(résidu aliphatique en $C_1$ à $C_4$), $CH_2F$, $CHF_2$, $CF_3$ et un résidu aliphatique en $C_1$ à $C_4$,
où le résidu aliphatique en $C_1$ à $C_4$ est dans chaque cas non substitué,
et le substituant de $R^3$ restant représente H ; F ;
Cl ; Br ; I ; $CH_2F$ ; $CHF_2$ ; $CF_3$ ; OH ; un résidu aliphatique en $C_1$ à $C_4$ ou un O-(résidu aliphatique en $C_1$ à $C_4$),
où le résidu aliphatique en $C_1$ à $C_4$ est dans chaque
cas non substitué,
ou bien représente un résidu cycloaliphatique en $C_3$ à $C_6$ ou un résidu hétérocycloaliphatique tri- à hexagonal, dans chaque cas non substitué,
et le substituant de $R^5$ restant représente H ; $CH_2F$ ; $CHF_2$ ; $CF_3$ ; ou un résidu aliphatique en $C_1$ à $C_4$,
où le résidu aliphatique en $C_1$ à $C_4$ est non substitué, ou représente un résidu cycloaliphatique en $C_3$ à $C_6$ ou un résidu hétérocycloaliphatique tri- à hexagonal, dans chaque cas non substitué,
à condition que, si $R^5$ représente un résidu hétérocycloaliphatique tri- à hexagonal, le résidu hétérocycloaliphatique tri- à hexagonal soit lié par l'intermédiaire d'un atome de carbone.

**9.** Composé suivant l'une quelconque des revendications précédentes, **caractérisé en ce que** :

$R^2$ et $R^3$, indépendamment l'un de l'autre, représentent H ; OH ; un résidu aliphatique en $C_1$ à $C_4$ non substitué, ou un O-(résidu aliphatique en $C_1$ à $C_4$) non substitué, sous réserve qu'au moins un de $R^2$ et $R^3$ représente H ou soit lié par l'intermédiaire d'un atome de carbone,
$R^4$ et $R^5$, indépendamment l'un de l'autre, représentent H ; $CH_2F$ ; $CHF_2$ ; $CF_3$ ; ou un résidu aliphatique en $C_1$ à $C_4$ non substitué,
ou
$R^2$ et $R^3$
ou $R^4$ et $R^5$
par paires, dans chaque cas indépendamment l'un de l'autre, conjointement avec l'atome de carbone les connectant, forment un résidu cycloaliphatique en $C_3$ à $C_{10}$, non substitué ou mono- ou polysubstitué avec au moins un substituant choisi dans le groupe consistant en F, Cl, Br, I, OH, $CH_2F$, $CHF_2$, $CF_3$, un O-(résidu aliphatique en $C_1$ à $C_4$) non substitué et un résidu aliphatique en $C_1$ à $C_4$ non substitué,
et les substituants respectifs restants de $R^2$ et $R^3$, chacun indépendamment l'un de l'autre, représentent H ; OH ; un résidu aliphatique en $C_1$ à $C_4$ non substitué ou un O-(résidu aliphatique en $C_1$ à $C_4$) non substitué, sous réserve qu'au moins un de $R^2$ et $R^3$ représente H ou soit lié par l'intermédiaire d'un atome de carbone,
et les substituants respectifs restants de $R^4$ et $R^5$, chacun indépendamment l'un de l'autre, représentent H ; $CH_2F$ ; $CHF_2$ ; $CF_3$ ; ou un résidu aliphatique en $C_1$ à $C_4$ non substitué,
ou
$R^2$ et $R^4$ conjointement avec les atomes de carbone les connectant, forment un résidu cycloaliphatique en $C_3$ à $C_{10}$, non substitué ou mono- ou polysubstitué avec au moins un substituant choisi dans le groupe consistant en F, Cl, Br, I, OH, un O-(résidu aliphatique en $C_1$ à $C_4$) non substitué, $CH_2F$, $CHF_2$, $CF_3$, et un résidu aliphatique en $C_1$ à $C_4$ non substitué,
et le substituant de $R^3$ restant représente H ; F ; Cl ; Br ; I ; OH ; un résidu aliphatique en $C_1$ à $C_4$ non substitué, ou un O-(résidu aliphatique en $C_1$ à $C_4$) non substitué,
et le substituant de $R^5$ restant représente H ; $CH_2F$ ; $CHF_2$ ; $CF_3$; ou un résidu aliphatique en $C_1$ à $C_4$ non substitué.

**10.** Composé suivant l'une quelconque des revendications précédentes, **caractérisé en ce que** :

$R^2$ et $R^3$, indépendamment l'un de l'autre, représentent H ; un groupe méthyle, éthyle, n-propyle, 2-propyle, n-butyle, isobutyle, sec.-butyle, tertiobutyle, n-pentyle, isopentyle, néopentyle, n-hexyle, $CH_2$-CH ($CH_3$) ($C_2H_5$), $C(CH_3)_2(C_2H_5)$, $OCH_3$ ou $OCH_2CH_3$, sous réserve qu'au moins un de $R^2$ et $R^3$ représente H ou soit lié par l'intermédiaire d'un atome de carbone,

$R^4$ et $R^5$, indépendamment l'un de l'autre, représentent H ; un groupe méthyle, éthyle, n-propyle, 2-propyle, n-butyle, isobutyle, sec.-butyle, tertiobutyle, n-pentyle, isopentyle, néopentyle, n-hexyle, $CH_2$-$CH(CH_3)(C_2H_5)$, $C(CH_3)_2(C_2H_5)$, $CH_2F$ ; $CHF_2$ ; $CF_3$ ;

ou

$R^2$ et $R^3$

ou $R^4$ et $R^5$

par paires, dans chaque cas indépendamment l'un de l'autre, conjointement avec l'atome de carbone les connectant, forment un résidu cycloaliphatique en $C_3$ à $C_6$ choisi dans le groupe consistant en les résidus cyclopropyle, cyclobutyle, cyclopentyle et cyclohexyle, non substitué ou mono- ou polysubstitué avec au moins un substituant choisi dans le groupe consistant en F, Cl, Br, I, OH, méthyle, éthyle, n-propyle, 2-propyle, n-butyle, isobutyle, sec.-butyle, tertiobutyle, $CH_2F$, $CHF_2$, $CF_3$, $OCH_3$ et $OCH_2CH_3$,

et les substituants respectifs restants de $R^2$ et $R^3$, chacun indépendamment l'un de l'autre, représentent H ; un substituant méthyle, éthyle, n-propyle, 2-propyle, n-butyle, isobutyle, sec.-butyle, tertiobutyle, n-pentyle, isopentyle, néopentyle, n-hexyle, $CH_2$-$CH(CH_3)(C_2H_5)$, $C(CH_3)_2(C_2H_5)$, $OCH_3$ ou $OCH_2CH_3$, sous réserve qu'au moins un de $R^2$ et $R^3$ représente H ou soit lié par l'intermédiaire d'un atome de carbone,

et les substituants respectifs restants de $R^4$ et $R^5$, chacun indépendamment l'un de l'autre, représentent H ; méthyle, éthyle, n-propyle, 2-propyle, n-butyle, isobutyle, sec.-butyle, tertiobutyle, n-pentyle, isopentyle, néopentyle, n-hexyle, $CH_2$-$CH(CH_3)(C_2H_5)$, $C(CH_3)_2(C_2H_5)$, $CH_2F$ ; $CHF_2$ ; $CF_3$ ;

ou

$R^2$ et $R^4$ conjointement avec les atomes de carbone les connectant, forment un résidu cycloaliphatique en $C_3$ à $C_6$ choisi dans le groupe consistant en les résidus cyclopropyle, cyclobutyle, cyclopentyle et cyclohexyle, non substitué ou mono- ou polysubstitué avec au moins un substituant choisi dans le groupe consistant en F, Cl, Br, I, OH, méthyle, éthyle, n-propyle, 2-propyle, n-butyle, isobutyle, sec.-butyle, tertiobutyle, $CH_2F$, $CHF_2$, $CF_3$, $OCH_3$ et $OCH_2CH_3$,

et le substituant respectif restant de $R^3$ représente H ; méthyle, éthyle, n-propyle, 2-propyle, n-butyle, isobutyle, sec.-butyle, tertiobutyle, n-pentyle, isopentyle, néopentyle, n-hexyle, $CH_2$-$CH(CH_3)(C_2H_5)$, $C(CH_3)_2(C_2H_5)$, $OCH_3$ ou $OCH_2CH_3$,

et le substituant respectif restant de $R^5$ représente H ; méthyle, éthyle, n-propyle, 2-propyle, n-butyle, isobutyle, sec.-butyle, tertiobutyle, n-pentyle, isopentyle, néopentyle, n-hexyle, $CH_2$-$CH(CH_3)(C_2H_5)$, $C(CH_3)_2(C_2H_5)$, $CH_2F$ ; $CHF_2$ ; $CF_3$.

**11.** Composé suivant l'une quelconque des revendications précédentes, **caractérisé en ce que** :

$R^1$ représente un groupe éthyle, n-propyle, 2-propyle, n-butyle, isobutyle, sec.-butyle, tertiobutyle, cyclo-propyle, cyclobutyle, cyclopentyle, ou cyclohexyle, ou représente un groupe $OR^6$, dans lequel :

$R^6$ représente un groupe méthyle, éthyle, n-propyle, 2-propyle, n-butyle, isobutyle, sec.-butyle, ou tertiobutyle,

$R^2$ et $R^3$, indépendamment l'un de l'autre, représentent H ; méthyle, éthyle, n-propyle, 2-propyle, n-butyle, isobutyle, sec.-butyle, ou tertiobutyle,

$R^4$ et $R^5$, indépendamment l'un de l'autre, représentent H ; méthyle, éthyle, n-propyle, 2-propyle, n-butyle, isobutyle, sec.-butyle, tertiobutyle, $CH_2F$, $CHF_2$, ou $F_3$ ;

ou

$R^2$ et $R^3$

ou $R^4$ et $R^5$

par paires, dans chaque cas, indépendamment l'un de l'autre, conjointement avec l'atome de carbone les connectant, forment un résidu cycloaliphatique en $C_3$ à $C_6$ choisi dans le groupe consistant en cyclopropyle, cyclobutyle, cyclopentyle et cyclohexyle, non substitué ou mono- ou polysubstitué avec au moins un substituant choisi dans le groupe consistant en OH, méthyle, éthyle, n-propyle, 2-propyle, n-butyle, isobutyle, sec.-butyle, $CH_2F$, $CHF_2$, $CF_3$, $OCH_3$ et $OCH_2CH_3$,

et les substituants respectifs restants de $R^2$ et $R^3$, indépendamment l'un de l'autre, représentent H ; méthyle, éthyle, n-propyle, 2-propyle, n-butyle, isobutyle, sec.-butyle, ou tertiobutyle,

et les substituants respectifs restants de $R^4$ et $R^5$, indépendamment l'un de l'autre, représentent H ; méthyle,

éthyle, n-propyle, 2-propyle, n-butyle, isobutyle, sec.-butyle, tertiobutyle, $CH_2F$, $CHF_2$, ou $CF_3$ ;
ou
$R^2$ et $R^4$ conjointement avec les atomes de carbone les connectant, forment un résidu cycloaliphatique en $C_3$ à $C_6$ choisi dans le groupe consistant en cyclopropyle, cyclobutyle, cyclopentyle et cyclohexyle, non substitué ou mono- ou polysubstitué avec au moins un substituant choisi dans le groupe consistant en OH, méthyle, éthyle, n-propyle, 2-propyle, n-butyle, isobutyle, sec.-butyle, tertiobutyle, $CH_2F$, $CHF_2$, $CF_3$, $OCH_3$ et $OCH_2CH_3$,
et $R^3$ représente H ; méthyle, éthyle, n-propyle, 2-propyle, n-butyle, isobutyle, sec.-butyle, ou tertiobutyle,
et $R^5$ représente H ; méthyle, éthyle, n-propyle, 2-propyle, n-butyle, isobutyle, sec.-butyle, tertiobutyle, $CH_2F$, $CHF_2$, ou $CF_3$.

**12.** Composé suivant l'une quelconque des revendications précédentes, **caractérisé en ce qu'**il est choisi dans le groupe comprenant :

1 l'amide d'acide 2-cyclopropyl-N-(3-hydroxy-4,4-diméthyl-pentyl)-4-méthyl-6-morpholine-4-yl-pyridine-3-carboxylique ;
2 l'amide d'acide 2-cyclopropyl-N-[[(1S,2R)-2-hydroxy-cyclohexyl]-méthyl]-4-méthyl-6-morpholine-4-yl-pyridine-3-carboxylique ;
3 l'amide d'acide 2-cyclopropyl-N-[[(1R,2S)-2-hydroxy-cyclohexyl]-méthyl]-4-méthyl-6-morpholine-4-yl-pyridine-3-carboxylique ;
4 l'amide d'acide 2-cyclopropyl-N-([2-hydroxy-cyclopentyl]-méthyl)-4-méthyl-6-morpholine-4-yl-pyridine-3-carboxylique ;
5 l'amide d'acide N-(3-hydroxy-4,4-diméthyl-pentyl)-2-isopropyl-4-méthyl-6-morpholine-4-yl-pyridine-3-carboxylique ;
6 l'amide d'acide 2-isopropyl-4-méthyl-6-morpholine-4-yl-N-(4,4,4-trifluoro-3-hydroxy-butyl)-pyridine-3-carboxylique ;
7 l'amide d'acide N-[[(1S,2R)-2-hydroxy-cyclohexyl]-méthyl]-2-isopropyl-4-méthyl-6-morpholine-4-yl-pyridine-3-carboxylique ; et
8 l'amide d'acide 2-éthoxy-N-(3-hydroxy-4,4-diméthyl-pentyl)-4-méthyl-6-morpholine-4-yl-pyridine-3-carboxylique,

éventuellement sous forme d'un stéréoisomère unique ou d'un mélange de stéréoisomères, sous forme du composé libre et/ou d'un de ses sels physiologiquement acceptables.

**13.** Composition pharmaceutique comprenant au moins un composé suivant l'une quelconque des revendications précédentes, éventuellement sous forme d'un stéréoisomère unique ou d'un mélange de stéréoisomères, sous forme du composé libre et/ou d'un de ses sels physiologiquement acceptables, et éventuellement au moins un auxiliaire pharmaceutiquement acceptable.

**14.** Composé suivant l'une quelconque des revendications 1 à 12, pour une utilisation dans le traitement et/ou la prophylaxie de troubles et/ou de maladies qui sont soumis à une médiation, au moins en partie, par les canaux KCNQ2/3$K^+$.

**15.** Composition suivant la revendication 13, pour une utilisation dans le traitement et/ou la prophylaxie de troubles et/ou de maladies choisis dans le groupe consistant en la douleur, de préférence une douleur choisie dans le groupe consistant en la douleur aiguë, la douleur chronique, la douleur neuropathique, la douleur musculaire, la douleur viscérale et la douleur inflammatoire, l'épilepsie, l'incontinence unaire, l'anxiété, la dépendance, la manie, les troubles bipolaires, la migraine, des maladies cognitives et des dyskinésies associées à une dystonie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020128277 A
- WO 2008046582 A
- WO 2010046108 A
- WO 2010102809 A
- WO 2002066036 A
- WO 2012052167 A
- DE 2513949
- GB 1420987 A


### Non-patent literature cited in the description


- **PASSMORE et al.** *J. Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **GRIBKOFF.** *Expert Opin Ther Targets,* 2008, vol. 12 (5), 565-81
- **MICELI et al.** *Curr Opin Pharmacol,* 2008, vol. 8 (1), 65-74
- **STRENG et al.** *J Urol,* 2004, vol. 172, 2054-2058
- **HANSEN et al.** *Eur J Pharmacol,* 2007, vol. 570 (1-3), 77-88
- **DENCKER et al.** *Epilepsy Behav,* 2008, vol. 12 (1), 49-53
- **RICHTER et al.** *Br J Pharmacol,* 2006, vol. 149 (6), 747-53
- Remington's Pharmaceutical Sciences. Mack Publishing Company, 1985
- **BENNETT, G.J. ; XIE, Y.K.** A peripheral mononeuropathy in rat that produces disorders of pain sensation like those seen in man. *Pain,* 1988, vol. 33 (1), 87-107
- **KIM, S.H. ; CHUNG, J.M.** An experimental model for peripheral neuropathy produced by segmental spinal nerve ligation in the rat. *Pain,* 1992, vol. 50 (3), 355-363
- **D'AMOUR ; SMITH.** *J. Pharm. Exp. Ther.,* 1941, vol. 72, 74-79
- **D. DUBUISSON et al.** *Pain,* 1977, vol. 4, 161-174
- **DE SARRO et al.** *Naunyn-Schmiedeberg's Arch. Pharmacol.,* 2001, vol. 363, 330-336